# EUROPEAN PATENT SPECIFICATION

(11) **EP 3 588 506 B1**
(45) Date of publication and mention of the grant of the patent: **10.11.2021**
(21) Application number: 18180792.6
(22) Date of filing: 29.06.2018
(51) Int. Cl.: G16B 20/20, G16B 30/10, G16B 40/20

(54) **SYSTEMS AND METHODS FOR GENOMIC AND GENETIC ANALYSIS**
SYSTEME UND VERFAHREN FÜR GENOMISCHE UND GENETISCHE ANALYSEN
SYSTÈMES ET PROCÉDÉS D'ANALYSE GÉNOMIQUE ET GÉNÉTIQUE

(43) Date of publication of application: 01.01.2020
(73) Proprietor: Molecular Health GmbH, 69115 Heidelberg (DE)
(72) Inventor: STEIN, Martin, Dr., 68307 Mannheim (DE); BOHNERT, Regina, Dr., 70186 Stuttgart (DE); RIEBER, Nora, Dr., 69115 Heidelberg (DE)
(74) Representative: Lahrtz, Fritz

(56) References cited:
- US-A1- 2013 190 321
- US-A1- 2014 067 280
- US-A1- 2016 321 395
- US-A1- 2018 148 773
- LINNÉA MALGERUD ET AL: "Bioinformatory-assisted analysis of next-generation sequencing data for precision medicine in pancreatic cancer", MOLECULAR ONCOLOGY, vol. 11, no. 10, 8 August 2017 (2017-08-08), pages 1413-1429, XP55535104, AMSTERDAM, NL ISSN: 1574-7891, DOI: 10.1002/1878-0261.12108

## Description

The present invention relates to systems and methods for genomic and genetic analysis of a human nucleic acid sample.

### Background of the invention

### Next-generation sequencing (NGS)

Next-generation sequencing, also known as high-throughput sequencing, is a routine method for the high-throughput and parallel sequencing of nucleic acid fragments which is well known to the person skilled in the art. The equipment and methodology of next-generation sequencing is commercially available from diverse suppliers (see, e.g., www.illumina.com).

Next-generation sequencing is the catch-all term used to describe a number of different modern sequencing technologies including:
- Illumina (Solexa) sequencing
- Ion torrent: Proton / PGM sequencing
- SOLiD sequencing.

NGS technologies produce high-quality DNA sequences ("reads"). These reads are substantially shorter than the reads produced by the capillary-based Sanger sequencing technology (650-1000 bp), developed by Frederick Sanger and colleagues in 1977, which was the most widely used sequencing method for approximately 30 years. Sanger reads are produced in low-throughput with high costs, while the NGS methods produce much shorter reads (25-500 bases) at moderate costs. However, the total number of base pairs sequenced in a NGS run is orders of magnitude higher. These two factors cause many new informatics challenges, including the ability to process these millions or even billions of short NGS reads. The reads are usually processed in one of two ways: either they are mapped back to their correct locations in an existing backbone/reference sequence, building a sequence that is similar but not necessarily identical to the backbone sequence (called "read mapping"), or they are built into a new sequence (called "de novo assembly").

The primary advantage of read mapping back to a reference genome as opposed to de-novo assembly is that it greatly simplifies the process of genome inference. While assembly needs to discover the entire genomic sequence and gives rise to many ambiguities, reference-based resequencing only needs to discover a sample's differences from the reference. In terms of complexity and time requirements, de novo assemblies are orders of magnitude slower and more memory intensive than mapping assemblies.

Read mapping is the first and most fundamental step in NGS analysis pipelines that aim to discover the variation of a newly sequenced human genome (or fractions of it, like the exome or a small targeted panel of genes) with respect to the previously sequenced human reference genome.

### The Human Reference Genome (HRG)

In February 2001 the Human Genome Project, a U.S. federal government effort, together with Celera Genomics, a private company, successfully completed drafts of the entire human genome, which was revised several times subsequently Lander et al. 2001, Venter et al. 2001, Church et al. 2011]. Over a number of years, the genome assembly has steadily improved and new versions ("builds") have been released, to the point that the current Genome Reference Consortium (GRC) human genome assembly, GRCh38 [Schneider et al. 2017], is arguably the best assembled mammalian genome in existence, with just 875 remaining assembly gaps and fewer than 160 million unspecified "N" nucleotides (as of GRCh38.p8), whereas the first version had ∼150,000 gaps ^{[Editorial (}October 2010). "E pluribus unum". Nature Methods. 7 (5): 331. doi:10.1038/nmeth0510-331

The HRG is the single most important resource used in human genetics and genomics today. It acts as a universal coordinate system and as such is the space in which annotations (genes, promoters, etc.) and genetic variants are described ^{[}Harrow et al. 2012; ENCODE, 2012; 1000 Genomes Project Consortium, 2012]. It is also the target for the alignment step in next-generation sequencing analysis pipelines. Downstream of this mapping, it is used for functional assays and variant calling pipelines [Li H & Durbin 2009; DePristo et al., 2011].

The initial build of the HRG was composed of DNA sequences from a small cohort of thirteen anonymous DNA donors who had volunteered in Buffalo, New York with primarily European origins [Snyder et al]. Donors were recruited by advertisement in the Buffalo News, on Sunday, March 23rd, 1997. The first ten male and ten female volunteers were invited to make an appointment with the project's genetic counselors and donated blood from which DNA was extracted. As a result of how the DNA samples were processed, about 80 percent of the reference genome came from eight people. One male, designated as RP11, accounted for 66 percent of the total.

To identify and resolve larger assembly issues, e.g. complex regions containing large-scale duplications and structural variations, sequence data from new genome mapping technologies and single haplotype resources originating from new donors have been brought into the most recent builds. At the time of filing the present application, GRCh38 contains sequences from about 50 different individuals, see http://www.bio-itworld.com/2013/4/22/church-on-reference-genomes-past-present-future.html.

### Limitations of the HRG

### 1. The HRG is linear

The human DNA is packaged into physically separate units called chromosomes. Humans are diploid organisms, containing two sets of genetic information, one set inherited from the mother and one from the father. Thus, each somatic cell has 22 pairs of chromosomes called autosomes (one member of each pair from each parent) and two sex chromosomes (an X and a Y chromosome in males and two X chromosomes in females). Each chromosome contains a single very long, linear DNA molecule. In the smallest human chromosomes, this DNA molecule is composed of about 50 million nucleotide pairs; the largest chromosomes contain about 250 million nucleotide pairs.

The diploid human genome is thus composed of 46 single DNA molecules of 24 distinct types. Because human chromosomes exist in pairs that are almost identical, only 3 billion nucleotide pairs (the haploid genome) needed to be sequenced to gain complete information concerning a representative human genome. The human genome is thus said to contain 3 billion nucleotide pairs, even though most human cells contain 6 billion nucleotide pairs. The haploid human genome consists of 22 autosomal chromosomes and the Y and the X chromosomes.

Each of the chromosomes represents a single DNA molecule, a sequence of millions of nucleotide bases. These molecules are linear, so one might expect that each chromosome should be represented by a single, continuous/linear nucleic acid sequence. Unfortunately, this is not the case for two main reasons: 1) because of the nature of genomic DNA and the limitations of sequencing methods, some parts of the genome remain unsequenced, and 2) some regions of the genome vary so much between individual people that they cannot be represented as a single continuous sequence. However, the HRG is represented as 24 linear DNA sequences consisting of the normal bases (A, C, T or G) with gaps represented as a series of "N"s clearly showing the position of gaps in the assembly.

The primary goal of the Human Genome Project was to produce a single representative sequence albeit with regions of uncertainty - that is, a single "scaffold" - for each physical chromosome. It also included a handful of alternate scaffolds representing allelic variation (different versions of the DNA bases present at a SNP locus are referred to as alleles), but they had no formalized relationship to the main scaffold. Recognizing that some highly polymorphic regions of the genome were particularly poorly represented by a single reference sequence, a formal model to introduce representative alternate versions of highly variable regions was added starting with GRCh37 [Church et al. 2011]. Sequences in the form of kilobase to multi-megabase "alternate locus scaffolds" were described relative to the "primary" (haploid) assembly, anchored to locations along the primary scaffolds. In the assembly at the time of filing the present application (GRCh38.p9), these cover 178 regions and total 261 linear sequences [Paten et al. 2017]

Another complicating factor is that the HRG was deduced in the original international genome sequencing project from a collection of DNAs from multiple anonymous individuals. Thus the resulting HRG is really a randomly mixed conglomerate, a haploid mosaic of different DNA sequences, that, in some cases, may be impossible to represent correctly as a single linear sequence.

### 2. The HRG is certainly not disease free

Chen & Butte (2011) identified 3,556 disease-susceptible variants including 15 rare variants (Major Allele Frequency <1%) in the HRG. Using a curated, high-quality quantitative human disease-SNP association database, the authors assessed the likelihood ratio of increased risk over healthy population on 104 diseases for the reference genome and found high risk for type 1 diabetes, hypertension and other disorders. This provides clear evidence that the HRG does not represent a regular person and is certainly not disease free. Although it has dramatically accelerated the analysis of personal genome sequencing efforts, focusing on variants different from the reference genome will likely miss many disease causal variants including rare variants [Chen & Butte 2011].

### 3. Reference allele bias towards European ancestry

A major challenge in using the HRG assemblies in prior art NGS analysis pipelines is the fact that they are derived from DNA samples from a relatively small number of anonymous donors with a bias towards European ancestry and therefore represent a small sampling of the broad array of human genetic variation.

Despite the relative effectiveness and ubiquity of the reference as a coordinate system for the majority of the genome, there is increasing concern that using the HRG as a lens to study all other human genomes excludes a great deal of common human variation and introduces a pervasive reference allele bias [Petrovski et al. 2016, Paten et al. 2017]. Reference allele bias is the tendency to over-report alleles present in the reference genome and under-report other alleles whose underlying DNA does not match a reference allele [Degner et al. 2009, Brandt et al. 2015].

This bias arises chiefly during the read mapping step in resequencing experiments. To map correctly, reads must derive from genomic sequence that is both represented in the reference and similar enough to the reference sequence to be identified as the same genomic element. When these conditions are not met, mapping errors introduce a systematic blindness to the true sequence [Paten et al. 2017]. Reference allele bias also has the potential to affect some genetic subpopulations and some regions of the genome more than others, depending on the ancestral history of the reference genome at each locus bias [Petrovski et al. 2016, paten et al. 2017]. Highly polymorphic regions such as HLA genes are especially susceptible to the effects of reference allele bias [Nielsen et al. 2011], particularly when a single reference genome is used as an index for the alignment of NGS reads. In this situation, many true variants fail to be identified because they are present in haplotypes that differ from the genome used as index, and thus reads generated from these regions are not aligned and are lost [Brandt et al. 2015].

As described above, reference bias is a known issue in human genome resequencing using the HRG for variant detection, and modifications to the reference can improve calling accuracy and interpretability [Fakhro et al. 2016]. One approach to mitigate this issue is to modify variant prevalence information early on in the genome-interpretation process by modifying the reference genome, such that variants discovered in the genome are the minor allele in the population [Dewey et al., 2011]. This modification to the reference results in a streamlined analysis workflow, as the number of false positives can be reduced and fewer variants need to be interpreted [Fakhro et al. 2016].

### The future: graph-based reference structures/genome graphs

There is increasing recognition that a single, haploid reference genome is a poor universal reference structure for human genetics and genomics, because it represents only a tiny fraction of human variation: there exist variants and annotations that can not easily be described with respect to the reference genome [Horton et al. 2008, Pei et al. 2012]. Furthermore, as a target for read mapping and interpretation, it introduces a reference allele bias as described above. To mitigate these issues, recent versions of the reference genome assembly, such as the human genome assembly at the time of filing the present application (GRCh38.p9), have included "alternate locus" sequences ("alts"): additional multiple sequence representations of regions of the human genome considered to be highly polymorphic, anchored at their ends to locations within the "primary" (haploid) reference assembly. Such a structure, which contains multiple partially-overlapping sequence paths, can be considered a form of a mathematical graph: a genome graph [Novak et al. 2017].

Graphs have a longstanding place in biological sequence analysis, in which they have often been used to compactly represent an ensemble of possible sequences.

As a rule, the sequences themselves are implicitly encoded as walks in the graph. This makes graphs a natural fit for representing reference cohorts, which are by their nature ensembles of related sequences [Paten et al. 2017]. The graph contains not only the sample's approximate sequence, but also many of its specific variants.

Genome graphs are expected to produce improvements in read mapping, variant calling, and haplotype determination. It is anticipated that graph-based references will supplant linear references in humans and in other applications where cohorts of sequenced individuals are available [Novak et al. 2017]. Various projects are underway to build and apply these genome graphs. Genome graphs can now be built from libraries of common variants, and some tools, though still experimental, illustrate the huge potential of the graph-based approach.

Despite the theoretical advantages, research on variant calling using genome graphs is still relatively nascent. There is a number of questions yet to be tackled. How should duplications and repeats be represented? How can one best map to a graph? How should short variants whose homologies are unclear be parsed? How can graphs be used to enable a more comprehensive taxonomy of variation? These questions all represent open avenues for future research.

To be useful in practice, genome graphs must be able to translate their promised reduction in reference bias into measurable improvements in variant calling over established methodologies. Accordingly, developing variant calling algorithms for genome graphs is currently an important research frontier.

### The Qatar genome (QTRG)

Qatar is a small peninsula on the Persian Gulf with a total population of approximately 300,000 Qatari citizens. Qataris have one of the highest rates of consanguineous marriages in the world, which is still increasing, and the rate of endogamous marriage in Qatar approaches -100%. All these factors together with the large family size are the main reasons for the high rate of indigenous genetic diseases, which represent a financial burden on the Qatari budget. These factors triggered the Qatari government to seek a means to protect their own people from the threat of genetic diseases [Zayed 2016].

Government officials decided to launch the Qatar genome project (QGP) in 2013 (http://www.gulf-times.com/story/374345/Qatarlaunches-genome-project). The intent of the project was to sequence the genome of each Qatari citizen in an effort to protect Qataris from the high rate of indigenous genetic diseases by allowing the mapping of disease-causing variants/rare variants and establishing a Qatari reference genome as a path towards personalized medicine. The final goal of the project is to apply the information to clinical practice and to allow this approach to become a routine part of the Qatari healthcare system [Zayed 2016]. To achieve the expected clinical application promise of the QGP, several serious challenges must be met, including reaching high variant-calling sensitivity and accuracy [Koboldt 2010]. To facilitate precision medicine in regions of the Middle East and North Africa, a population-specific genome, custom tailored to disease research in the indigenous Arab population of Qatar (QTRG) was constructed by incorporating allele frequency data from whole genome sequencing of 1,161 Qataris, representing 0.4% of the population. A total of 20.9 million single-nucleotide polymorphisms (SNPs) and 3.1 million inDels were observed in Qatar, including an average of 1.79% novel variants per individual genome [Fakhro et al. 2016].

### 1000 Genomes Project (1kG)

The 1000 Genomes Project was formed in 2008 to sequence and generate a catalog of human genetic variation (with respect to HRG GRCh37) and haplotypes from the genomes of at least 1,000 people around the world (hence the name the 1000 Genomes Project). The current phase 3 analysis of the project contains 2,504 individuals from 26 populations and defined 5 so-called super-populations, which are formed as unions of 4 to 7 populations each [1000 Genomes Project Consortium et al. 2015]. This haplotype resource at finer scales will facilitate the understanding of genetic variation at genomic and geographic levels [Baye, 2011].

Document US2014/067280 A1 discloses a method for constructing an ancestral-specific reference genome database, involving preparing ancestral-specific reference genomes based on single nucleotide polymorphisms and haplotypes that are shared by composite familial genome sequence. The ancestral-specific reference genomes are used to identify ancestral-specific therapeutic, diagnostic and prognostic markers and to identify individuals who would respond to therapeutics based on their unique DNA sequence.

### Objectives of the invention

Recent advances in NGS technologies allow for quick and cheap DNA and RNA sequencing, and as such have revolutionized the study of genomics and molecular biology. Genome sequencing projects for healthy and disease cohorts have identified numerous functional or disease-associated genomic variants, which can give clues about therapeutic targets or genomic markers for novel clinical applications.

Genetic variant calling is predominantly based on alignment of raw sequence reads against a reference genome (read mapping). This alignment-based approach has many limitations including incompleteness of genome assembly [Meyer, L. R. et al., 2013], structural variations existing in the genomes of normal individuals [Sudmant et al., 2015], sequencing errors in reads, and interference of single-nucleotide polymorphisms (SNP) on read mapping [Iqbal, Z.et al., 2012].

Currently, at the time of filing the present application, read mapping against the linear HRG is the standard approach and is expected to remain the standard in clinical NGS analysis pipelines and resequencing human individuals, due to the relative effectiveness and ubiquity of the HRG as a coordinate system for the majority of the genome. In addition (and in contrast to the nascent state of genome inference using genome graphs), many successful methodologies have been published for calling variants using the linear reference genome [Nielsen et al. 2011].

However, as described above, one major issue is bias in the HRG, ignoring prior information about genetic variation within the species. At present, the issue is typically solved by modifying the reference genome, such that variants called against the modified reference genome are the minor allele in the population.

One objective of the invention is the detection of new biomarkers, in particular genetic variants such as single-nucleotide variants (SNVs), insertions and deletions (inDels), copy number variations (CNV) and structural variants (SVs), e.g. chromosomal translocation, inversion, duplication, large inDels, for the usage of next-generation sequencing in human genome research. The success of clinical genomics using NGS technologies requires the accurate and consistent identification of personal genome variants. A prerequisite for these objectives is accurate alignment and variant calling.

### Disclosure of the invention

In accordance with a first aspect of the invention, the present invention provides a method for genomic and/or genetic analysis of a human nucleic acid sample comprising the following steps:
a) providing a group of human reference genomes;
b) testing of the human nucleic acid sample for sex and/or ancestry;
c) selecting one or more population-specific human reference genomes (PHREGs) from the group of human reference genomes on the basis of the results of the sex and/or ancestry test in step b); and
d) aligning the human nucleic acid sample to the selected PHREGs.

"**P**opulation-specific **h**uman **r**eference **g**enome**s**" are understood in the following as ancestry-specific reference genomes and sex-specific reference genomes. PHREGs minimize the reference bias considerably and improve the alignment accuracy, and, if variant calling is performed subsequently, the variant calling accuracy. Advantageously, the invention does not only improve the precision of the alignment, but also improves calculation speed, the number of correctly aligned reads and the number of calculation steps of the alignment. The benefit of using PHREGs in the genomic and/or genetic analysis of a human nucleic acid sample can also result in an improved read coverage depth and can be assessed by an improved variant calling sensitivity.

In the context of the present invention, the term "human nucleic acid sample" generally means any nucleic acid sample which is isolated from a human sample. The human nucleic acid sample may in particular include NGS reads as defined in more detail below.

The human nucleic acid sample may generally comprise samples from all kind of standard biochemical, molecular and/or cell biological procedures which are suitable for the preparation of human nucleic acid. Such procedures comprise paracentesis, biopsy, liquid biopsy, cell free DNA isolation kits, or the like. The human nucleic acid sample may be or be derived from all kinds of suitable sources, including, but not limited to, body fluids, mucosa, tissues, tissue extracts or cells or any combination thereof. The human nucleic acid sample may also be a control sample derived from all kinds of suitable sources. The human nucleic acid sample may e.g. comprise blood samples, blood plasma samples, urine samples, tumor samples, possibly including undesired artefacts caused by the fixation in the tissue handling procedure FFPE (formalin-fixed paraffin-embedded tissue or formaldehyde-fixed paraffin-embedded tissue).

The human nucleic acid sample may in particular comprise DNA, RNA and/or size fractionated total DNA or RNA. Providing DNA from a sample of interest may include one or more biochemical purification steps such as, e.g., centrifugation, lysis and/or fractionation steps, cell lysis by means of mechanical or chemical disruption steps including, but not limited to, multiple freezing and/or thawing cycles, salt treatment(s), phenol-chloroform extraction, sodium dodecyl sulfate (SDS) treatment and proteinase K digestion. Optionally, providing DNA from a sample of interest may further include the removal of large RNA, such as abundant ribosomal rRNA, by precipitating in the presence of polyethylene or salt, or the removal of interfering sodium dodecyl sulfate (SDS) by precipitation in the presence of salt, preferably in the presence of potassium chloride solution. Methods of purifying total DNA or RNA from a cell and/or a tissue are well known to a person skilled in the art and include, e.g., standard procedures such as the use of guanidinium thiocyanate - acidic phenol-chloroform extraction (e.g. TRIzol®, Invitrogen, USA). Equally preferred, however, is that DNA from the sample of interest is provided without any of the herein described biochemical precipitation and/or purification steps.

In the context of the present invention, the term "nucleic acid" generally refers to any kind of single stranded or double stranded oligonucleotide molecule composed of either deoxyribonucleotides or ribonucleotides or both, including genomic DNA, nuclear DNA, somatic DNA, germline DNA, synthetically designed and/or manufactured DNA, including, but not limited to, in vitro generated DNA derived from messenger RNA profiles, preferably in form of cDNA. The term "nucleic acid" generally means single stranded or double stranded oligonucleotide molecules of identical or similar length, i.e. composed of either an identical or similar number of nucleotides.

The human nucleic acid sample may comprise genomic sequences which may serve for evaluating, analyzing, aligning, indexing and/or profiling of specific mutations on genomic, transcriptional or post-transcriptional level. As such, a human nucleic acid sample according to the present invention may refer and include, but not be limited to, any kind of coding regions, non coding regions, exons, introns, chromosomal and/or intra chromosomal regions, promoter regions, enhancer regions, regions encoding small and/or long regulatory RNAs, regions of active transcription and/or non transcribed regions, transposons, regions of hot spot mutations, regions of frame-shift mutations etc. and alike.

The "group of human reference genomes" comprises at least two human reference genomes, preferably a plurality of human reference genomes. The sex and/or ancestry test in step b) allows for the selection of one or more of the best suited human reference genomes from the group of human reference genomes. The best suited human reference genomes are used for the alignment and for the subsequent analysis. In the preferred case, the sex and/or ancestry test in step b) will allow for the selection of one PHREG from the group of human reference genomes for the alignment step, but the selection of one or more additional PHREGs for the subsequent analysis is also possible.

According to one embodiment, the testing may comprise a sex test. According to another embodiment, the testing may comprise an ancestry test. According to still another embodiment, the testing may comprise a sex test and an ancestry test.

In one exemplary embodiment, the group of human reference genomes comprises both male and female reference genomes. If the sex test in step b) determines that the human nucleic acid sample is a male or a female reference genome, then in step c), the respective male or female reference genome or genomes will be selected as the respective PHREGs for the subsequent alignment step d).

As sex chromosomes contain homologous sequences, using a sex-adjusted reference genome (with chromosomes X and Y for males, and without chromosome Y for females) will prevent misalignment of reads. Therefore, using sex-specific reference genomes reduces subsequent false positive and false negative variant calls.

In another exemplary embodiment, the group of human reference genomes comprises a number of ancestry-specific reference genomes. The ancestry test in step b) determines the best one or ones out of the number of ancestry-specific reference genomes. Then in step c), the closest one or ones will be selected as the PHREG or PHREGs for the subsequent alignment step d).

Choosing an incorrect ancestry can lead to large amounts of false positive and false negative variant calls. Using ancestry-specific reference genomes effectively increases the number of correctly aligned reads and reduces the false positives and false negatives.

Likewise, a combination of a sex and ancestry test is decisive in case that the group of human reference genomes comprises ancestry-specific male reference genomes and ancestry-specific female reference genomes.

The term "testing" in step b) is to be understood as encompassing at least one genetic and/or genomic test of the human nucleic acid sample. Genetic and/or genomic testing is more reliable than any information derived from "self-reporting". Self-reported and investigator-assigned ancestry typically relies on the subjective interpretation of a complex combination of both genetic and non-genetic information including behavior, cultural, and societal norms, skin color, and other influences. It is rarely the case that a study participant or patient will report his/her ethnicity without errors. Self-reported ethnicity errors may occur for various reasons; some people may not be fully aware of their true ancestry or only know recent ancestry (or their geographic origin) while others may identify with one ethnic group despite their admixed background [Mersha & Abebe 2015]. Literature confirms that self-declared ancestry and sex are often incorrect [Ainsworth, 2015; Mersha & Abebe, 2015]. In fact, Ainsworth even explains that 1 person in 100 is affected by a disorder of sex development, leading to a physical appearance that does not match the person's genome.

Advantageously, the method can be also used as an additional quality check to identify sample swaps based on sex and ancestry. Mismatches between self-declared and predicted sex and ancestry in sequencing runs can reveal e.g. specimen transposition and other lab processing errors.

The term "alignment" generally means a computational step wherein a sequenced sample is compared with and fitted to a reference sequence. To this end, one needs to find the corresponding part of that sequence for each read in the generated sequencing data. In other words, alignment or read mapping is the process of determining the most likely source within the genome sequence for the observed nucleic acid sequencing read. In typical embodiments, the reads will be NGS reads, but it shall be understood that reads from other sequencing technologies are also encompassed by the teaching of the present invention.

The aligned reads derived from the human nucleic acid sample may be displayed, stored, printed, sent via a communication network, or otherwise processed further. Further applications or uses of the aligned human nucleic acid sample may in particular comprise one or more of the following:

### 1) Local realignment around insertions and deletions (inDels)

The term "inDels" means insertions or deletions of base pairs in the genome, typically including small genetic variations from 1 to 10000 bp in length. Realignment around inDels improves subsequent data analysis, in particular subsequent variant calls.

### 2) Base quality score recalibration (BQSR)

The term "base quality score" describes per-base estimates of error expressing how confident the base call made by the sequencing instrument is. The score may e.g. be used for weighing the evidence of subsequent variant calls. BQSR allows for adjusting the quality scores by taking into account systematic technical errors due to the physics or the chemistry of how the sequencing was performed.

### 3) Machine learning to separate true segregating variation from machine artifacts common to next-generation sequencing technologies

### 4) Variant discovery and genotyping to find all potential variants; herein also referred to as variant calling

Variant discovery may include the discovery of SNPs/SNVs, InDels, CNVs and SVs (chromosomal translocation, inversion, duplication, large inDels).

### 5) Evolutionary analysis studies

Evolutionary analysis studies may comprise tools measuring nucleotide diversity, population divergence, linkage disequilibrium, and the frequency spectrum of mutations from one or more populations. Evolutionary analysis may generally comprise computational tools for calculating evolutionary sequence statistics. The computational tools may be adapted to perform analyses in sliding windows across chromosomes or scaffolds. The computational tools may e.g. produce a phylogenetic tree of the sample.

Such evolutionary analysis may be performed e.g. by "POPBAM" software, described e.g. in https://www.ncbi.nlm.nih.gov/pmc/articles/PMC3767577/.

### 6) Testing for wildtype biomarkers

Furthermore, the aligned human genome sample may be tested for the presence of wildtype biomarkers, i.e. biomarkers that will not be detected during variant calling because they are contained within the PHREG. The computation step after the alignment may thus comprise a test for each known biomarker, the test indicating whether the biomarker is present in the aligned human genome sample, irrespective of what the information of the PHREG at this position is.

According to one embodiment, the method comprises the additional step of performing variant calling of the aligned human nucleic acid sample with respect to the selected PHREGs. Advantageously, the invention improves the accuracy of variant calling by introducing the initial sex and/or ancestry test to determine the correct PHREGs for usage in the subsequent alignment and variant calling steps.

The aligned human nucleic acid sample, more specifically, the aligned NGS reads derived from the human nucleic acid sample may thus be further processed by one or more so-called variant callers which are computational modules, comprising different variant calling algorithms detecting any variant type (e.g. SNVs, InDels, Copy number alterations, structural variants). Subsequent method steps may comprise variant interpretation. The results of the variant calling and/or of the variant interpretation may be displayed, stored, printed, sent via a communication network, or otherwise processed further. Advantageously, the method will allow the detection of previously undiscovered biomarkers (e.g., in the context of cancer or other diseases) through the removal of bias from the used reference genome. In particular, the method according to the present invention allows for the discrimination of a variety of gene mutations, including, but not limited to SNVs, Multi-Nucleotide Variants (MNVs), complex events and large structural variants, in particular hot spot mutations, frame-shift mutations, non-silent mutations, stop-codon mutations, nucleotide insertions, nucleotide deletions, copy number variations, copy number alterations and/or splice sites.

The donor of the human nucleic acid sample can be a patient, i.e. a person having a disease or being suspected of having a disease. The application of the method, however, shall not be understood to be limited to patients only.

Variant calling and interpretation may comprise the analysis of genomic sequences indicative for the presence or absence of a certain disease. Based on the variant interpretation, the patient may be classified into a first group including patients where a certain treatment is not indicated, and into a second group including patients where a certain treatment is indicated. The invention may thus advantageously be used as a part of a disease screening procedure, evaluating the presence or absence of a disease in a patient.

Additionally or alternatively, the method may comprise a step of retrieving an indication of a disease related to, or associated with the human nucleic acid sample. The indication of a disease may be e.g. retrieved from an electronic health record or manually be entered via an input means of a computing device by the patient him/herself or by the attending physician. The indication may be identified according to disease ontologies, for instance ICD-10, MeSH, or MedDRA. For certain classes of indications there may also be specialized ontologies that may offer advantages like more precise categorization of the indication. In oncology it may be beneficial to use ICD-O-3 and/or the TNM staging system.

Based on the results of the variant calling and interpretation, and taking into account the disease of the patient, the method may involve providing a therapy plan for the patient. In the present context, the therapy plan may in particular be a personalized therapy plan for the patient, wherein the personalized therapy plan comprises treatment options tailored to the patient's genetic data, in particular to his/her clinical, molecular, and/or genetic condition.

In order to identify promising treatments for a patient, the method may comprise checking whether any of the variants, e.g. mutations found in the patient, e.g. in a patient's tumor or in the patient's normal control tissue, are indicative of the patient's outcome under any treatment. The method may further include identifying all treatments associated with any of the found variants. The method may include scoring the identified treatments and ranking the identified treatments according to the score to provide a treatment option or treatment contraindication prioritization for the patient.

In the context of the present invention, the term "treatment" includes the administration of a therapeutically effective drug or a pharmaceutically active compound in form of a pharmaceutical composition which prevents, ameliorates or treats the symptoms accompanying with the indication. The term "treatment" also includes any kind of surgery, radiotherapy and/or chemotherapy or any combination thereof.

For both alternatives, i.e. in the context of a screening method or a personalized therapy plan, the present invention may provide the physician with improved diagnostic capabilities, e.g. allowing for improved treatment decisions, because the precision of the alignment and the variant calling are improved.

According to one embodiment, the alignment is performed against the PHREG on a majority allele level. The majority allele level uses unique nucleotide codes (A,C,G,T) within the PHREG to adjust the reference sequence to a population. The single nucleotide is chosen which is most frequently observed at the given locus in the population. In case of ties in the allele frequency, the allele present in the underlying reference sequence (e.g. GRCh37 or GRCh38) may be used.

According to another embodiment, the alignment is performed against the PHREG on a non-rare alleles level. The non-rare alleles level uses ambiguity nucleotide codes according to the established IUPAC nomenclature [Cornish-Bowden, 1985], e.g., "R" for "A or G". The non-rare alleles level may encode up to two or three, preferably two, alleles of substantial frequency in the population. Substantial frequency can be defined as being more than or equal to 30%, 20%, 15%, 10%, 5%, 3%, 1% or 0,1%, in particular more than or equal to 5%. As more than one variant allele per genomic position is incorporated into the PHREG, a more precise read alignment can be expected. In one embodiment, only single-nucleotide variations (SNVs) are considered in the non-rare alleles level. In other embodiments, inDels and other structural variations are considered as well.

According to one embodiment, the variant calling is performed against the PHREG on a majority allele level. In some embodiments, the alignment may be performed on a non-rare alleles level and the variant calling may be performed on a majority allele level. Alternatively, the variant calling is performed on a non-rare alleles level.

According to an embodiment, the human reference genomes provided in step a) are published human reference genomes. Published human reference genomes may in particular comprise the builds of the HRG, specifically the builds of GRCh37 and GRCh38. Additionally or alternatively, published human reference genomes may comprise the QTRG. Additionally or alternatively, published human reference genomes may comprise genomes derived from the 1000 Genomes (1kG) project. For the 1kG project, the VCF files for all chromosomes from the most current release on the 1kG FTP site ftp://ftp.1000genomes.ebi.ac.uk/vol1/ftp/release/20130502/ may be downloaded and used. If more datasets with more individuals and ethnicities become publically available (e.g. the 1000 Arab genome project to study the Emirati population [Al-Ali, M.et al., 2018]), these could also be used in the method of the present invention.

Additionally or alternatively, the human reference genomes provided in step a) are derived from the published human reference genomes. The term "derived from" may in particular encompass error correction and/or adjusting the human reference genomes to a majority allele encoding level or to a non-rare alleles level.

Error correction may be performed such that reference nucleotides that are observed in zero individuals of a given population are replaced by the corresponding majority nucleotide.

In an embodiment, step a) comprises adjusting the human reference genomes to an encoding level, the encoding level comprising either unique nucleotide codes or ambiguous nucleotide codes. The encoding level comprising unique nucleotide codes may in particular be used to define the PHREGs on a majority allele level. The encoding level comprising ambiguous nucleotide codes may in particular be used to define the PHREGs on a non-rare alleles level.

In one embodiment, for the adjustment to the encoding levels single-nucleotide variations are considered. For each population (or super-population) all reported SNVs together with their allele frequencies are used. In other embodiments, inDels, CNVs and/or SVs are also considered.

According to an embodiment, four different levels of adjusting the reference sequence to a population are proposed, wherein two of these are confined to unique nucleotide codes (A,C,G,T) and another two utilize ambiguous nucleotide encoding according to the IUPAC nomenclature [Cornish-Bowden, 1985], e.g. "R" for "A or G". These PHREG encoding levels are defined as follows:
1. Maximally conservative error correction: reference nucleotides that are observed in zero individuals of the population are replaced by the corresponding majority nucleotide, e.g. by the corresponding majority 1kG nucleotide.
2. Majority allele: choose the single nucleotide which is most frequently observed at the given locus in the population (in case of ties in the allele frequency, the allele present in the underlying reference sequence, e.g. GRCh37 or GRCh38, is used).
3. Non-rare alleles: encode up to two alleles of substantial frequency (e.g. ≥ 5%) in the population, using IUPAC codes as necessary.
4. Complete modelling of observed alleles: encode at each position all (up to four) alleles that are reported in at least one individual of the population.

The complete representation of 1kG variants in Level 4 PHREGs is, however, paid for by a disproportionately large number of genome modifications that introduce ambiguity and thereby may substantially impede the seed finding by read mappers. Therefore, in one embodiment, Level 3 is used for alignment using a IUPAC ambiguity-aware alignment algorithm. Since the currently best-performing variant callers are not designed to handle ambiguity codes, Level 2 PHREGs are used for subsequent variant calling unless better performing IUPAC ambiguity-aware alignment algorithms are available.

Advantageously, the method thus allows for user-defined levels of adjustments of the PHREG to population genetic variation, depending on the population(s) in focus and depending on the downstream analysis.

According to an embodiment, the human reference genomes provided in step a) are PHREGs. Step a) thus may comprise e.g. downloading the PHREGs from a public source.

As defined above, PHREGs are in a first place understood as ancestry-specific reference genomes and/or sex-specific reference genomes. In one embodiment, the human reference genomes provided in step a) are already population-specific because they comprise meta data indicating their ancestry and/or sex. For example, at the time of filing the present application, the current phase 3 analysis of the 1kG project contains 2,504 individuals from 26 populations and from 5 so-called super-populations, which are formed as unions of 4 to 7 populations each. The 26 populations from the 1kG study phase 3, and their associated 5 superpopulations (AFR, African; AMR, Ad Mixed American; EAS, East Asian; EUR, European; SAS, South Asian) can be found on http://www.internationalgenome.org/faq/which-populations-are-part-your-study.

In one embodiment, the data from the 1kG project is used to build optimized population-specific human reference genomes for each of the 31 (super-) populations, and an additional super-population encompassing all other populations.

In case the human reference genomes provided in step a) are PHREGs, the public meta-data of the PHREG may as well be provided, e.g. via download from a public source. The meta-data may serve as a quality control for the method. If the meta-data and the sex- and ancestry classifier data coincide, then the quality control may be considered as successful. If not, the software may produce a warning or an alert which is displayed to the user, and, additionally or alternatively, the software may stop the procedure e.g. before the alignment step.

According to an embodiment, the sex test comprises at least one of the following: testing at least one position in a sex-specific gene on chromosome X and/or on chromosome Y; leveraging alignment differences of human genome samples on chromosome X and/or chromosome Y; cytogenetic tests; FISH analysis; CGH analysis, or any other experimental method allowing the determination of a human nucleic acid sample's sex, directly or indirectly.

The sex test may thus also be a result or a side product of a FISH analysis (fluorescence in situ hybridization analysis) [Gall J. G. 1969] of the human nucleic acid sample. The sex test may thus also be a result or a side product of a CGH analysis (comparative genomic hybridization) [Kallioniemi A. et al. 1992] of the human nucleic acid sample.

The sex test enables efficient and reliable distinction of a male or female human nucleic acid sample.

As individuals from an ancestry or ethnicity share many SNPs that set them apart from other ancestries or ethnicities, the best-fitting PHREG for read alignment and variant calling can be identified by examining a range of ancestry-determining SNPs. The PHREGs may thus be selected from the group of human reference genomes on the basis of the results of an ancestry test.

Different experimental setups can be used in an upstream genomic analysis pipeline step to ascertain an individual's ancestry before proceeding with the alignment, to determine the best matching PHREG reference and to avoid errors.
1) The ancestry test may be based on a machine learning algorithm used on a human nucleic acid sample, or on another classification scheme that leverages ancestry-specific variants. The ancestry test method may in particular be based on machine learning that leverages the genotypes of exonic positions, e.g. more than 100, 500, 1000, 2000 or preferably more than 5000 exonic positions.
2) The determination of relevant genotypes can be based on NGS data or on an alternative experimental approach, for example a SNP array, as it is done in forensics research [Fondevila et al. 2013]. Here, the use of non-coding SNPs can help determining ethnicity.
3) The same non-coding SNPs (plus flanking regions) as tested in the forensic SNP array from alternative 2) could be added to existing targeted NGS panels to determine relevant genotypes.

In particular, the ancestry test may comprise using the genotype of at least one genomic position.

In one particular embodiment, the ancestry test comprises testing at least one gene selected from the enclosed sequence protocol. The 249 genes from the enclosed sequence protocol were shown to yield exact results.

Additionally or alternatively, the ancestry test may comprise the testing of SNP arrays and/or SNP chips and/or testing of markers from Sanger sequencing or mass spectroscopy, or any other experimental method designed to determine relevant genotypes.

In one particular embodiment, the ancestry test comprises testing at least one gene selected from the group of genes consisting of ABL2, ATP1A3, CIC, CYP2C8, CYP2C9, EPHA3, EPHA7, ERBB3, ERG, ETV1, F2, FAS, HFE, IL11RA, IL2RA, ITGB6, KIF11, KIT, KLK3, LRP6, MDM4, NAT2, NTRK2, PDGFB, PIK3R1, PLA2G3, PLAU, PRKCB, RICTOR, SLC7A11, STAT3, T, TSC1, VCAM1, VDR, VEGFB, ACVRL1, AXL, CA9, CALCR, CASP9, ENG, EPHB1, ERBB4, ESR1, FGFR2, HPSE, HSP90AA1, ITK, MRE11A, PLK1, PTPRC, SERPINE1, SMC4, TERT, TLR3, WISP3, WT1, XRCC1, ANGPT2, ARID2, BARD1, CBR3, CDH2, CYP1B1, DDR2, DNMT3A, EPCAM, ERCC2, FANCG, FANCL, GSTP1, IRS2, ITGB1, JAK3, LHCGR, MSH6, NCF2, RNF43, SLC5A5, TMPRSS2, TNFRSF8, AKT1, CD248, CD4, ESR2, EZH2, IGF1R, ITGAV, ITGB2, KLHL6, MAP3K1, MET, MLL, MTHFR, NFKB1, NUP93, PARP8, RB1, RPE65, TSHR, ABL1, BLM, CYP19A1, DPP4, EPHA6, ERBB2, EWSR1, FOXP4, ITGAM, KDM5A, LPA, LTK, MLH1, PBRM1, PHLPP2, SF3B1, TNFRSF10A, ABCG2, ACPP, ADAM15, DPYD, EPHA5, EPHB6, FOLH1, KDR, MSH3, MST1R, NTRK1, ROCK2, SLC6A2, TET2, TGM2, TH, ABCB1, CD22, CD40, CD44, CDH20, CYP11B2, ERCC5, GPR124, IL7R, ITGB3, ITGB5, NCL, NOD2, NR4A1, PGR, PLCG1, PPP2R1A, PRAME, PTCH2, RET, SETD2, XPC, ASXL1, EPHB4, PLA2G6, SYK, TET1, EP300, FLT1, ITGA1, LOXL2, PDGFRB, PIK3CD, SSTR5, TEC, APC, ATR, CLU, CREBBP, CYP2D6, EML4, MMP2, PARP2, PDGFRA, TRPM8, CSF1R, DOT1L, FGFR3, FGFR4, GLP2R, IKBKE, JAK1, NOTCH2, SPEN, SPG7, BRCA1, CYP11B1, GNAS, ITGA5, LTF, NRP2, PTK2B, TNKS, ABCC1, CEACAM5, CYP4B1, EGFR, FLT3, INSR, PTCH1, SMARCA4, ZNF217, BCR, EEF2, SELP, SLCO1B1, ABCC2, FLT4, MTR, IL4R, MTOR, RPTOR, TEK, ATM, CARD11, FANCD2, MEFV, NF1, TP73, BRCA2, CD109, PTPRD, ABCC6, IGF2R, P2RX7, ROS1, ACE, PARP1, PRKDC, CENPE, TSC2, ALK, NOTCH1, TNC, NOTCH3, POLE, MLL2, MYH11, POLD1, GRIN3B, F5, FANCA, LRP1B, LRP2, VWF.

In a more particular embodiment, the ancestry test comprises testing at least one of the genomic coordinates selected from the group of genomic coordinates listed in Appendix 1. Appendix 1 describes the GRCh37-based genomic coordinates of the features used for the ancestry classifier. The first 3 columns are formatted according to the BED file standard (https://www.ensembl.org/info/website/upload/bed.html) and (from the left to the right) correspond to chromosome, 0-based start of the feature, and 0-based end of the feature (i.e., the first position after the end of the feature). Column 4 shows the bases that are relevant to the classifier at this position, and column 5 the corresponding gene name.

Gene names were approved by the HUGO Gene Nomenclature Committee (HGNC, https://www.genenames.org/). HGNC is responsible for approving unique symbols and names for human loci, including protein coding genes, ncRNA genes and pseudogenes, to allow unambiguous scientific communication. The gene names used in the present application were retrieved in August 2013.

In another particular embodiment, the ancestry test comprises at least one of the SNPs listed in Appendix 2 [Fondevila et al. 2013]. Appendix 2 indicates the number of the chromosome on which an SNP is positioned (left column), the exact chromosomal position (middle column) as well as the corresponding rs number (right column). The rs number is an accession number assigned by the NCBI (National Center for Biotechnology Information) in its SNP database (dbSNP, https://www.ncbi.nlm.nih.gov/projects/SNP/) and it is widely used to refer to specific SNPs across genomic databases. When researchers identify a SNP, they send a report (which includes the sequence immediately surrounding the SNP) to the dbSNP database. If overlapping reports are sent in, they are merged into the same, non-redundant Reference SNP cluster, which is assigned a unique rsid. More information is available in the following url http://www.ncbi.nlm.nih.gov/sites/books/NBK44406/.

Such an ancestry test may comprise genetic and/or genomic tests enabling a distinction of ancestry categories. Such ancestry categories may be defined as AFR, AMR, EAS, EUR, SAS, in accordance with the 1kG project. The method is, however, not limited to the 1kG project data, e.g. in case that more comprehensive datasets with more individuals/ethnicities were/became available, these could be used for the same purpose alternatively.

According to an embodiment, the human nucleic acid sample comprises a set of reads issued from a next-generation sequencing procedure, and the alignment comprises a step of mapping the reads to the selected PHREGs. Additionally or alternatively, the human nucleic acid sample comprises a set of reads issued from a targeted sequencing procedure, e.g. from panel sequencing.

Advantageously, the method can be seamlessly integrated into any existing NGS analysis workflow which is based on read mapping against a HRG.

Aligning the human nucleic acid sample to the selected PHREGs by mapping the reads to the selected PHREGs may presuppose the preparation of sequencing libraries by random fragmentation of the DNA or cDNA sample, followed by 5'- and 3'-adapter ligation in advance. In some embodiments, the fragmentation and ligation reaction is combined into a single step, followed by PCR amplification of the adapter-ligated fragments.

Aligning the human nucleic acid sample to the selected PHREGs by mapping the reads to the selected PHREGs may presuppose the sequencing of this set of DNA fragments, resulting in reads of approximately between 28 base pairs (bp) and 1000 base pairs (bp) in lengths [Goodwin S. et al. 2016]. The set encompasses enough reads to reach a pre-determined target region coverage suitable for the experimental questions asked (typically between a few x and several 1000x).

In one embodiment, the next-generation sequencing procedure involves whole exome sequencing. In another embodiment, the next-generation sequencing procedure involves whole genome sequencing. The term "whole exome sequencing" generally means a technique for sequencing all the protein-coding genes in a genome (known as the exome). It consists of first selecting only the subset of DNA that encodes proteins (known as exons) and then sequencing this DNA using any high-throughput DNA sequencing technology. Humans have about 180,000 exons, constituting about 1.5% of the human genome, or approximately 30 million base pairs. In particular, the exome sequencing may be carried out by next-generation sequencing. "Whole genome sequencing" (also known as WGS, full genome sequencing, complete genome sequencing, or entire genome sequencing) is a laboratory process that determines the complete DNA sequence of an organism's genome at a single time. This entails sequencing all of an organism's chromosomal DNA as well as DNA contained in the mitochondria.

In accordance with another aspect of the invention, a computer system for gene analysis of a human genome sample comprises:
a) a first module comprising computer instructions for providing a group of human reference genomes;
b) a second module for testing of a human nucleic acid sample for sex and/or ancestry;
c) a third module comprising computer instructions for selecting one or more population-specific human reference genomes, PHREGs, from the group of human reference genomes on the basis of the results of the sex and/or ancestry test; and
d) a fourth module comprising computer instructions for aligning the human nucleic acid sample to the determined PHREGs

In particular, the computer system may be adapted for, or may be configured for performing any one of the methods disclosed above. Therefore, it is understood that features which have been described in the context of the methods are disclosed for the computer system, and, vice versa, that features which will be described in the context of the computer system are disclosed for the methods as well.

The modules may be software modules, software routines or software subroutines stored on a machine-readable storage medium such as a permanent or rewriteable storage means, or on a storage medium assigned to a computer means, for instance a mobile storage medium such as CD-ROM, DVD, Blu-ray disc, sticks or memory cards. Additionally or alternatively, the modules may be provided on a computer means such as a server or a cloud server for download, for example via a data network such as the internet or via a communication line such as a telephone line or a wireless line.

Any of the modules disclosed herein may be functional units which are not necessarily physically separated from each other. Several units of the modules may be realized in the form of one single physical unit, for instance if several functions are implemented in a software package.

The computer modules disclosed herein may not necessarily be part of an integral system, but may be distributed over several individual systems interacting with each other over a communication network.

According to one embodiment, the second module for testing of a human nucleic acid sample for sex and/or ancestry is a computer module comprising computer instructions. Additionally or alternatively, the second module may comprise a wet lab experiment, e.g. an experiment performing a FISH test. The results of the FISH test may be electronically or visually analyzed for determining the sex of the sample.

In accordance with yet another aspect of the invention, a computer program comprises instructions which, when the program is executed by a computer, cause the computer to carry out the steps a), b), c) and d) of any one of the methods described above.

In accordance with still another aspect of the invention, a computer-readable storage medium comprises instructions which, when executed by a computer, cause the computer to carry out the steps a), b), c) and d) of any one of the methods described above.

### Brief description of the figures

The foregoing and other objects, aspects, features, and advantages of the disclosure will become more apparent and better understood by referring to the following description taken in conjunction with the accompanying drawings, in which:
**Fig. 1** is a flow diagram depicting a method for genomic and/or genetic analysis of a human nucleic acid sample in accordance with the present invention;
**Fig. 2** is a flow diagram depicting methods for data analysis in accordance with the present invention;
**Fig. 3** is a representation of read mapping steps;
**Fig. 4** is a flow diagram depicting a method for genomic and/or genetic analysis of a human nucleic acid sample in accordance with the present invention;
**Fig. 5** is a diagram representing the distribution of chosen features for the sex classifier by class computed on the MH Panel data; and
**Fig.** 6 are boxplots of memory usage and runtime of the two Ansextry classifiers and EthSEQ.

### Detailed description of the figures

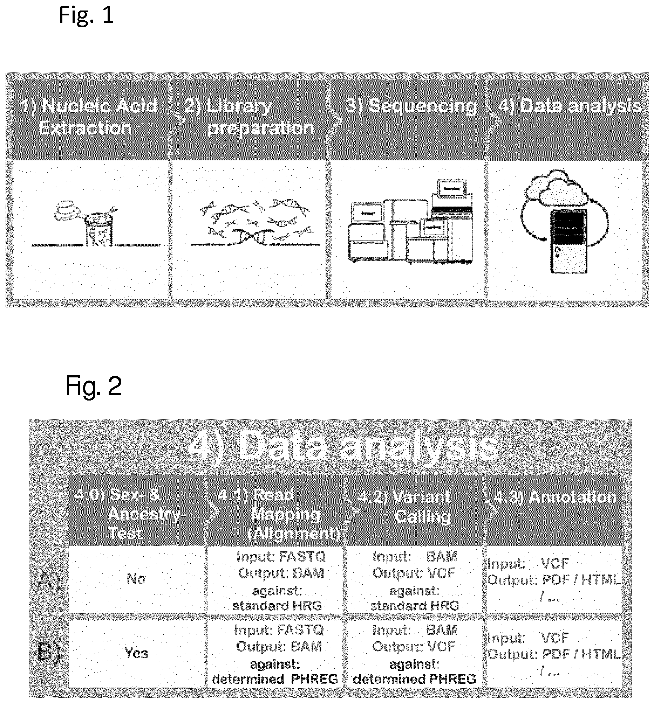

Figure 1 illustrates the general workflow for genomic and/or genetic analysis of a human nucleic acid sample, which comprises the process of extraction of the human nucleic acid sample, the preparation of a sequencing library, the sequencing and the subsequent data analysis. In the context of the present invention, the processes of extraction of the human nucleic acid sample, the preparation of a sequencing library and the sequencing may involve well-known standard processes and will not be explained in more detail. The inventive data analysis part is shown in Figure 2 in more detail.

Figure 2 shows the data analysis step of Figure 1 comprising a first sex and ancestry test step, followed by an alignment (or read mapping) step, a variant calling step and an annotation step. The input file for the read mapping computational module is raw sequence data, e.g., in the form of a FASTQ file. The output file of the read mapping computational module is, e.g., a BAM file, being the input file for the variant calling computational module. The output file of the variant calling computational module is, e.g., a VCF file. The subsequent annotating computational module may annotate the data from the VCF file and export it in the required format, such as PDF, HTML, or the like. The file formats are merely exemplary and may be different, e.g. instead of BAM, there may be SAM or CRAM files, and the like. The data analysis pipeline in Figure 2 may also comprise computer modules which transform the input or output files from one file format into another file format.

Figure 2 also compares the prior art scenario with the scenario of the present invention. Prior art methods (referred to under "A" in Figure 2) do not provide for a sex and ancestry test. Alignment and variant calling is thus performed against the standard HRG. The method according to the present invention (referred to under "B" in Figure 2) provides a sex and ancestry test, which allows for the selection of one or more determined PHREGs. Subsequent alignment and variant calling is then performed against the determined PHREGs.

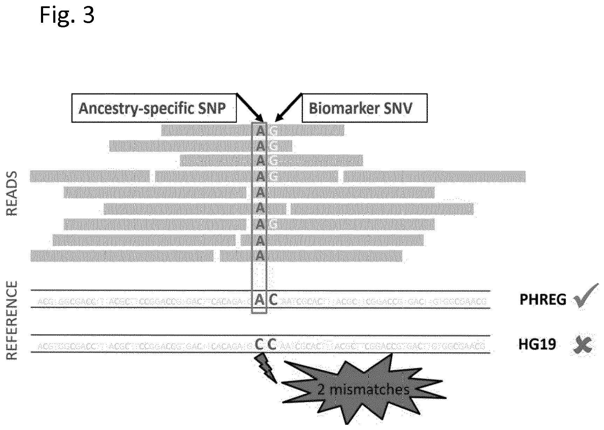

Figure 3 is a schematic representation of an exemplary read mapping step. In the example, NGS reads carry an ancestry-specific SNP "A". Ancestry-specific SNP "A" is located within the close vicinity of a previously undiscovered biomarker variant "G". The vicinity can be up to the read length.

During alignment, the NGS reads will produce 2 mismatches when they are compared against the standard HRG, namely the ancestry-specific SNP and the biomarker variant. During alignment, the NGS reads will, however, produce only one mismatch, namely the biomarker variant, when they are compared against the corresponding PHREG, as the PHREG has been modified at the ancestry-specific position, such that the PHREG is identical to the ancestry-specific SNP.

The alignment algorithm uses a scoring system that involves penalties for every mismatch and/or gap between a sequenced read and the chosen reference genome. Reads are then aligned to the best-scoring position, or may not be aligned at all due to a low overall score, or too many genomic positions with equal alignment score. Because of mismatch penalties in the alignment algorithm, reads are less likely to be aligned against the HRG than against the PHREG, especially if further variants are within read length. The reads will thus be discarded or, in the worst case, even be aligned in a wrong position of the HRG.

Thus, PHREGs have the effect that reads originating from regions of ancestry-specific variation can be rescued, especially if they carry additional variants (e.g. disease causing variants) besides the ancestry-specific variation. This allows the detection of previously undiscovered biomarkers.

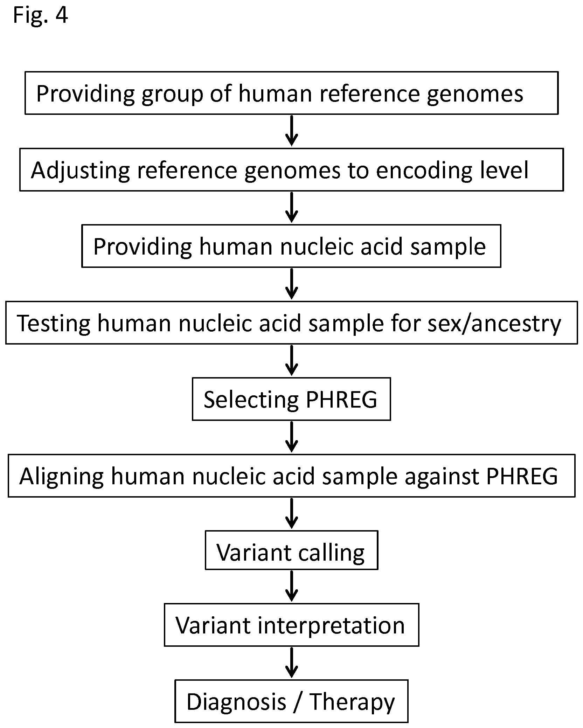

Figure 4 shows a flow chart depicting a method for genomic and/or genetic analysis of a human nucleic acid sample according to the present invention.

In a first step, a group of human reference genomes is provided to a system comprising a processing unit. To this end, a first computer module of the system may download reference genomes from a remote facility, such as an internet database. The processing unit can be any programmable computer means which substantially includes at least a processor with an internal memory such as RAM (Random Access Memory), which allows for storing and executing instructions. The processing unit has access to a non-volatile storage means in which data sets and computer files may be stored, e.g. human reference genomes, as well as clinical data and the genetic profiles of patients. The system has access to a communication network such as LAN or the internet.

In a second step, a computer module of the system adjusts the human reference genomes to an encoding level, preferably set by a user of the system. The encoding level may comprise unique nucleotide codes or ambiguous nucleotide codes. In some embodiments, four different levels of adjusting the human reference genomes to a population are proposed, wherein two of these are confined to unique nucleotide codes (A,C,G,T) and another two utilize ambiguous nucleotide encoding according to the IUPAC nomenclature, in particular maximally conservative error correction, majority allele level, non-rare alleles level and complete modelling of all observed alleles.

In a third step, a human nucleic acid sample of a patient is provided. To this end, another computer module of the system may download the raw sequence data, e.g. in the form of a FASTQ file, from a sequencing laboratory which performs the sequencing of a sample of interest on a remote platform. In an alternative embodiment, the sequencing is performed locally and the results are internally transferred. In the context of the third step, the system may also receive further clinical data of the patient from further input sources, e.g. the information about a disease the patient suffers from, information about current treatments, or the like. The clinical patient data may e.g. be directly received from the patient, e.g. may be typed on a keyboard or may be deduced from a free text typed on a keyboard, or may also be received from a multiple-choice element in a GUI. The clinical patient data may also be retrieved from an electronic health record (EHR) or from electronic medical record (EMR), possibly on a chip-card or in a database retrievable over a communication network.

In a fourth step, the human nucleic acid sample is tested for sex and/or ancestry. Again, the tests may be performed locally, or another computer module of the system may retrieve the results of the tests from an external service provider via a communication network. The sex and/or ancestry test may be performed by a second computation module or another wet lab experiment.

In a fifth step, one or more PHREGs are selected from the group of human reference genomes on the basis on the results of the sex and/or ancestry test in step. The selection may be performed by a third computation module.

In a sixth step, the human nucleic acid sample is aligned to the selected PHREGs. The alignment comprises mapping the set of reads issued from an NGS procedure to the selected PHREGs. The alignment may be performed by a fourth computation module, and the output file may be a BAM file.

In a seventh step, variant calling of the aligned human nucleic acid sample is performed with respect to the selected PHREGs. Before the variant calling takes place, a computer module of the system may re-adjust the human reference genomes to an encoding level, preferably set by a user of the system. The encoding level may comprise unique nucleotide codes or ambiguous nucleotide codes and be different from the encoding level used at the alignment step. Variants may be identified using best suited state-of-the-art algorithms. The variant calling may be performed by a fifth computation module, and the output may comprise sequence data in the form of variants with respect to the PHREG in variant call format (VCF file).

In an eighth step, variant interpretation is performed. The system may thus comprise a further post-processing computation module adapted for performing an analysis of the identified variants. In one embodiment, the post-processing module may analyze a set of genes and/or variants indicative for the presence or absence of a disease in the patient. Additionally or alternatively, the post-processing module may determine a set of treatments for the disease of the patient, taking the further clinical patient data into account, and may determine the best fitted personal treatment for the patient based on the patient's genetic data, in particular based on the identified genetic variants. In yet another embodiment, the post-processing module may perform statistics and determine mutational load, nucleotide substitution rates and hotspot mutations from the identified variations.

The variants found can also be used as an input for classifier predicting treatment efficacy or treatment safety or for classifier for diagnostic and/or therapeutic purposes.

In a ninth step, diagnostic and/or therapeutic implications may be generated and provided. To this end, the system may include an output interface in functional connection with any one of the third, fourth, fifth and the post-processing module, such that their results can be outputted. The output interface may be coupled to any display means or printers such that information calculated by the processing unit may be presented. Furthermore, there can be links to communication systems for an intranet and/or the internet such as a program for the sending and receiving of e-mails realized via an output interface.

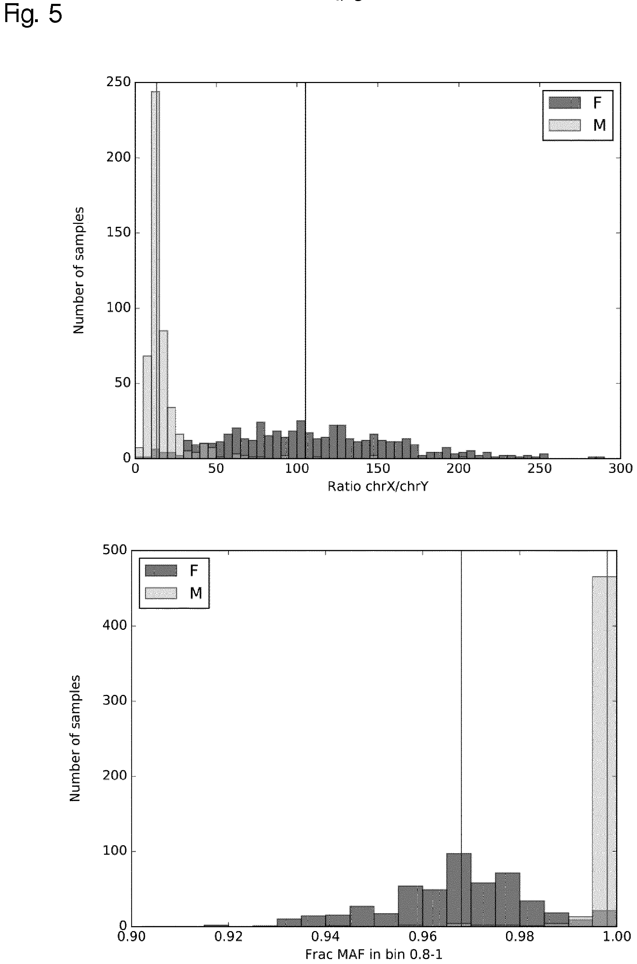

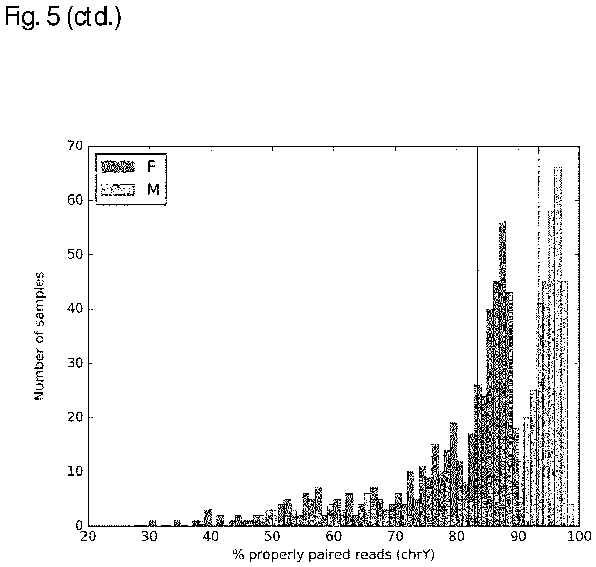

Figure 5 shows a diagram representing the distribution of chosen features for the sex classifier by class (F: female; M: male), computed on the MH Panel data. Colored vertical lines represent the class median. Top: chrX/chrY aligned read ratio; middle: for 500 common SNP positions on chrX, fraction of the major allele frequencies in bin 0.8-1.0; bottom: percentage of properly paired reads on chrY. Figure 5 should be viewed in the context of the example described below.

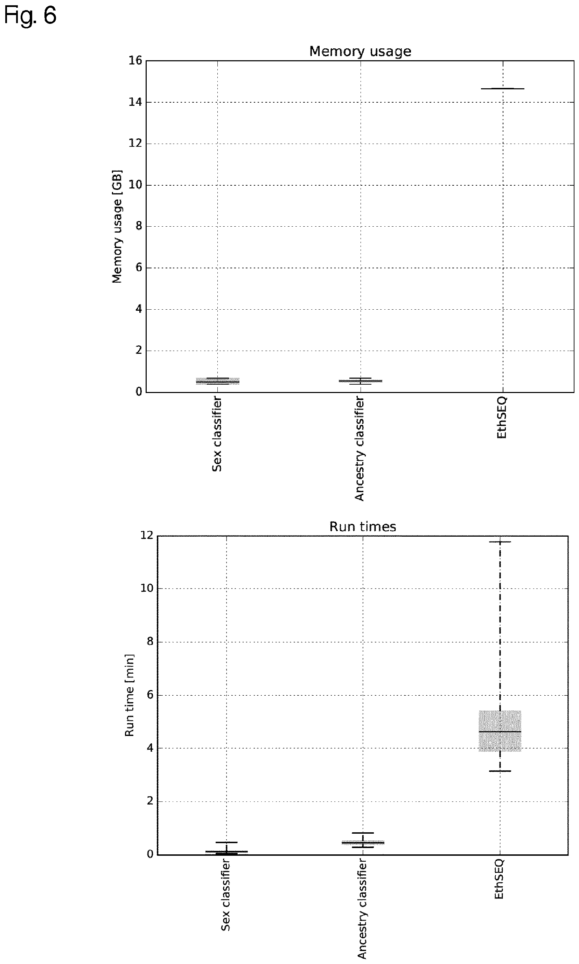

Figure 6 shows boxplots of memory usage (top, in GB) and runtime (bottom, in minutes) of the two Ansextry classifiers and EthSEQ on the 300 TCGA whole-exome samples. Figure 6 should be viewed in the context of the example described below.

### Example

AnSextry, a machine-learning based tool that derives sex and ancestry of a sample using read alignments from whole-exome sequencing data is presented. Self-declaration of both traits is known to be unreliable, and AnSextry predictions are useful both in the context of sample-swap detection and for unbiased genomic variant interpretation, especially in large cohort studies. A benchmark of AnSextry on over 1,300 samples showed high precision and low time and memory requirements.

### 1 Introduction

With the sharp drop in cost observed over the last decade, next-generation sequencing of large cohorts is becoming commonplace [Cancer Genome Atlas Research Network et al., 2013; Rand et al., 2016], and whole-exome approaches play a major role in large studies, especially in the area of precision medicine or the comprehensive characterization of disease. In this context, reliable knowledge of a sample's ancestry and sex comes with multiple benefits. First, it can be used as an easy quality control to help identifying sample swaps arising from the complex protocols and manual work involved in sample processing. Second, ancestry is crucial for the interpretation of variant impact, to circumvent the strong European bias present in most genomic studies and in the human reference genome, and to improve clinical care for people with diverse ancestries [Petrovski et al., 2016; Mersha et al., 2015; Fakhro et al., 2016]. Finally, ancestry is widely used in genetic association studies to avoid false associations with disease due to population stratification [Wu et al., 2011]. Self-declaration of sex and ancestry is often unreliable [Mersha et al., 2015; Ainsworth, 2015], calling for an identification using genomic information.

AnSextry, a machine-learning method based on logistic regression, was developed to quickly and reliably characterize sex and ancestry from whole-exome sequencing paired-end read alignments. The algorithm relies on standard file formats and can be readily integrated in an existing next-generation sequencing analysis workflow. It provides a ready-to-use model and only requires a simple BAM file as an input. In addition, its low memory requirements allow it to be run on a desktop computer. A benchmark against EthSEQ [Romanel et al., 2017], the only other whole-exome, BAM-file-based ancestry inference tool known, shows that AnSextry compares favorably in terms of precision, runtime and memory usage. No other published method for sex prediction is known to date.

### 2 Methods

### 2.1 Algorithm

A set of two classifiers was prepared, which infer the most probable sex and ancestry of an individual, based on whole-exome sequencing paired-end read alignments. This tool leveraged differences in read mapping and individual genotypes for its predictions.

The sex and the ancestry classifier were based on logistic regression using Python and the scikit-learn machine learning library. Features for both were derived from an input BAM file. Paired-end reads were aligned with BWA 0.7.15 with default settings for alignment and without post-processing steps like local realignment or duplicate removal. The GRCh37 reference genome was used without the non-chromosomal supercontigs and with masked pseudo-autosomal regions PAR1 and PAR2 to avoid alignment distortions on chromosomes X and Y. In the context of the present invention, the term "supercontigs" is generally to be understood as an ordered set of contigs, i.e. contiguous lengths of genomic sequence in which the order of bases is known to a high confidence level.

The sex classifier worked with two-class logistic regression using L1-regularization and returned a probability for each class. 5-fold cross-validation was used to determine a suitable regularization strength. The model yielding the highest area under the precision-recall curve for the training data was chosen as best model and evaluated on test data sets.

The ancestry classifier was based on multinomial logistic regression using L2-regularization and Principal Component Analysis, and returned a probability for each of the 5 continental ancestries defined in the 1000 Genomes Project: African (AFR), Ad Mixed American (AMR), East Asian (EAS), European (EUR), South Asian (SAS) (The 1000 Genomes Project Consortium et al., 2015). 5-fold cross-validation was used to determine suitable parameters. The model yielding the highest F1 score for the training data was chosen as best model and evaluated on test data sets.

### 2.2 Features

Features for the sex classifier were based on alignment differences between chromosome X and Y (Figure 5). The chrX to chrY reads ratio as well as the percentage of properly paired reads on chrY were used. In addition, the major allele frequencies at 500 common exonic SNP positions on chrX were compounded. To avoid population bias, SNPs frequent across major ancestries were chosen.

For the ancestry classifier, the genotypes of all autosomal SNPs with a genomic position within the intersection of target regions of common Agilent All Exon kits (V5, V6, V6+COSMIC) and the Molecular Health Pan-Cancer gene panel (target size 2.9 Mbp) were determined from the 1000 Genomes data described in section 2.3. Feature selection was used to retain meaningful SNPs showing variance across ancestries, resulting in 10,000 genotypes corresponding to 5,040 genomic positions used as features for the classifier. A corresponding BED file can be found in Appendix 1 and could be used to determine overlap with any targeted sequencing kit.

### 2.3 Data

In order to obtain data from diverse ancestries, genotype data from 1735 individuals from the 1000 Genomes Project phase 3 was used to train and test the ancestry classifier. Continental ancestries (AFR, AMR, EAS, EUR, SAS) were used for classification and individuals were randomly chosen to achieve balanced classes. 694 individuals were part of the test set.

Primary whole-exome control data from 300 individuals with self-reported race and sex was downloaded from TCGA (cancergenome.nih.gov) as a test set, corresponding to 3 cancer types (urothelial bladder carcinoma, lung adenocarcinoma/squamous cell lung cancer, gastric adenocarcinoma). All samples were sequenced using the Agilent SureSelect Human All Exon 50Mb kit. Records were chosen randomly to achieve balanced class sizes corresponding to TCGA categories: 150 male and 150 female individuals, as well as 100 white, 100 asian, and 100 black or African American individuals.

Targeted sequencing data from 988 cancer patients with self-reported sex, sequenced using the Molecular Health Pan-Cancer gene panel was used to train and test the sex classifier. Individuals were chosen randomly to achieve female/male class balance. 396 cases were randomly chosen as test data for the sex classifier. The 300 TCGA cases described above were used as an additional test set.

### 3 Results

### 3.1 Sex classifier

The sex classifier was trained using 592 datasets sequenced with the Molecular Health Pan-Cancer gene panel. Paired-end reads were aligned and features computed as described in the Methods section. After tuning the method with cross-validation, performance was evaluated on two test datasets: 396 individuals sequenced with the abovementioned gene panel, and 300 TCGA individuals with whole-exome data available.

On the panel test data, the sex classifier reached an average precision of 97.5%, with 10 individuals (5 male, 5 female) being misclassified (see Table 1). Misclassification was not associated with lower coverage.

**Table 1: Details on individuals sequenced with MH Panel where predicted sex did not match self-declared sex. Median coverage over all samples used was 2116x. The mean coverage of all misclassified samples was either close or above this median, showing that misprediction did not seem to be linked to lower-than-median coverage.**

| **Sex** | **Predicted sex** | **Predicted probability for true sex [%]** | **Mean coverage (with duplicates)** |
|---|---|---|---|
| F | M | 39.0 | 2579 |
| F | M | 15.3 | 2099 |
| F | M | 33.8 | 1656 |
| F | M | 17.1 | 1787 |
| F | M | 0.8 | 1797 |
| M | F | 0.0 | 6016 |
| M | F | 28.0 | 2401 |
| M | F | 0.3 | 3603 |
| M | F | 0.0 | 1606 |
| M | F | 0.0 | 1705 |

Since disorders of sex development are thought to occur with a frequency of 1% in the general population [Ainsworth, 2015], it is possible that some of the misclassified cases were actually correctly classified, but had incorrect self-declared sex.

On the TCGA test data, the sex classifier reached a precision of 100%. All 300 individuals were correctly classified. In terms of runtime and memory usage, sex prediction took less than a minute in all cases with an average memory usage of 526 MB (Figure 6).

### 3.2 Ancestry classifier

The ancestry classifier was trained on 1041 datasets from the 1000 Genomes Project. Individual genotypes were used as features, as described in 2.2. The best-performing model was evaluated on two test datasets: a remaining 694 individuals from the 1000 Genomes Project, as well as 300 TCGA individuals with sequenced whole-exomes.

The ancestry classifier reached a high average precision of 99% on the 1000 Genomes test data, performing best for Asian ancestries (100% precision both for South and East Asian), followed by African and South American ancestry (99% precision), and European ancestry (98%). In total, only 5 individuals out of 694 were misclassified.

On the 300 TCGA exome test datasets, the ancestry classifier reached a slightly lower precision of 96.33%, with a total of 11 individuals misclassified. These results were compared to EthSEQ [Romanel et al., 2017], which is currently the only other ancestry prediction method known that provides a suitable pre-computed model and is designed to work out of the box on single whole-exome BAM files. Results were highly concordant between the two methods, however, the precision reached by EthSEQ was a bit lower (94%) with a total of 18 individuals misclassified. In addition, runtime and memory required by EthSEQ were much higher: while the ancestry classifier had an average runtime of 28 seconds with 540 MB average memory usage, EthSEQ, even with the use of multithreading (4 cores), took 4.8 minutes and 14.7 GB on average (Figure 6).

As an important observation, the concordance between the two algorithms was also high for misclassified datasets: 10 out of 11 individuals whose ancestry prediction did not match the race provided by TCGA were also differently classified by EthSEQ, and in 8 out of 10 cases, both methods predicted the same ancestry. This suggests that at least a part of these individuals may have been wrongly categorized by TCGA, whose race information is based on self-declaration. 6 out of the 10 concordant cases were predicted as AFR or AMR, which is in accordance with Mersha et al. who claim that errors in self-declaration are most prevalent in African American and Latino populations. Table 2 shows misclassified individuals.

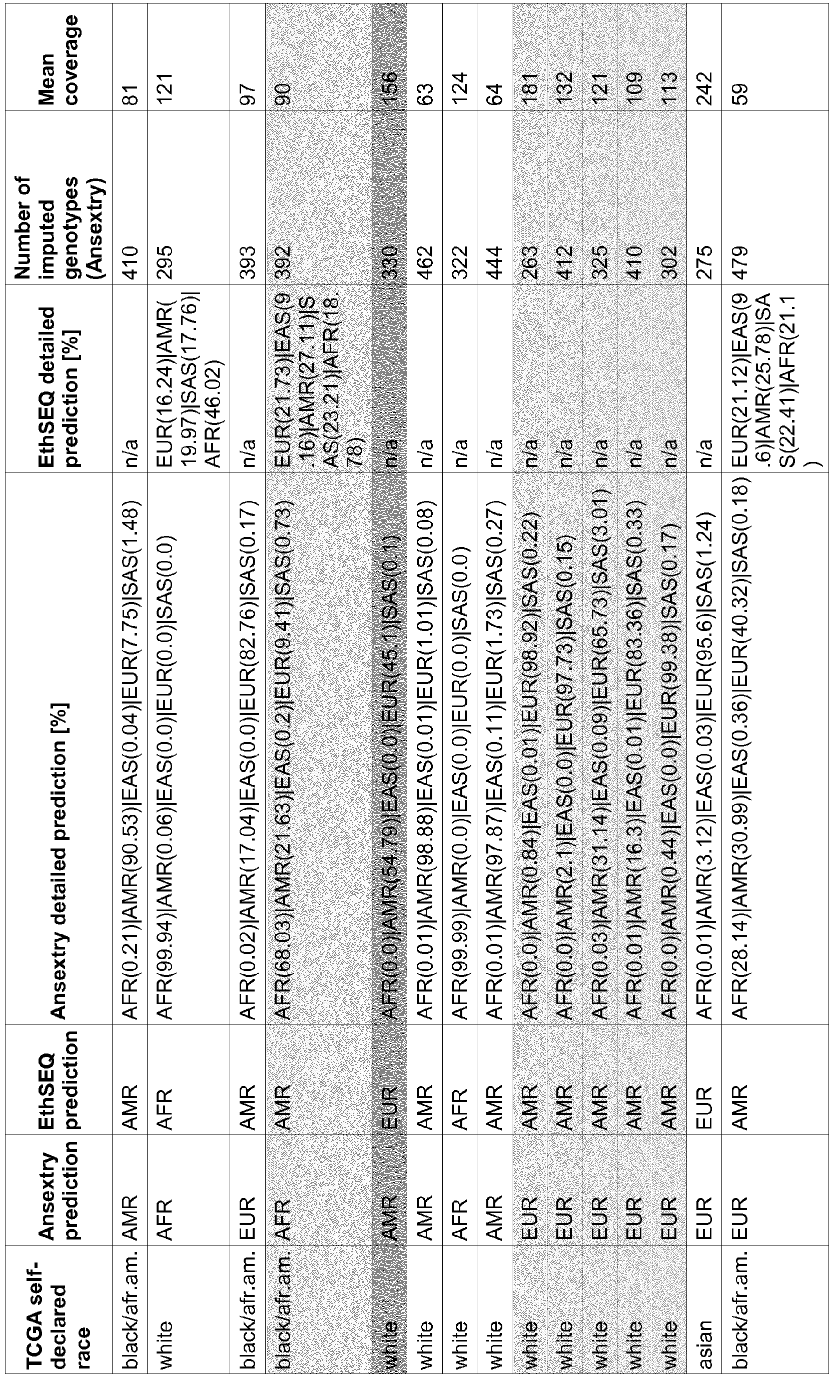

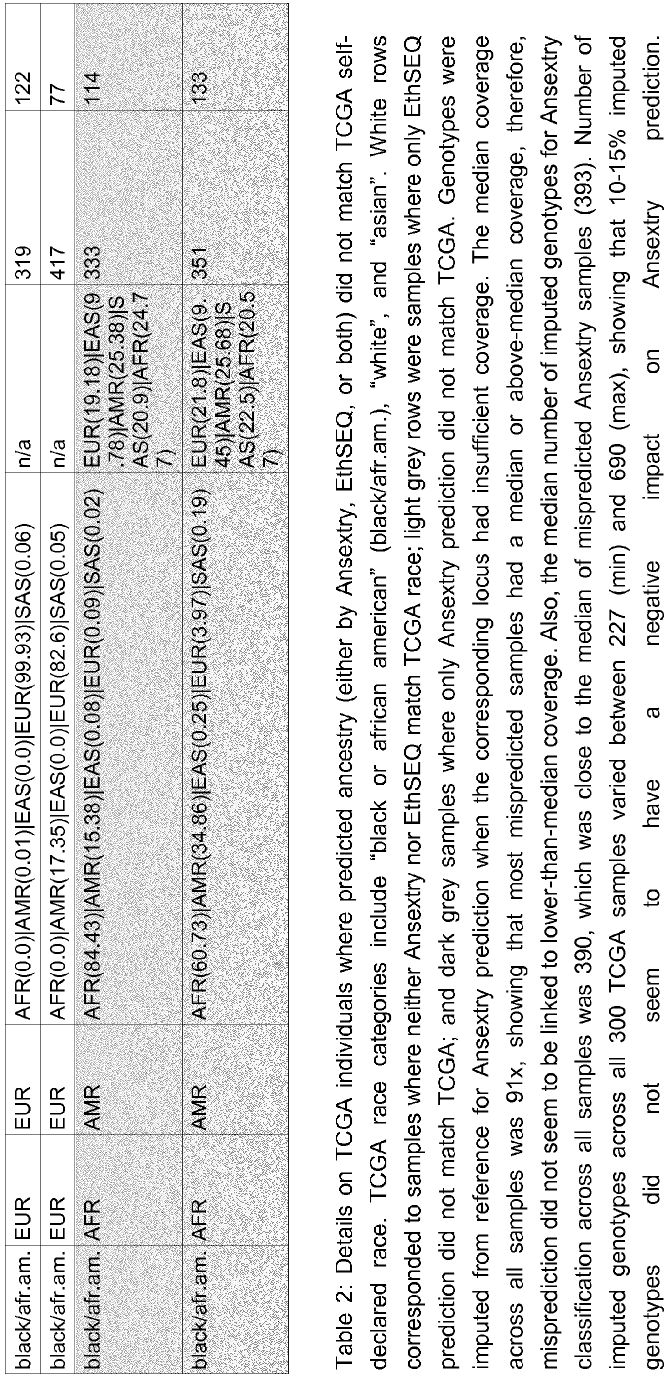

Interestingly, the only individual misclassified by AnSextry and not by EthSEQ, which was categorized as white by TCGA, but predicted as AMR by the ancestry classifier, was actually predicted to be of admixed ancestry with a probability of 54.7% AMR and 45.1% EUR.

### 4 Conclusions

AnSextry, a novel method to reliably and easily determine the sex and ancestry of an individual based on aligned paired-end reads from whole-exome or, if target size permits, targeted sequencing experiments is presented. The tool provides a set of two Python-based classifiers relying on logistic regression, and the ancestry prediction represents an alternative approach to the mainly PCA-based methods used in the field of population genetics. AnSextry provides a ready-to-use reference model and requires minimal user input. It is fast, precise, and straightforward to use.

### Disclaimer

Throughout this document the terms "ancestry-specifc" / "ethnicity-specific" / "population-specific" have been used interchangably, as different authors use different terms for the same purpose.

### References

1. Lander, E. S. et al. Initial sequencing and analysis of the human genome. Nature 409: 860-921 (2001). [PMID: 11237011]
2. Church, D. M. et al. Modernizing reference genome assemblies. PLoS Biol. 9: e1001091 (2011). [PMID: 21750661]
3. Harrow, J. et al. GENCODE: the reference human genome annotation for The ENCODE Project. Genome Res. 22: 1760-1774 (2012). [PMID 22955987]
4. ENCODE Project Consortium. An integrated encyclopedia of DNA elements in the human genome. Nature 489: 57-74 (2012). [PMID: 22955616]
5. 1000 Genomes Project Consortium et al. A global reference for human genetic variation. Nature 526: 68-74 (2015). [PMID: 26432245]
6. Li H & Durbin R. Fast and accurate short read alignment with Burrows-Wheeler transform. Bioinformatics 25: 1754-1760 (2009). [PMID: 19451168]
7. DePristo, M. A. et al. A framework for variation discovery and genotyping using next-generation DNA sequencing data. Nat. Genet. 43: 491-498 (2011). [PMID: 21478889]
8. Horton, R. et al. Variation analysis and gene annotation of eight MHC haplotypes: the MHC Haplotype Project. Immunogenetics 60: 1-18 (2008). [PMID: 18193213]
9. Pei, B. et al. The GENCODE pseudogene resource. Genome Biol. 13: R51 (2012). [PMID: 22951037]
10. Degner, J. F. et al. Effect of read-mapping biases on detecting allele-specific expression from RNA-sequencing data. Bioinformatics 25: 3207-3212 (2009). [PMID: 19808877]
11. Brandt, D. Y. C. et al. Mapping Bias Overestimates Reference Allele Frequencies at the HLA Genes in the 1000 Genomes Project Phase I Data. G3 5: 931-941 (2015). [PMID: 25787242]
12. Novak A.; Hickey G.; Garrison E.; Blum S.; Connelly A.; Dilthey A; Eizenga J.; Elmohamed M.; Guthrie S.; Kahles A.; Keenan S.;e Kelleher J.; Kural D.; Li H.; Lin M.; Miga K.; Ouyang N.; Rakocevic G.; Smuga-Otto M.; Zaranek A.; Durbin R.; McVean G.; Haussler D.; (https://www.biorxiv.org/content/biorxiv/early/2017/01/18/101378.full.pdf)
13. Paten B, Novak AM, Eizenga JM, Garrison E. Genome graphs and the evolution of genome inference. Genome Res. 5: 665-676 (2017) [PMID: 28360232]
14. Snyder M., et al. Personal genome sequencing: current approaches and challenges. Genes Dev. 5, 423-431 (2010) [PMID: 20194435]
15. Young, A.L. et al. A new strategy for genome assembly using short sequence reads and reduced representation libraries. Genome Res 2: 249-256 (2010) [PMID: 20123915]
16. Flicek, P & Birney, E. Sense from sequence reads: methods for alignment and assembly. Nat Methods. 6: S6-S12 (2009) [PMID 19844229]
17. Chen R . & Butte A.J. The reference human genome demonstrates high risk of type 1 diabetes and other disorders. Pac Symp Biocomput. 2011:231-242 (2011) [PMID: 21121051]
18. International Human Genome Sequencing Consortium. 2001. Initial sequencing and analysis of the human genome. Nature 409: 860-921 (2001) [PMID: 11237011]
19. International Human Genome Sequencing Consortium. 2004. Finishing the euchromatic sequence of the human genome. Nature 431: 931-945 (2004) [PMID: 15496913]
20. Schneider V.A. et al. Evaluation of GRCh38 and de novo haploid genome assemblies demonstrates the enduring quality of the reference assembly. Genome Res. 5:849-864. (2017) [PMID: 28396521]
21. [Editorial (October 2010). "E pluribus unum". Nature Methods. 5: 331. doi:10.1038/nmeth0510-331. (2010) [PMID: 20440876]
22. Nielsen R., Paul J. S., Albrechtsen A., Song Y. S. Genotype and SNP calling from next-generation sequencing data. Nat. Rev. Genet. 12: 443-45. (2011) [PMID: 21587300]
23. Fakhro, K. A., Staudt M. R., Ramstetter M. D., Robay A., Malek J. A., Badii R., et al. The Qatar genome: a population-specific tool for precision medicine in the Middle East. Hum. Genome Var. 3:16016 Human Genome Variation (2016) 3, 16016 doi:10.1038/hgv.2016.16; published online 30 June 2016 (2016) [PMID: 27408750]
24. Zayed H. The Qatar genome project: translation of whole-genome sequencing into clinical practice. Int J Clin Pract. 10: 832-834 doi: 10.1111/ijcp.12871. Epub 2016 Sep (2016) [PMID: 27586018]
25. Sanger F., et al. DNA sequencing with chain-terminating inhibitors. Proc Natl Acad Sci USA. 74:5463-5467. (1977) [PMID: 271968]
26. Venter, J.C. et al. The Sequence of the Human Genome. Science 291: 1304-1351. (2001) [PMID: 11181995]
27. Petrovski S & Goldstein D.B. Unequal representation of genetic variation across ancestry groups creates healthcare inequality in the application of precision medicine. Genome Biol 2016;17:157.doi: 10.1186/s13059-016-1016-y. (2016) [PMID: 27418169]
28. Koboldt DC, Ding L, Mardis ER, Wilson RK. Challenges of sequencing human genomes. Brief Bioinform. 11:484-498. (2010) [PMID: 20519329]
29. Dewey F.E., Chen R., Cordero S.P., Ormond K.E., Caleshu C., Karczewski K.J. et al. Phased whole-genome genetic risk in a family quartet using a major allele reference sequence. PLoS Genet. 2011 Sep;7(9):e1002280. doi: 10.1371/journal.pgen.1002280. Epub 2011 Sep 15. (2011) [PMID: 21935354]
30. Cao H, Wu H, Luo R, Huang S, Sun Y, Tong X et al. De novo assembly of a haplotype-resolved human genome. Nat Biotechnol 33: 617-622. (2015) [PMID: 26006006]
31. Wu L., Yavas G., Hong H., et al. Direct comparison of performance of single nucleotide variant calling in human genome with alignment-based and assembly-based approaches. Sci Rep. 2017 Sep 8;7(1):10963. doi: 10.1038/s41598-017-10826-9. (2017) [PMID: 28887485]
32. Meyer, L. R. et al. The UCSC Genome Browser database: extensions and updates 2013. Nucleic acids research 41: D64-D69 (2013). [PMID: 23155063]
33. Sudmant, P. H. et al. An integrated map of structural variation in 2,504 human genomes. Nature 526: 75-81 (2015). [PMID: 26432246]
34. Iqbal, Z., Caccamo, M., Turner, I., Flicek, P. & McVean, G. De novo assembly and genotyping of variants using colored de Bruijn graphs. Nature genetics 44: 226-232 (2012). [PMID: 22231483]
35. Cornish-Bowden A. (1985). Nomenclature for incompletely specified bases in nucleic acid sequences: recommendations 1984. Nucleic Acids Res. 13: 3021-3030. (1985) [PMID: 2582368]
36. Mersha T. B., & Abebe T. Self-reported race/ethnicity in the age of genomic research: its potential impact on understanding health disparities. Hum. Genomics 9:1. (2015) [PMID: 25563503]
37. Baye T. M. Inter-chromosomal variation in the pattern of human population genetic structure. Hum Genomics 5:220-240. (2011) [PMID: 21712187]
38. Fondevila M. et al. Revision of the SNPforlD 34-plex forensic ancestry test: Assay enhancements, standard reference sample genotypes and extended population studies. Forensic Sci Int Genet 7: 63-74. (2013) [PMID: 22749789]
39. Ainsworth C. Sex redefined. Nature 518: 288-291. doi: 10.1038/518288a. (2015) [PMID: 25693544]
40. Gall J. G., Pardue M.L. Formation and detection of RNA-DNA hybrid molecules in cytological preparations. Proc. Natl. Acad. Sci. USA 63, Nr. 2, 1969, S. 378-383, [PMID 4895535].
41. Kallioniemi A. et al. Comparative genomic hybridization for molecular cytogenetic analysis of solid tumors. Science Band 258, Nr. 5083, 1992, S. 818-821*.*
42. Goodwin S., McPherson JD, McCombie WR. Coming of age: ten years of next-generation sequencing technologies. Nat. Rev. Genet. 2016 May 17;17(6):333351
43. Al-Ali M, Osman W., Tay G.K., AlSafar H.S. A 1000 Arab genome project to study the Emirati population. J. Hum. Genet. 63(4): 533-536 (2018). [PMID: 29410509]
**44.** Cancer Genome Atlas Research Network et al. The Cancer Genome Atlas Pan-Cancer analysis project. Nat. Genet., 45(10), 1113-1120 (2013).
45. Rand,K.A. et al. Whole-exome sequencing of over 4100 men of African ancestry and prostate cancer risk. Hum. Mol. Genet., 25(2), 371-381 (2016).
46. Wu,C. et al. A Comparison of Association Methods Correcting for Population Stratification in Case-Control Studies. Ann. Hum. Genet., 75(3), 418-427 (2011).
47. Romanel,A. et al. EthSEQ: ethnicity annotation from whole exome sequencing data. Bioinformatics, 33(15), 2402-2404 (2017).

### Appendix 1

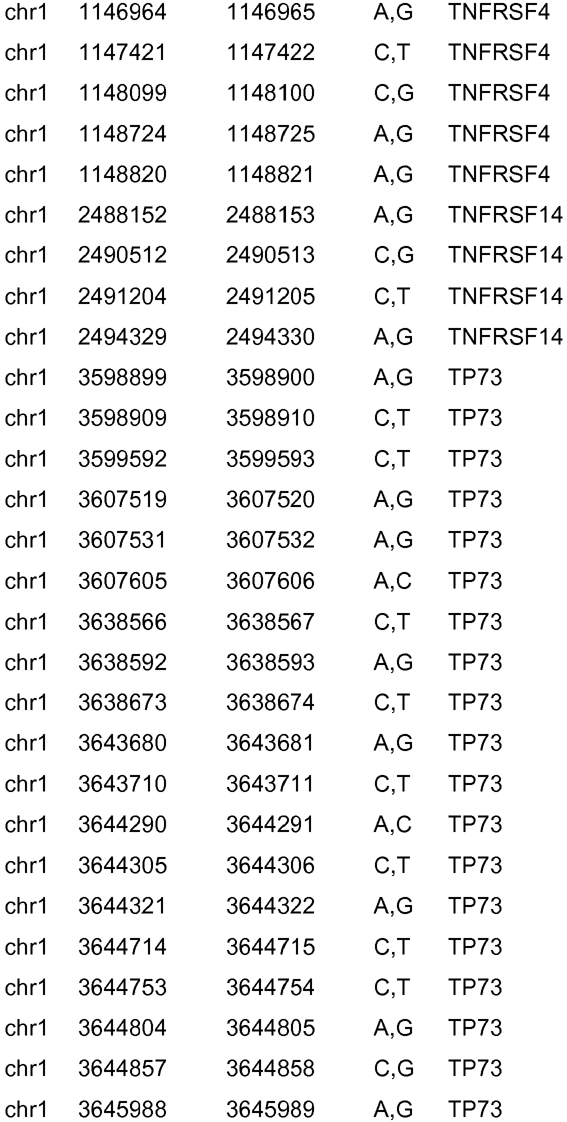

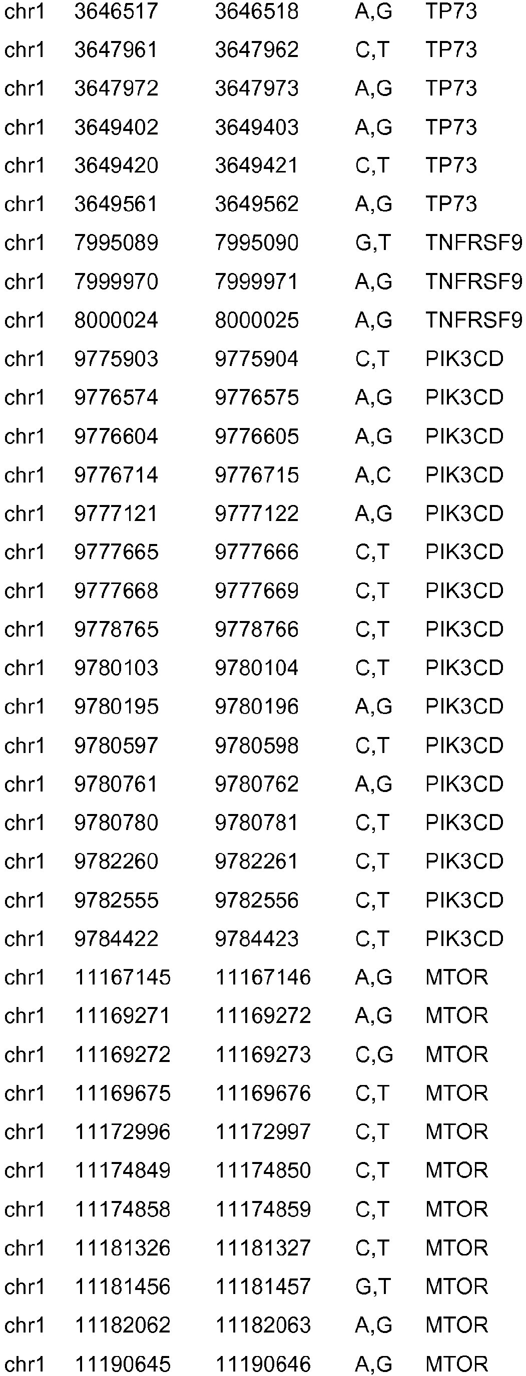

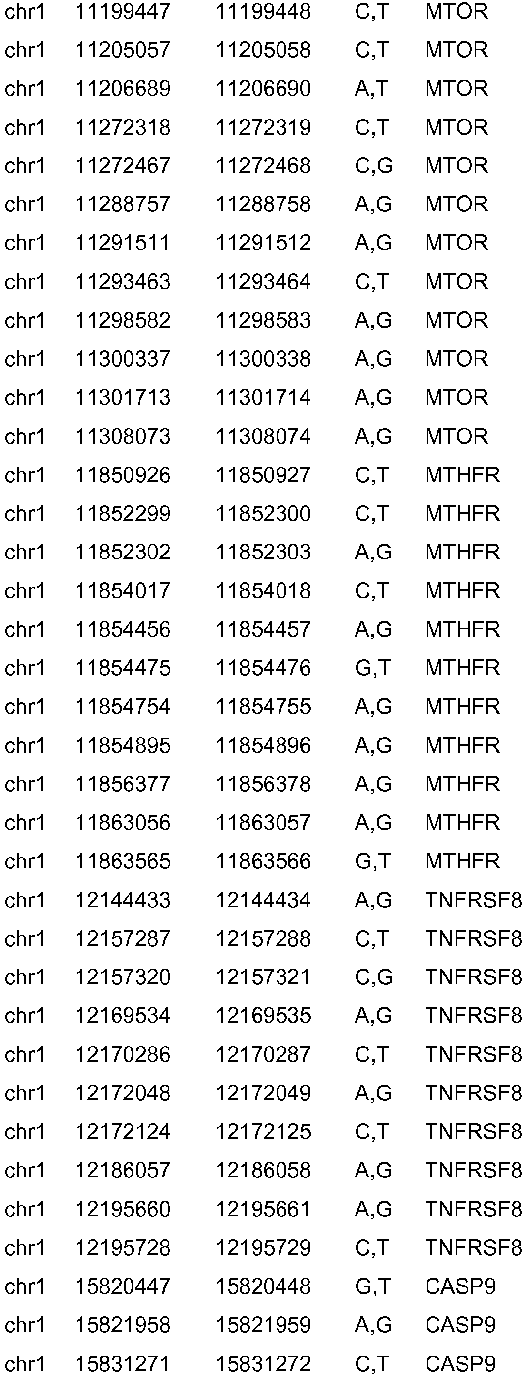

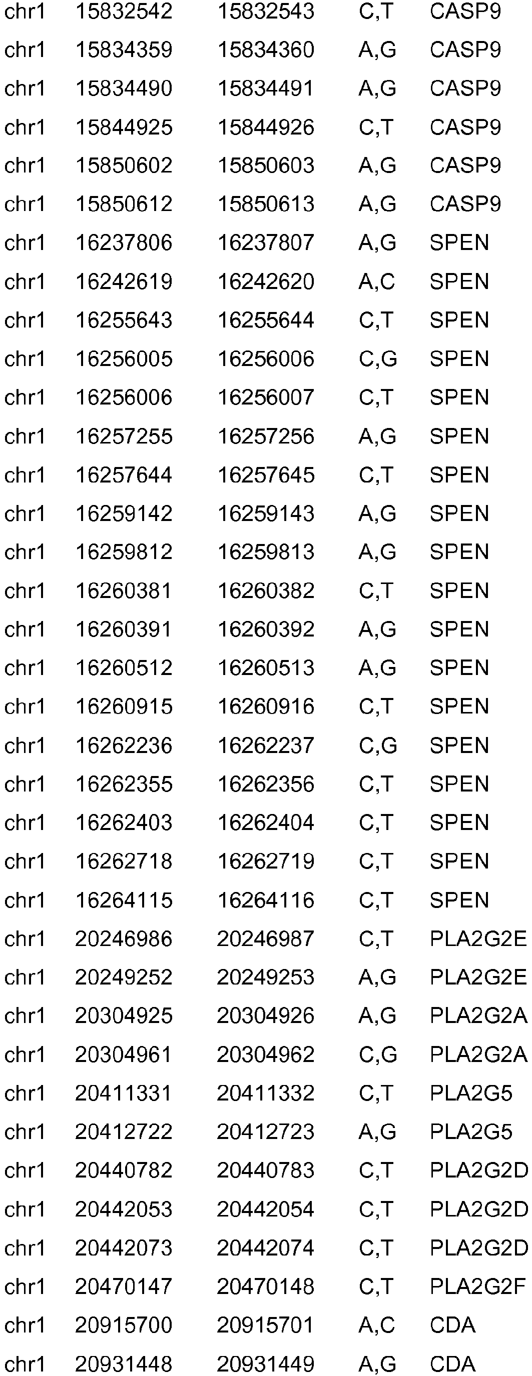

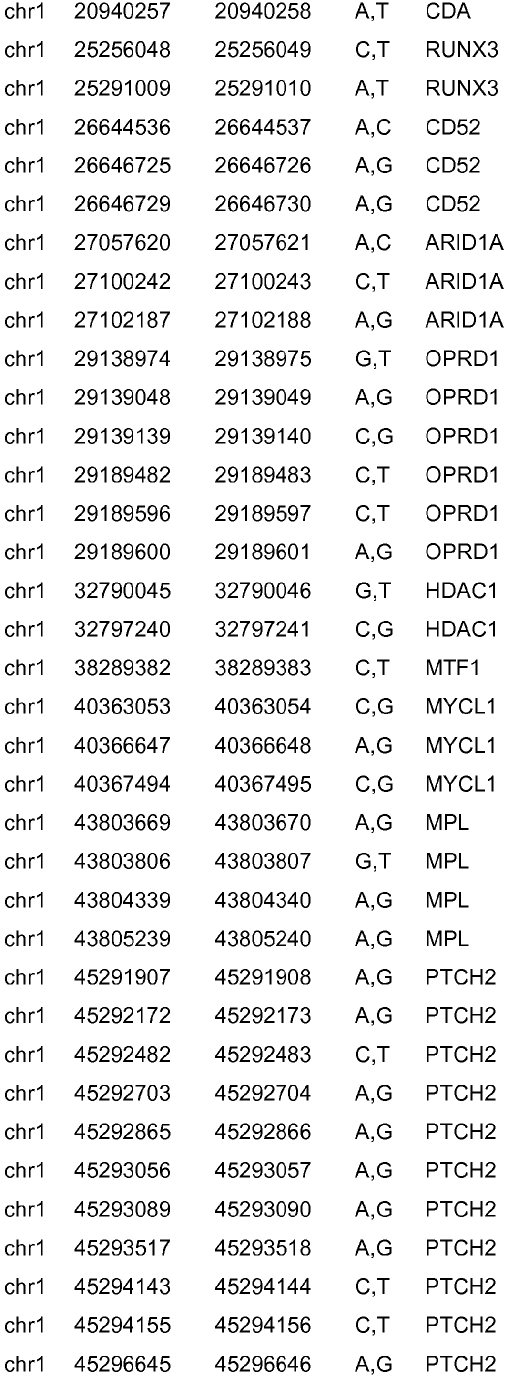

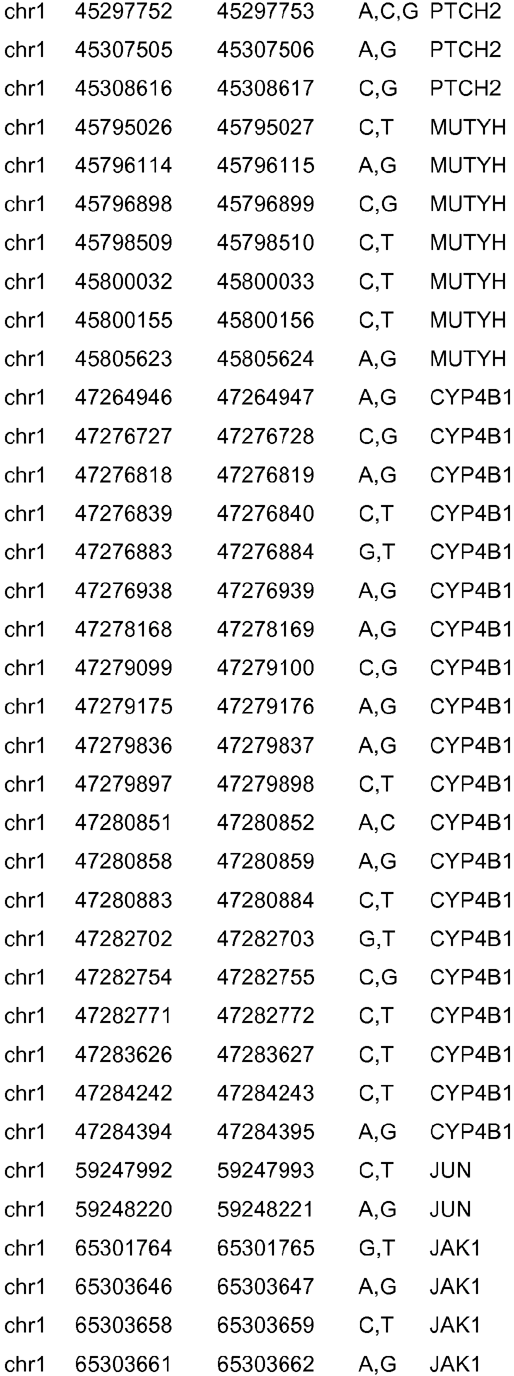

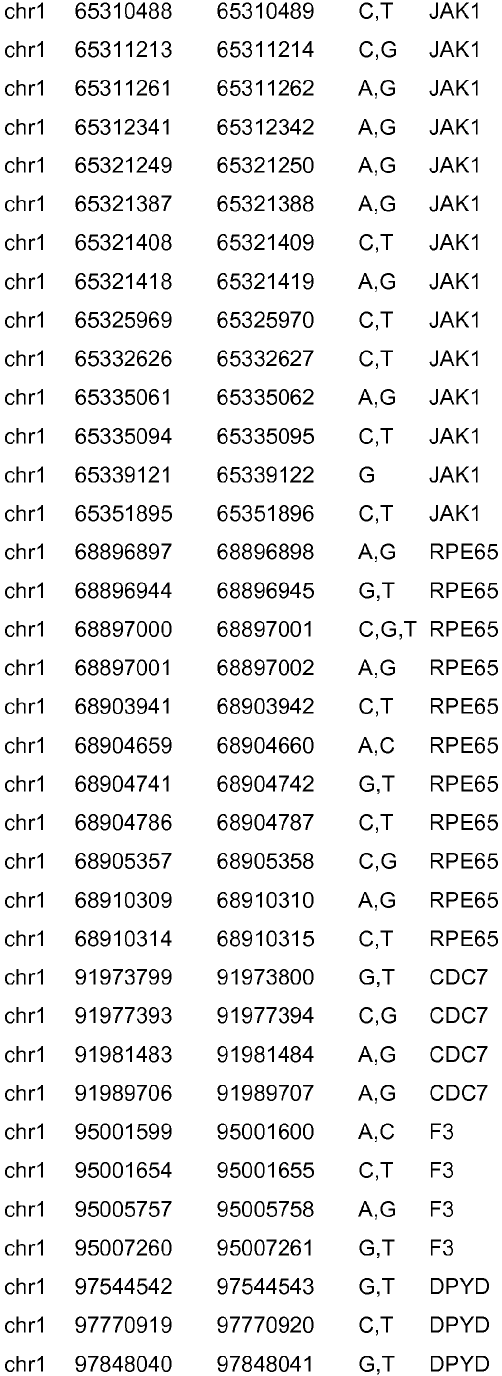

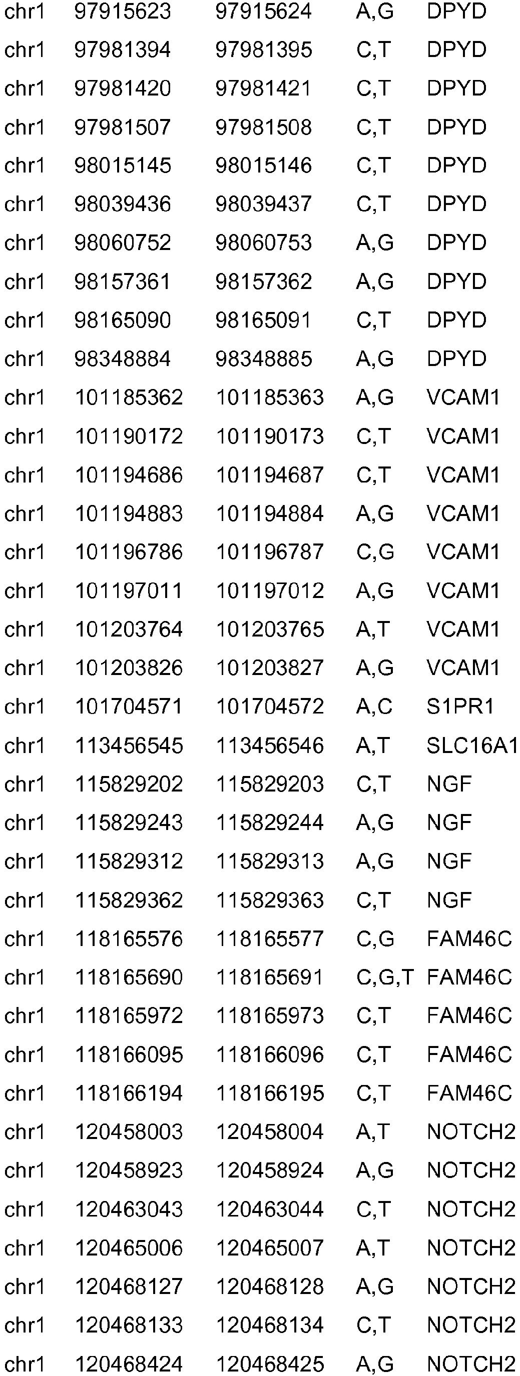

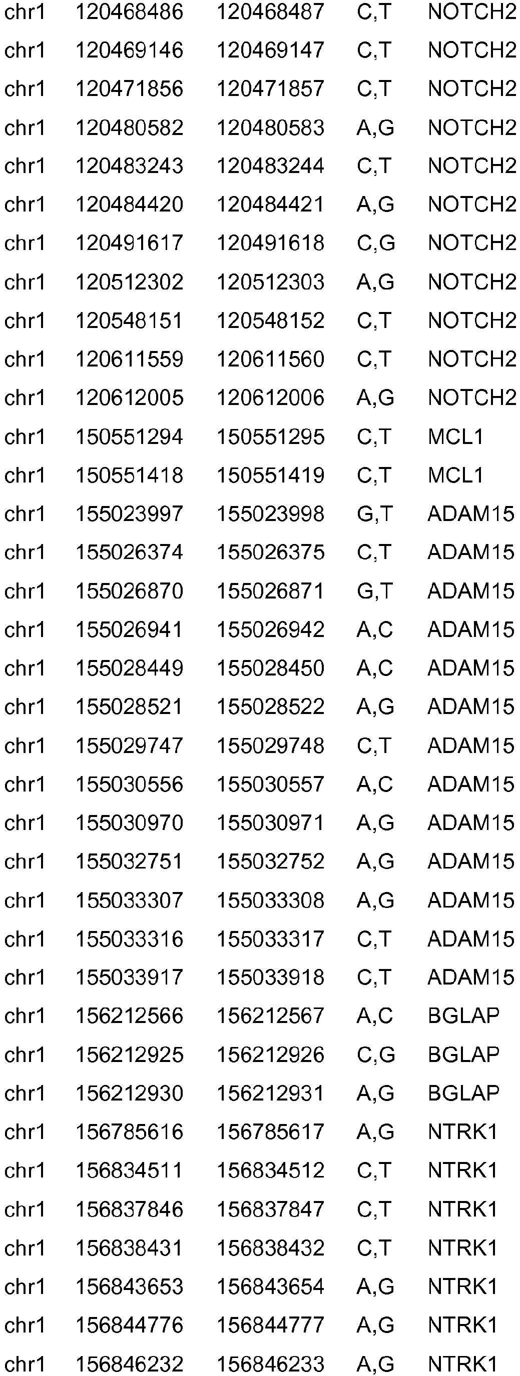

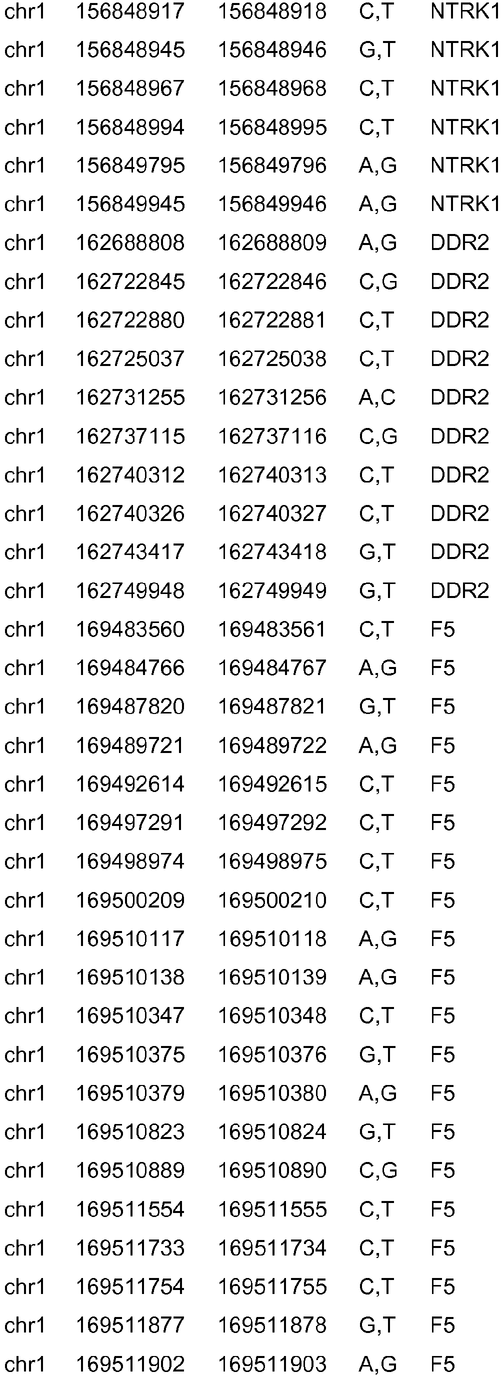

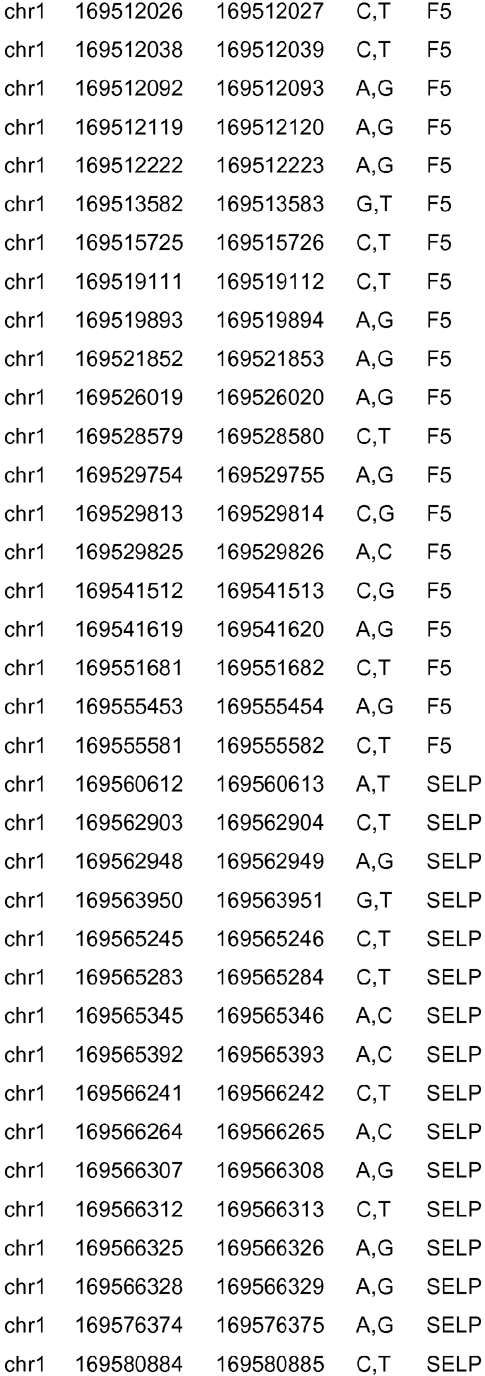

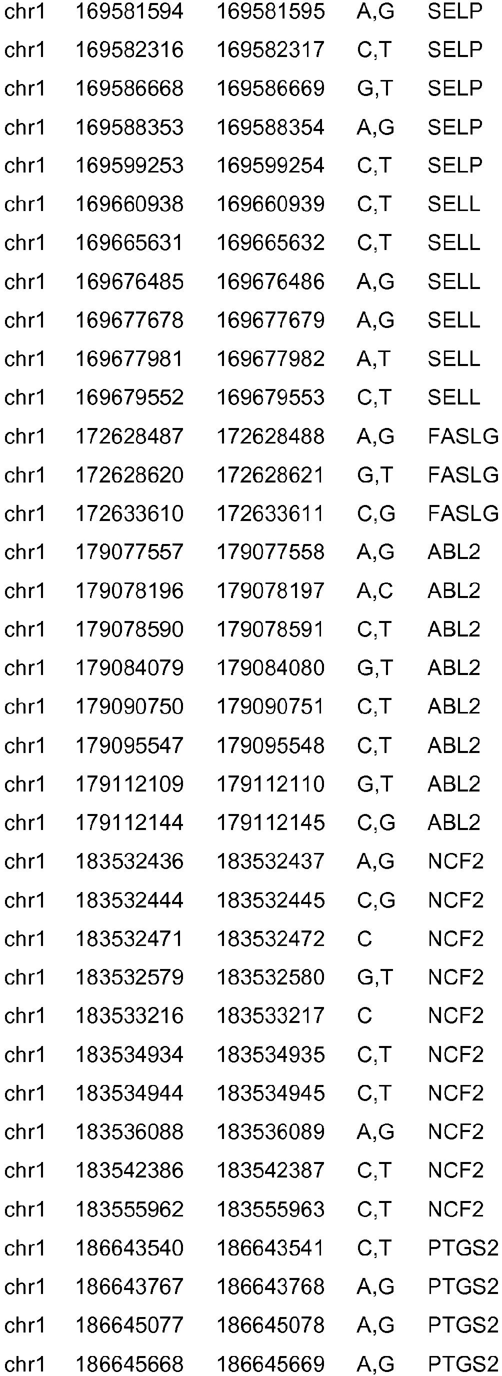

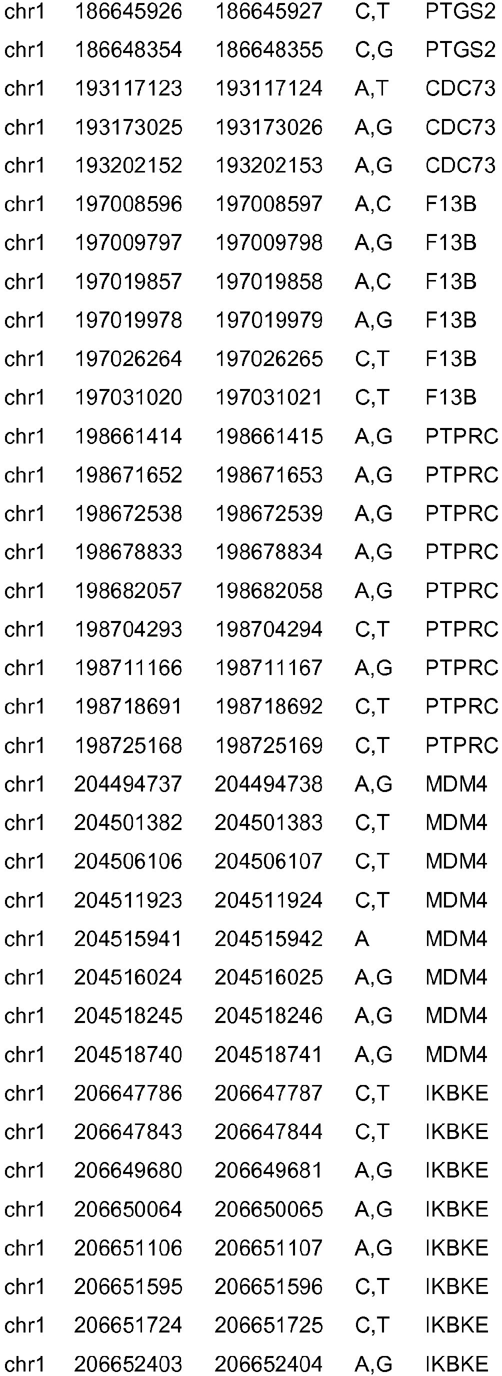

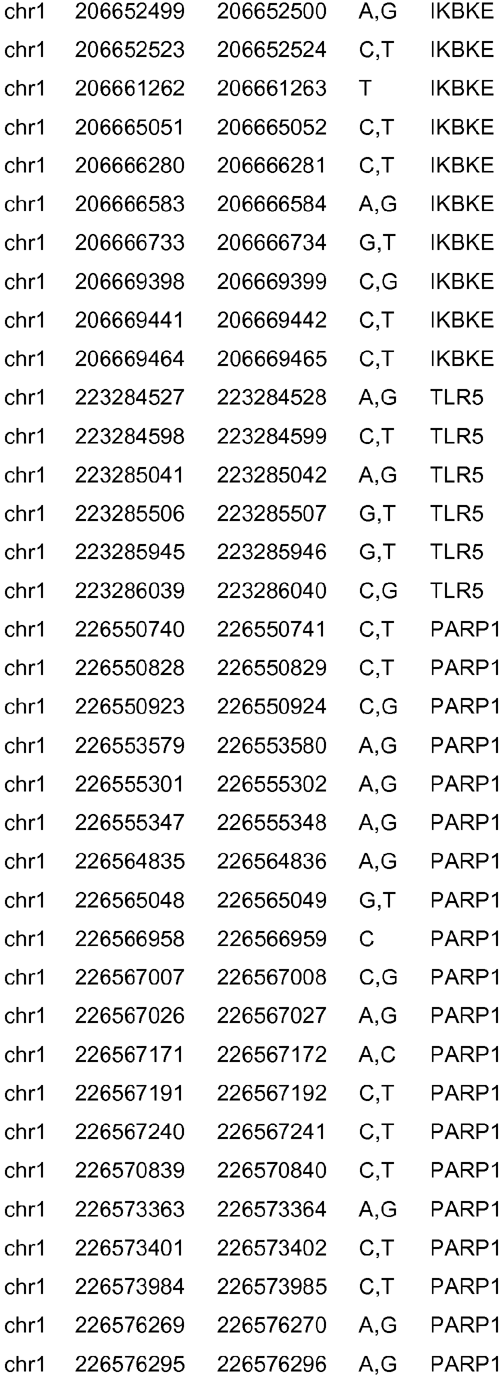

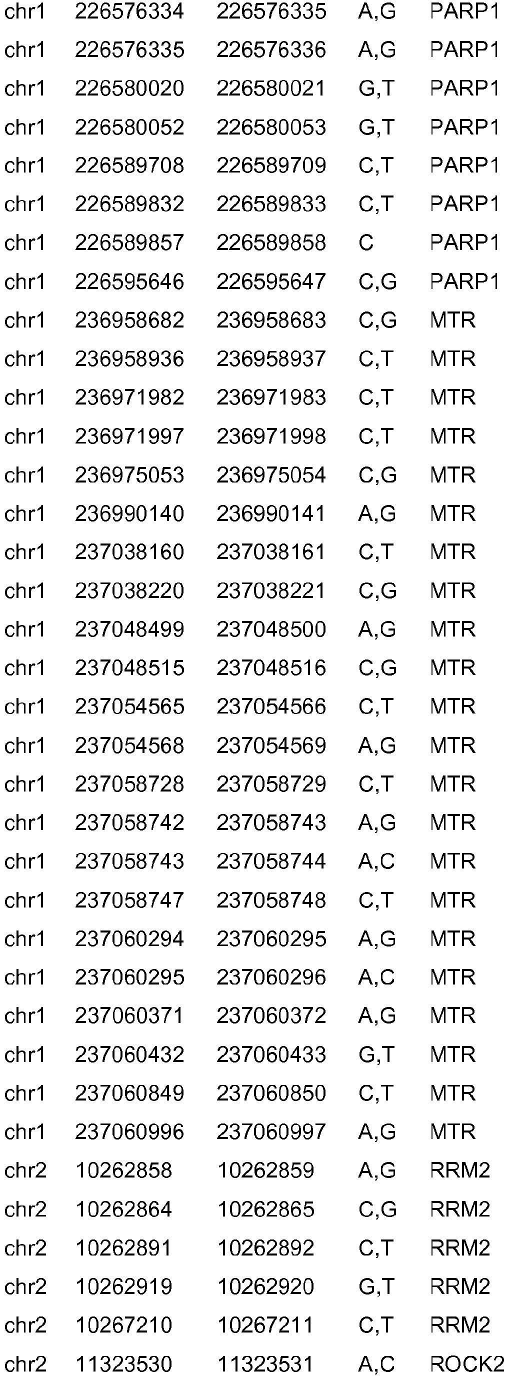

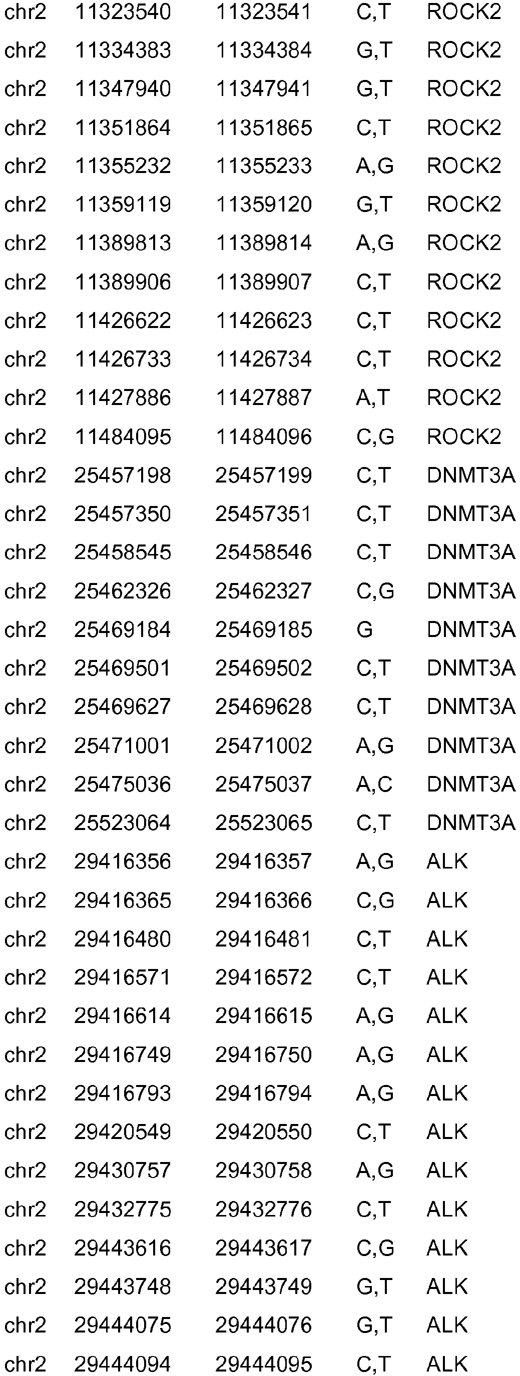

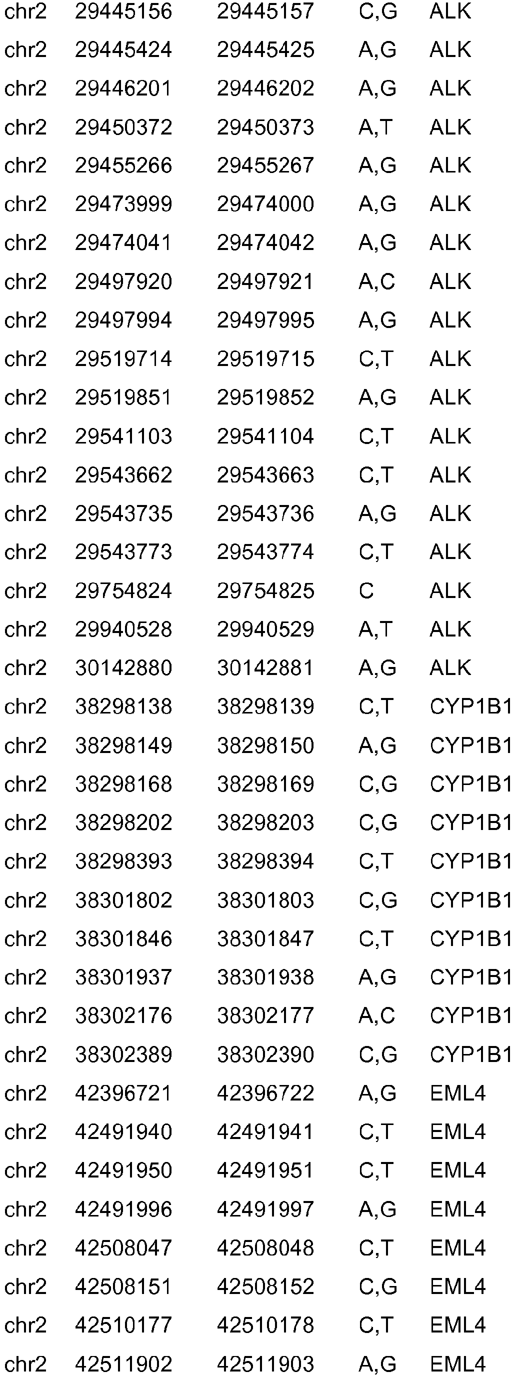

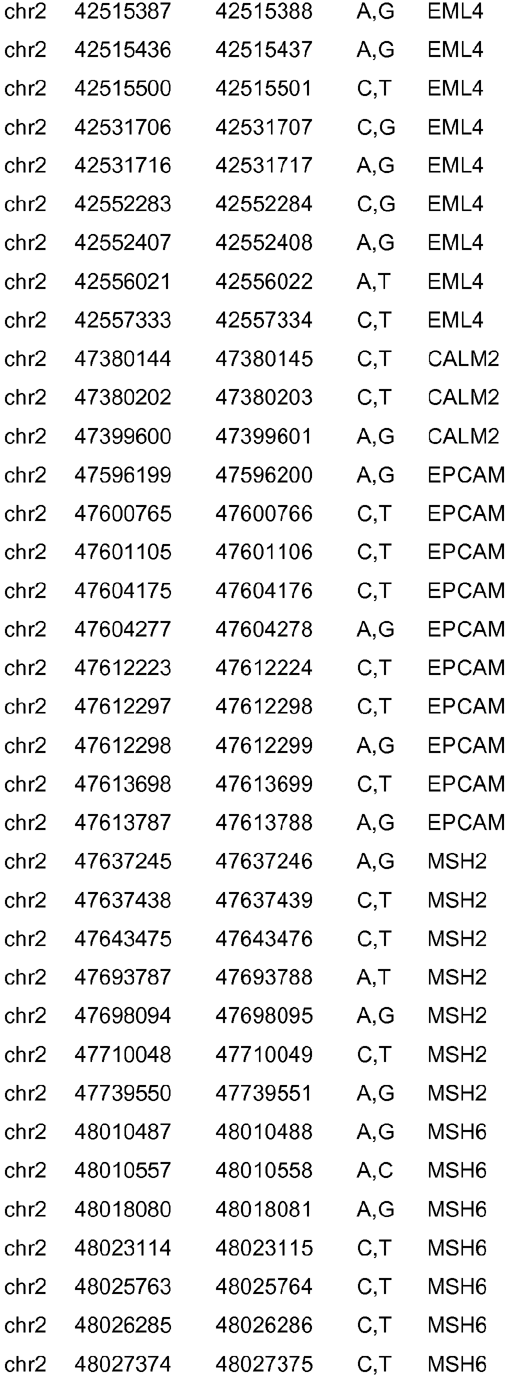

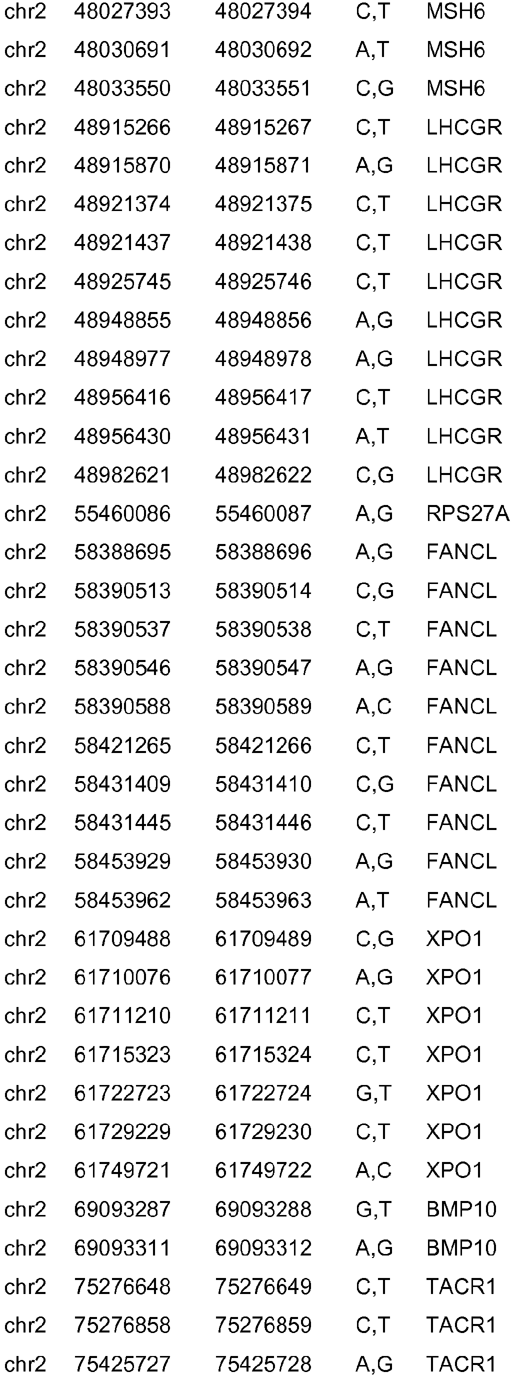

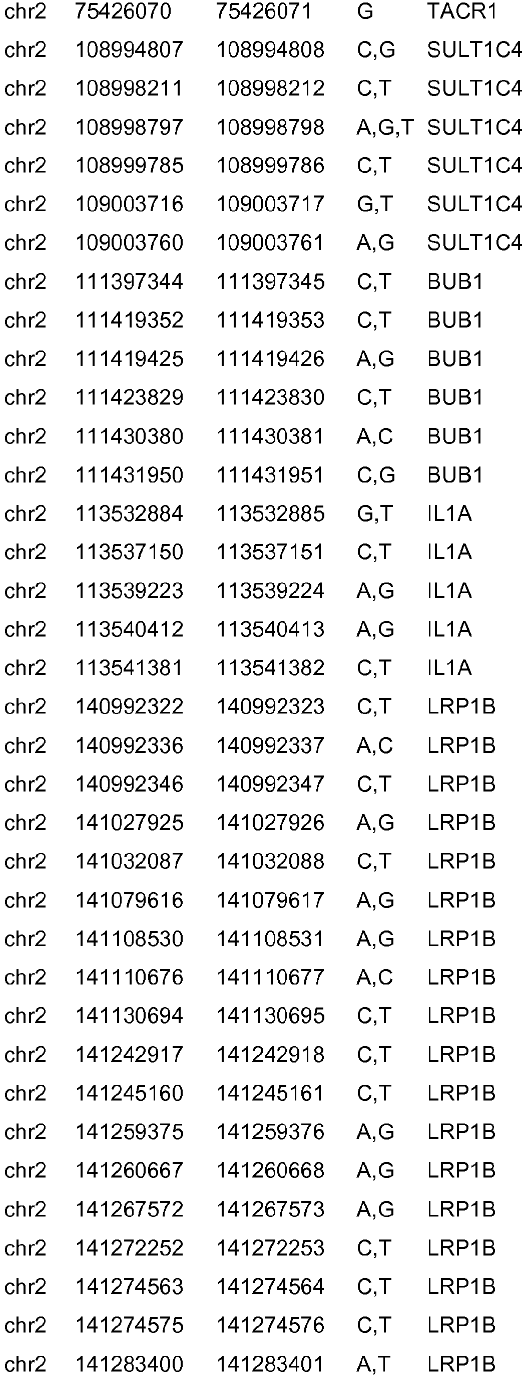

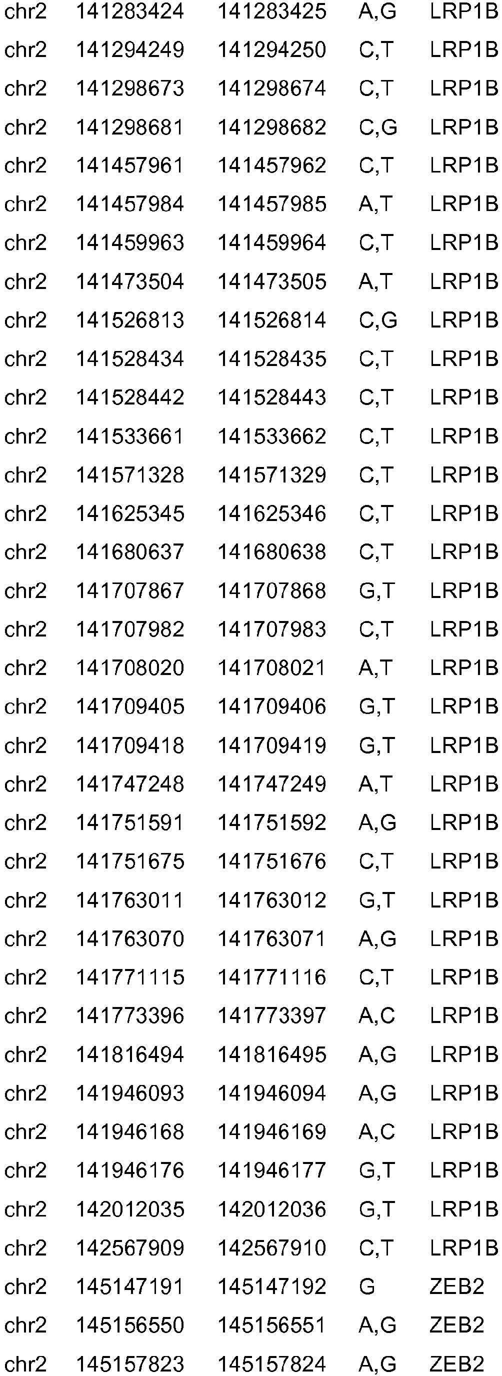

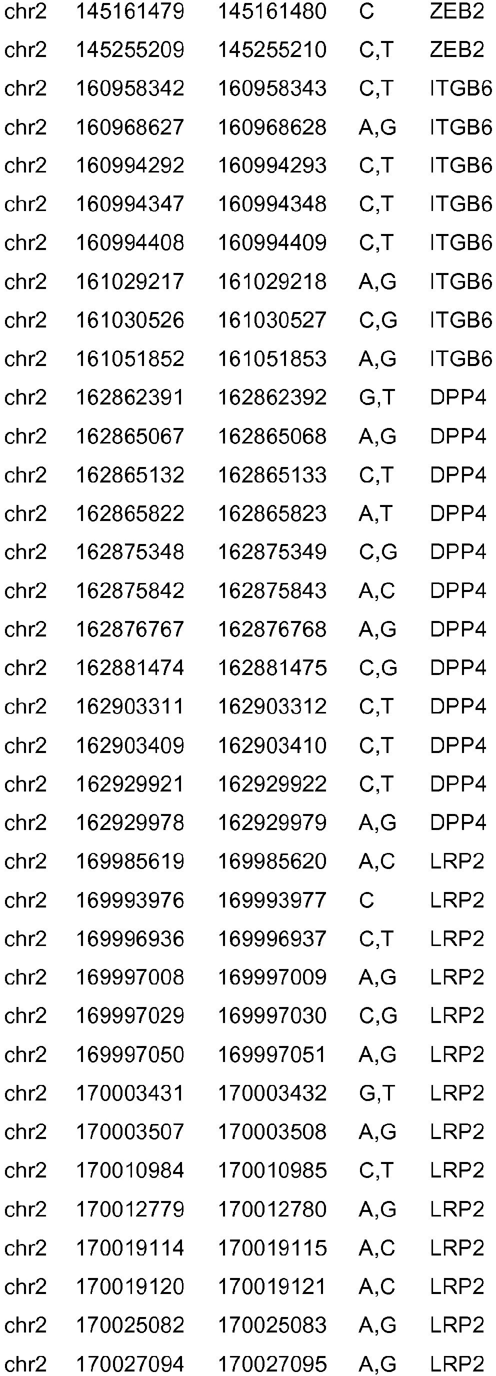

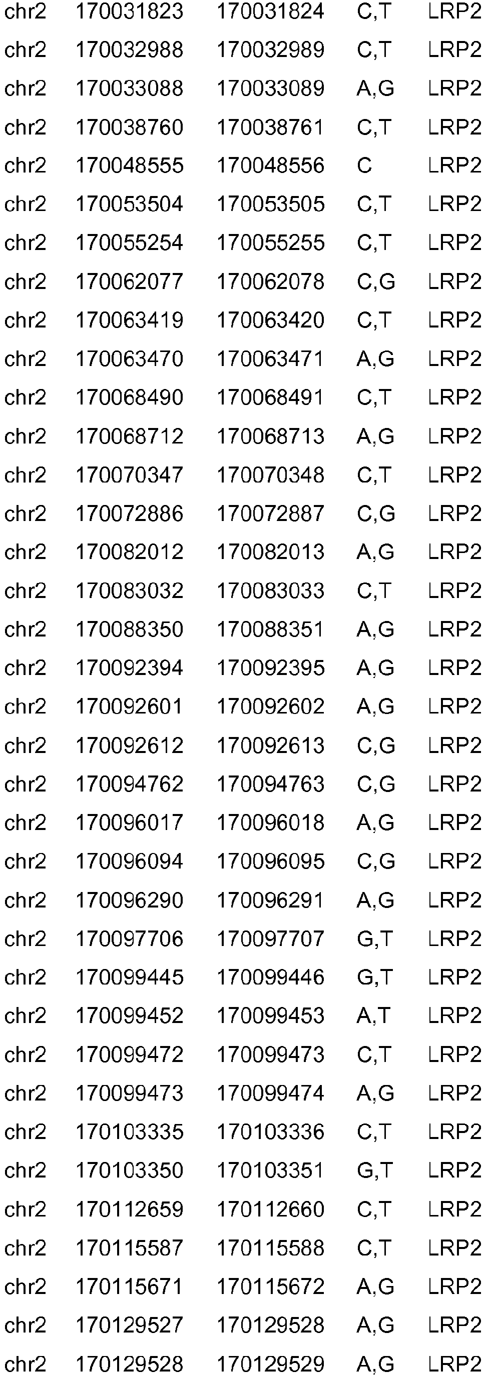

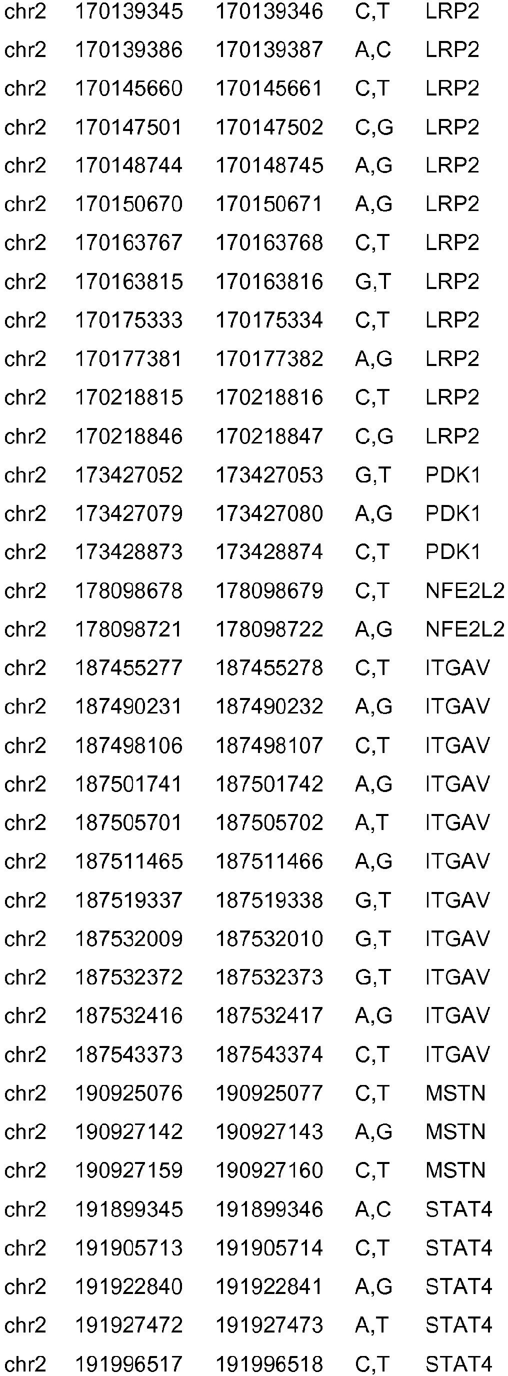

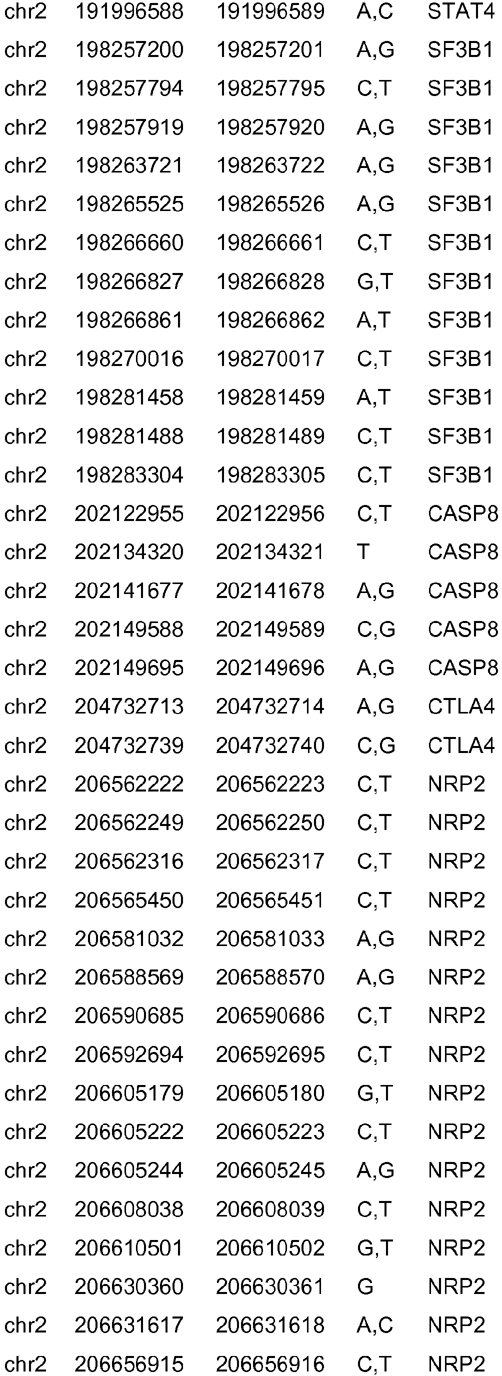

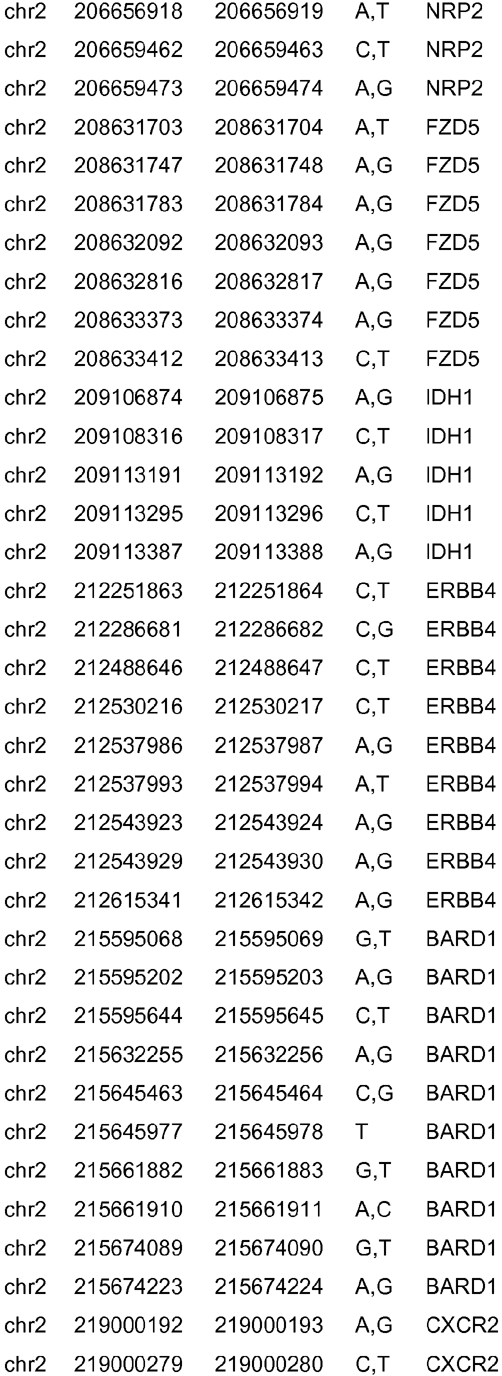

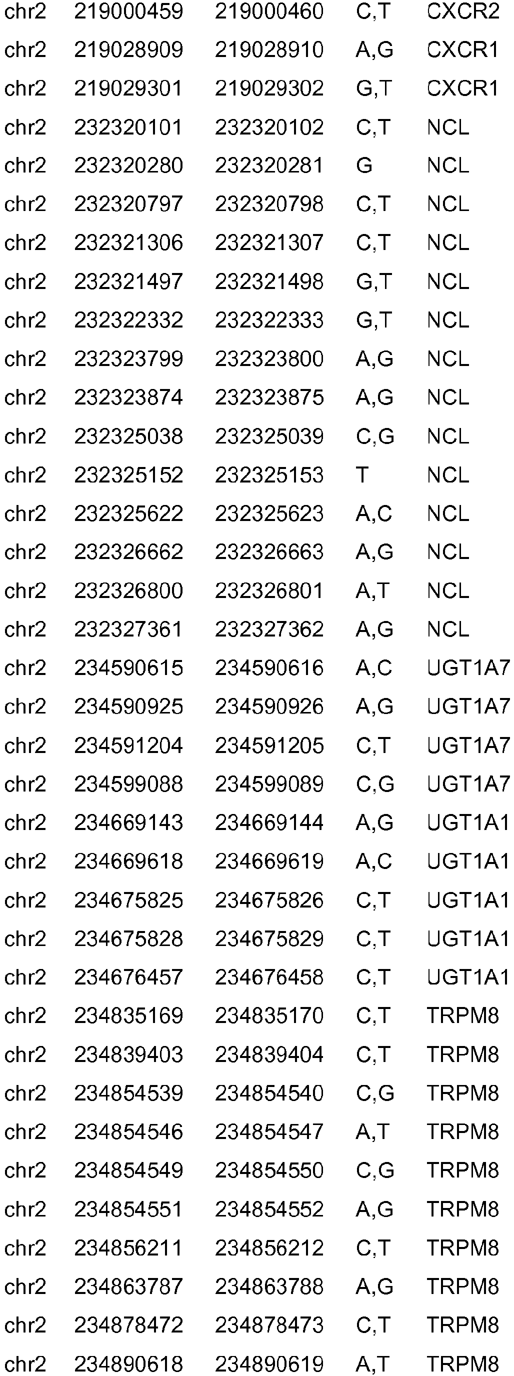

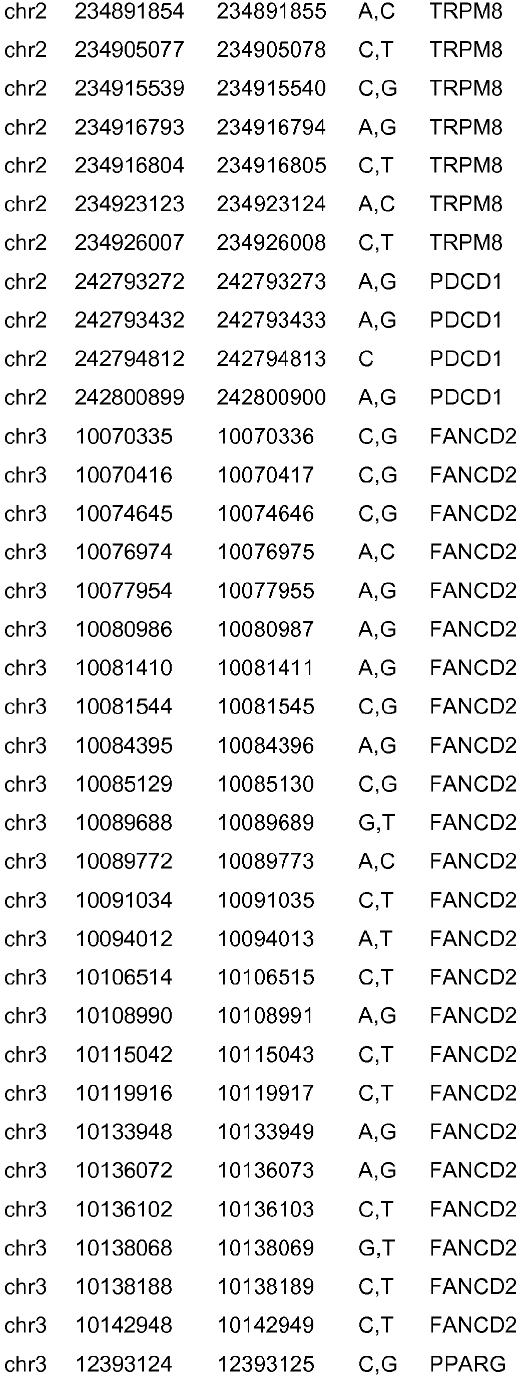

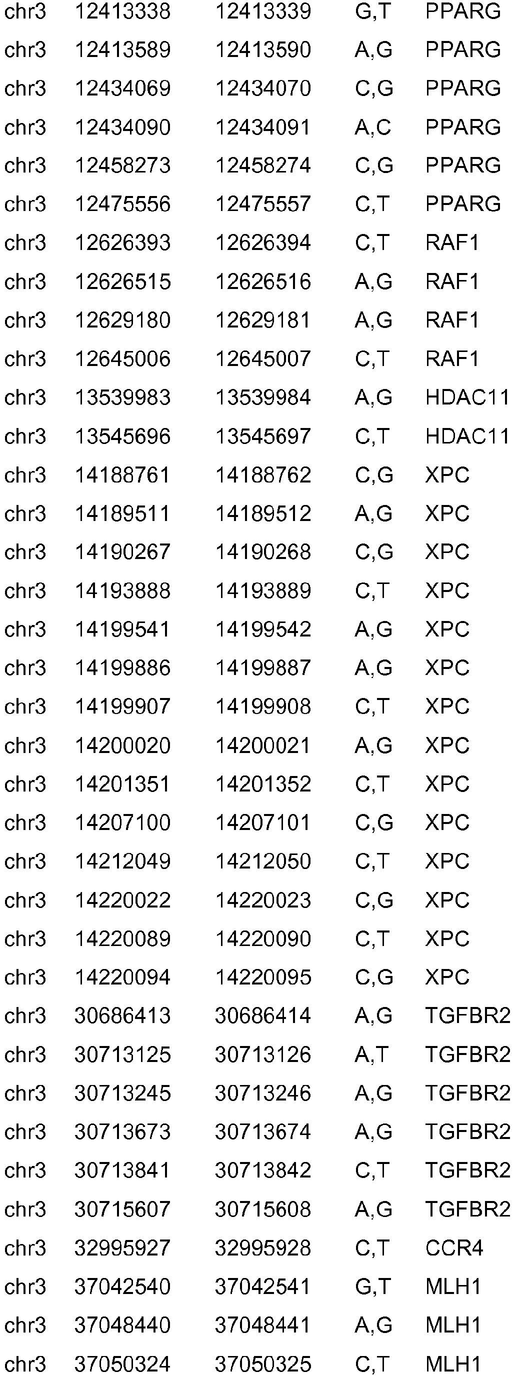

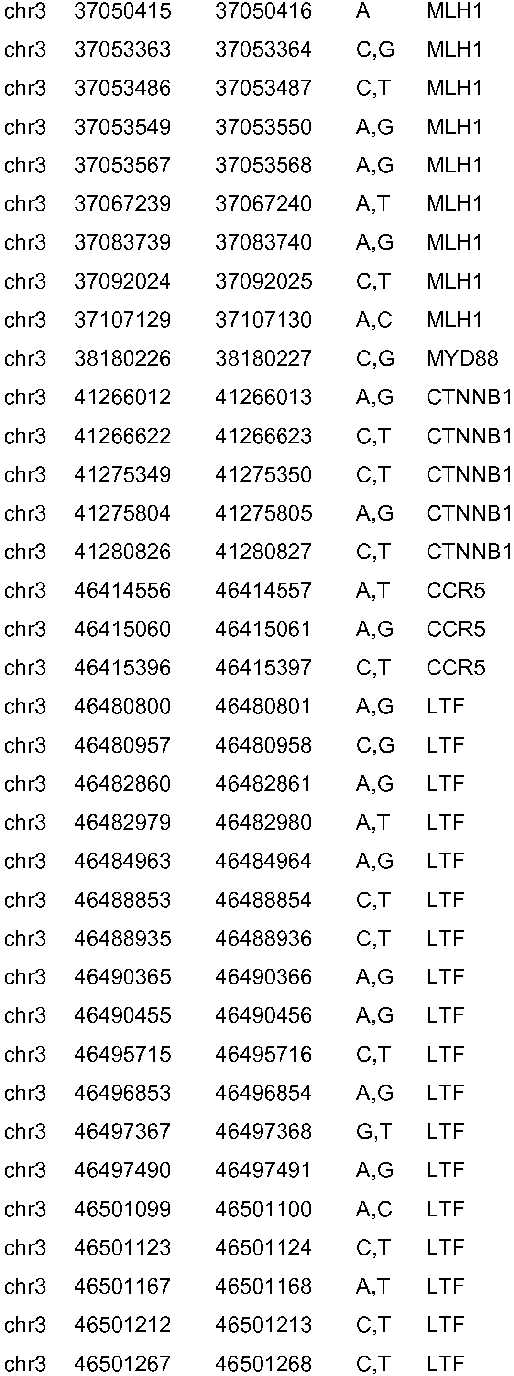

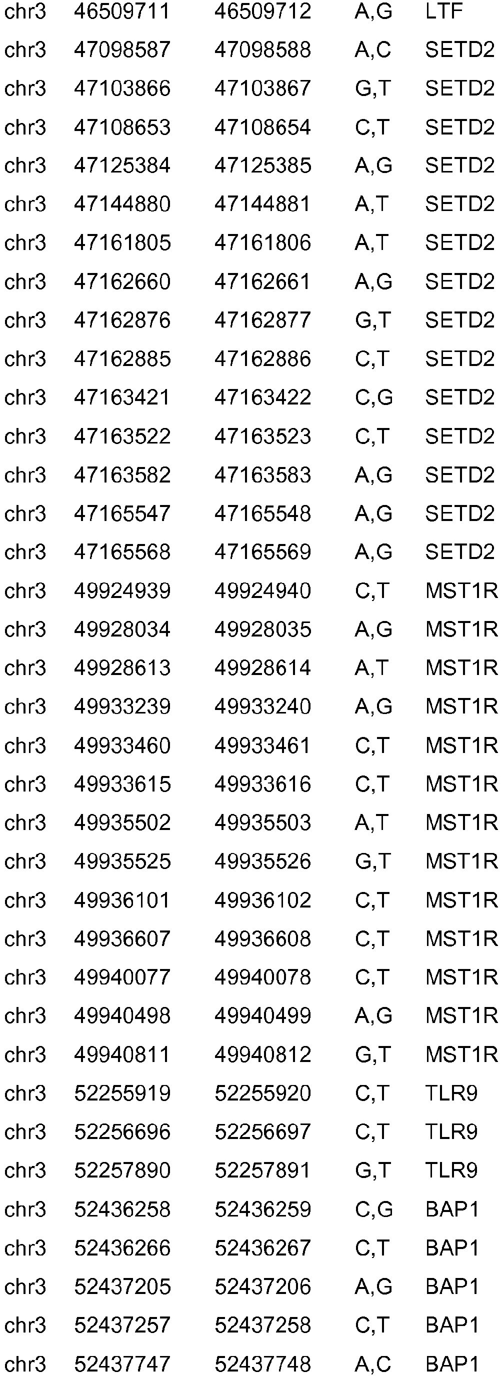

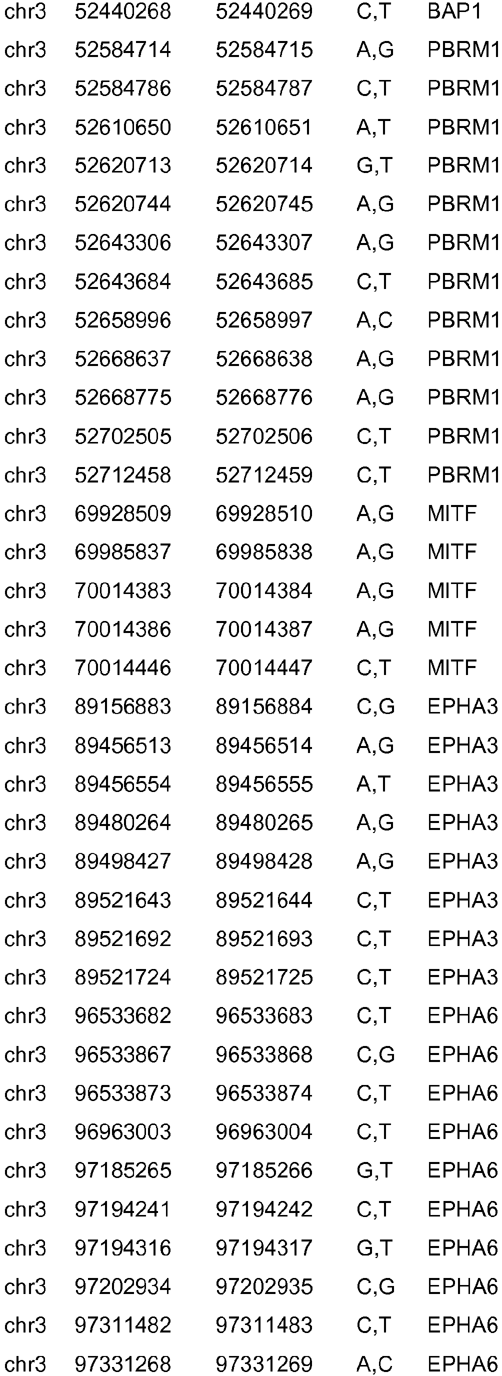

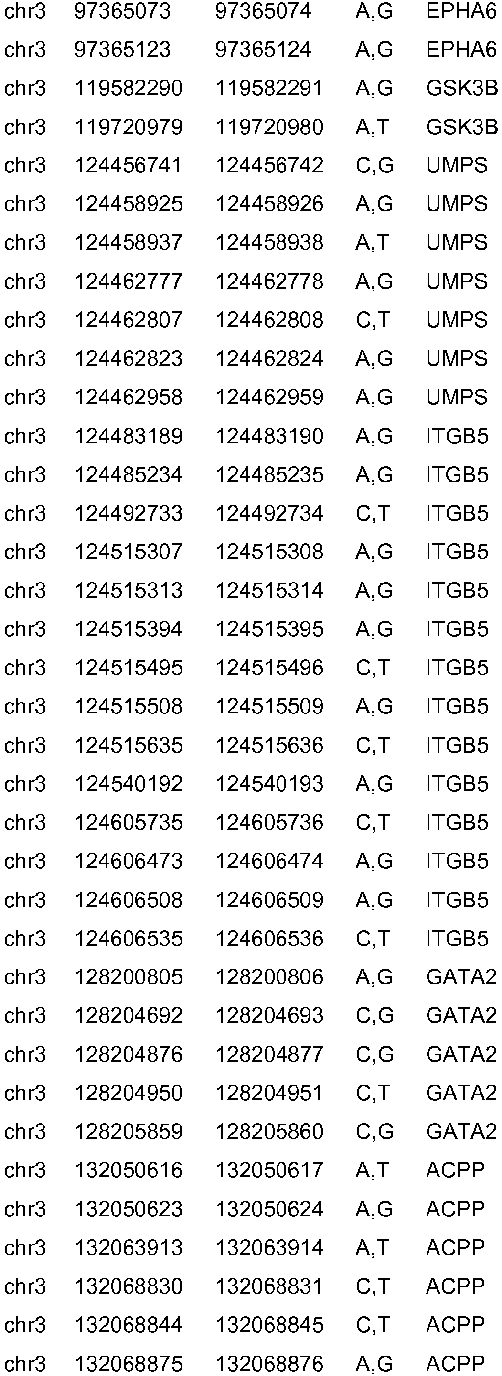

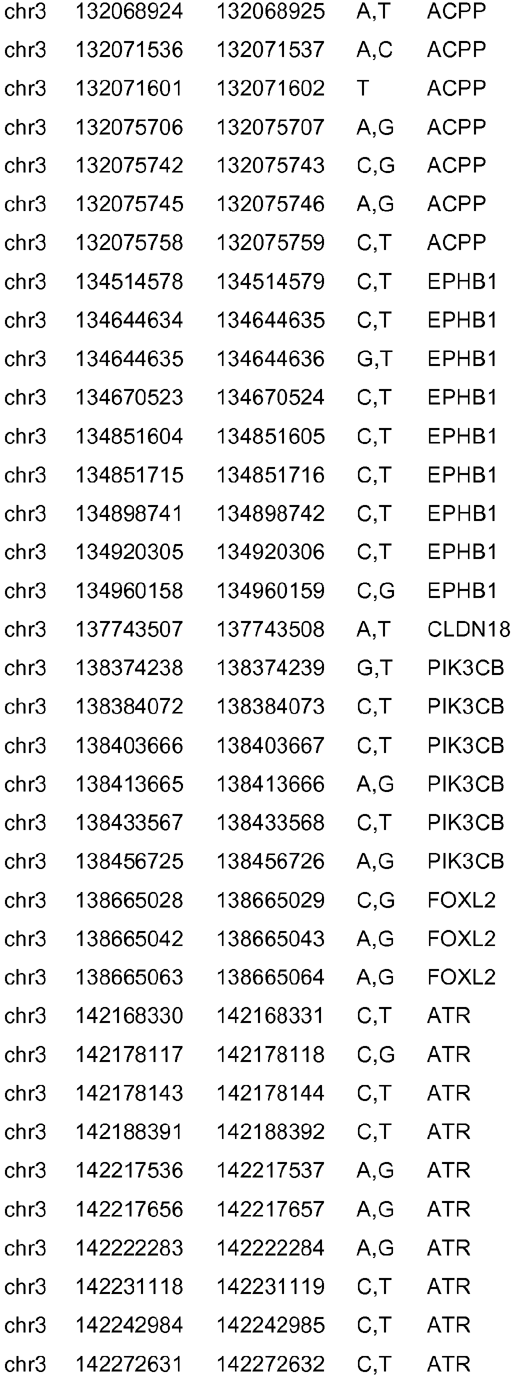

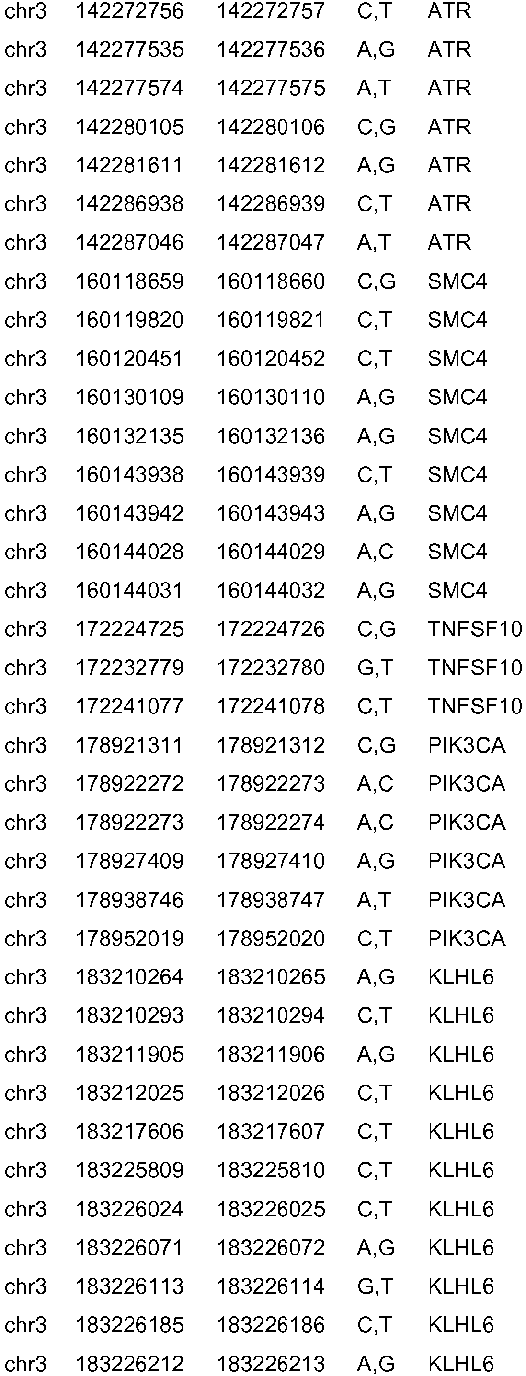

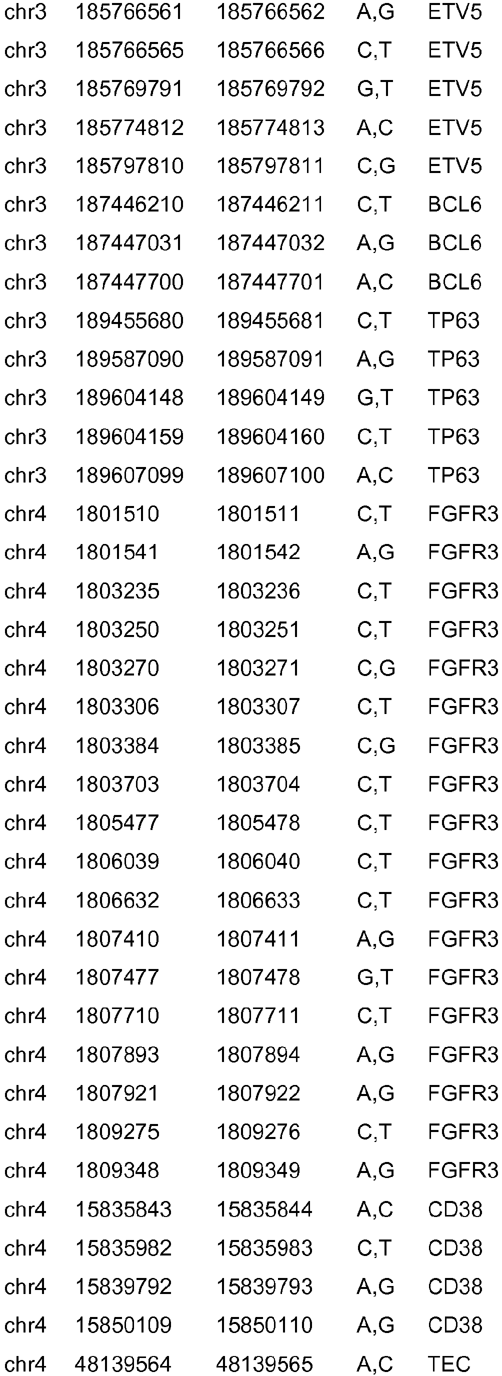

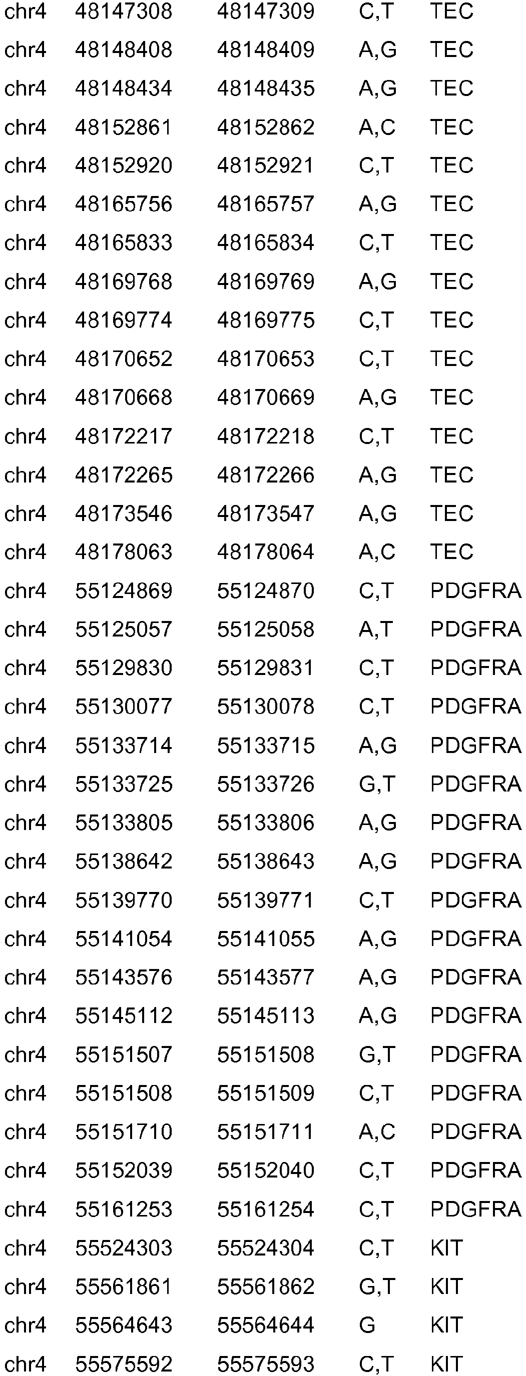

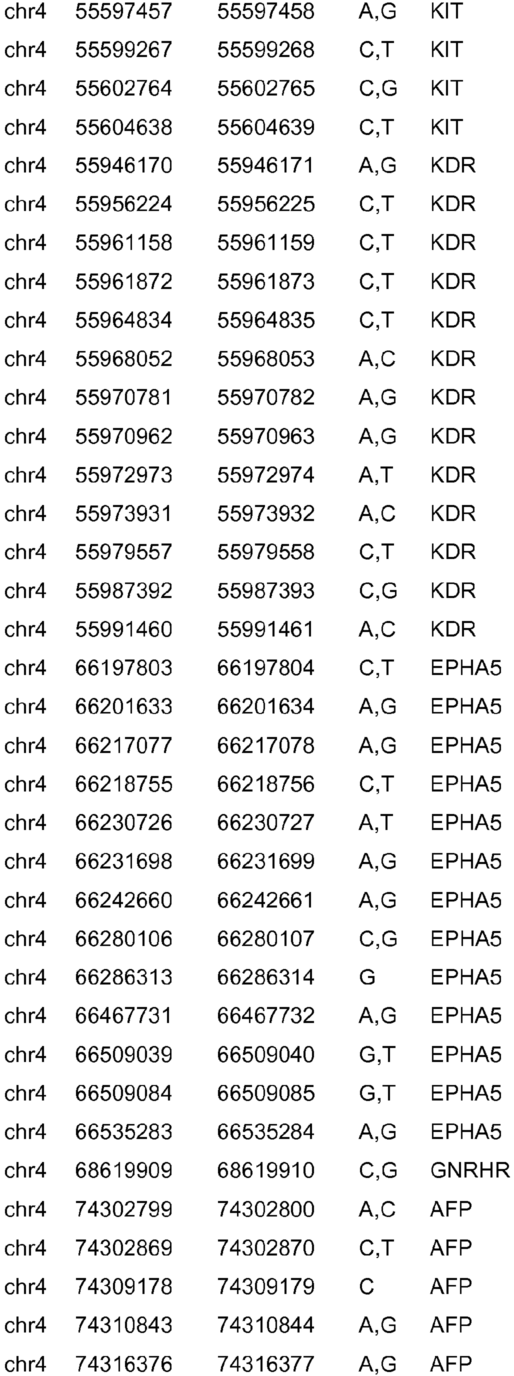

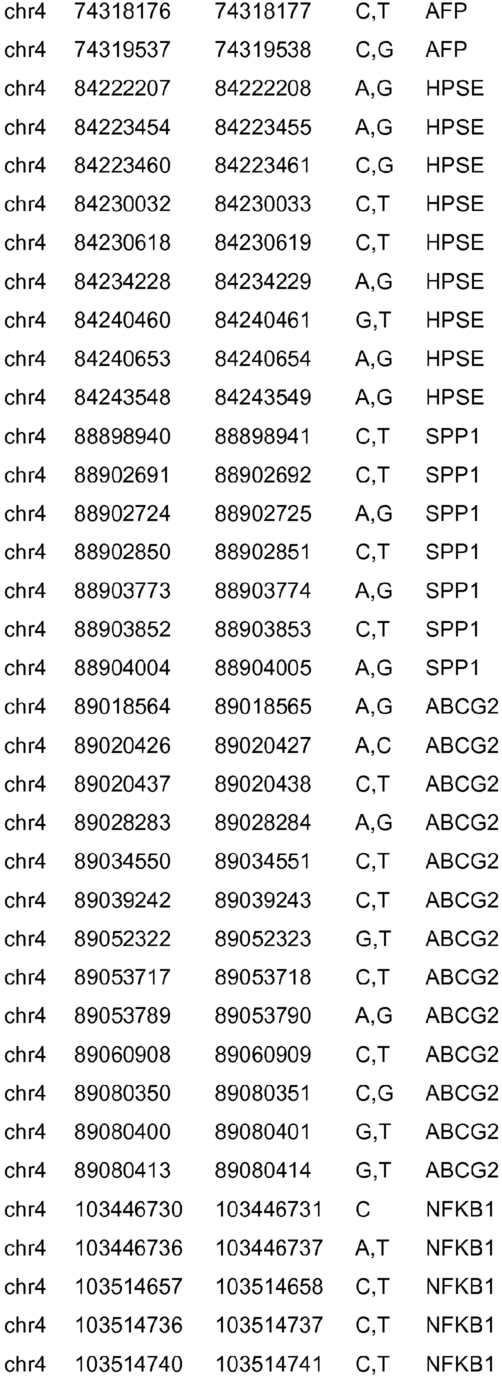

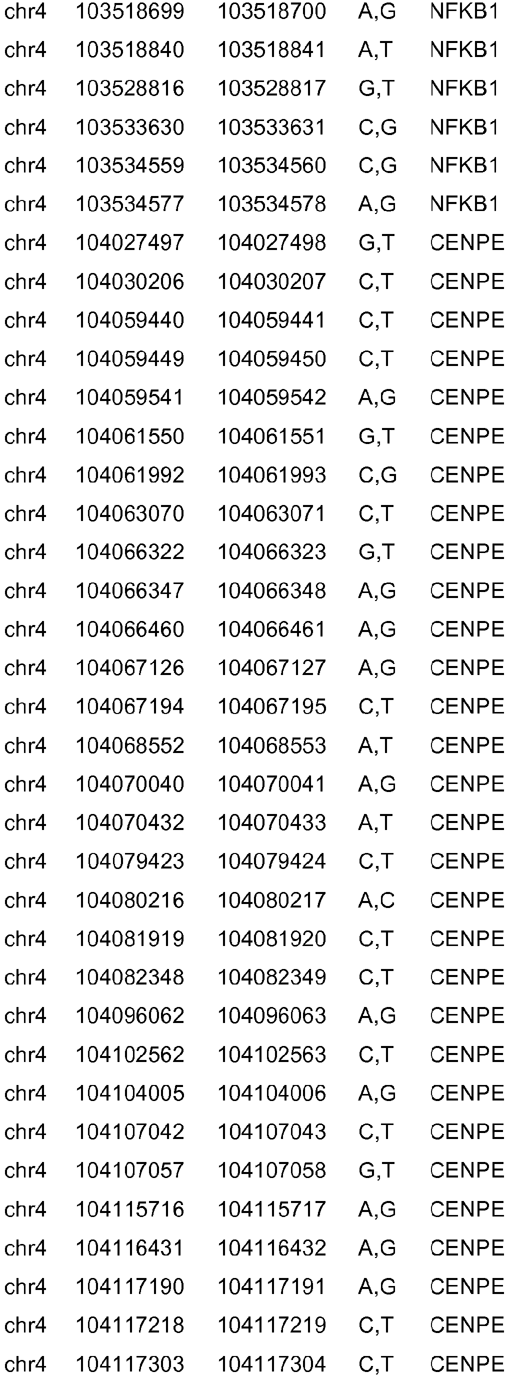

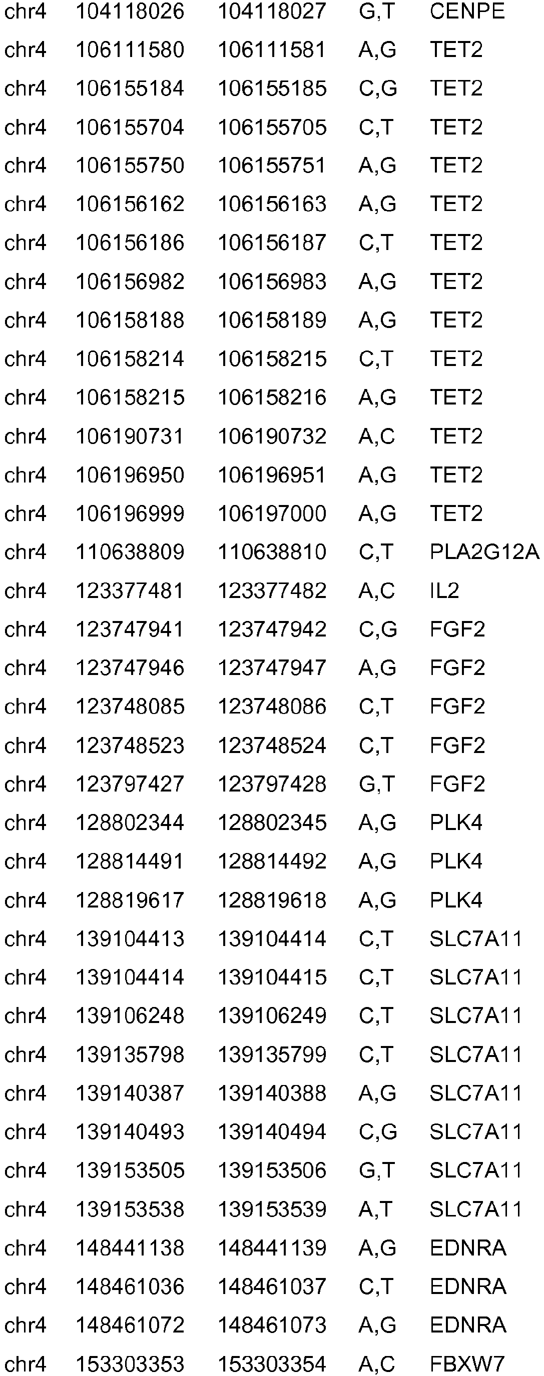

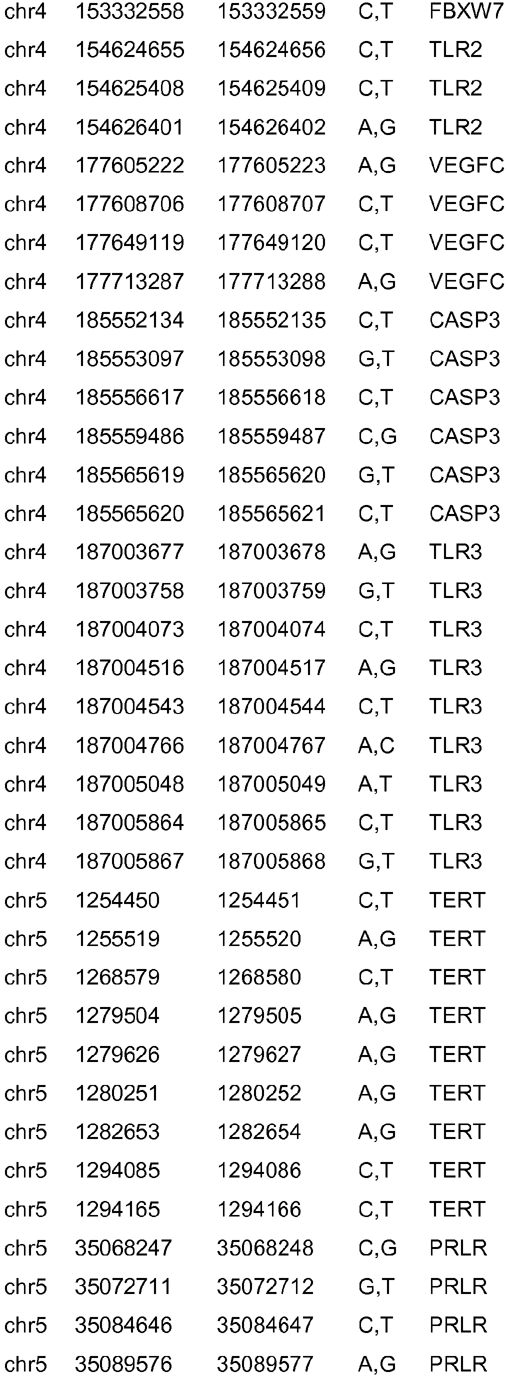

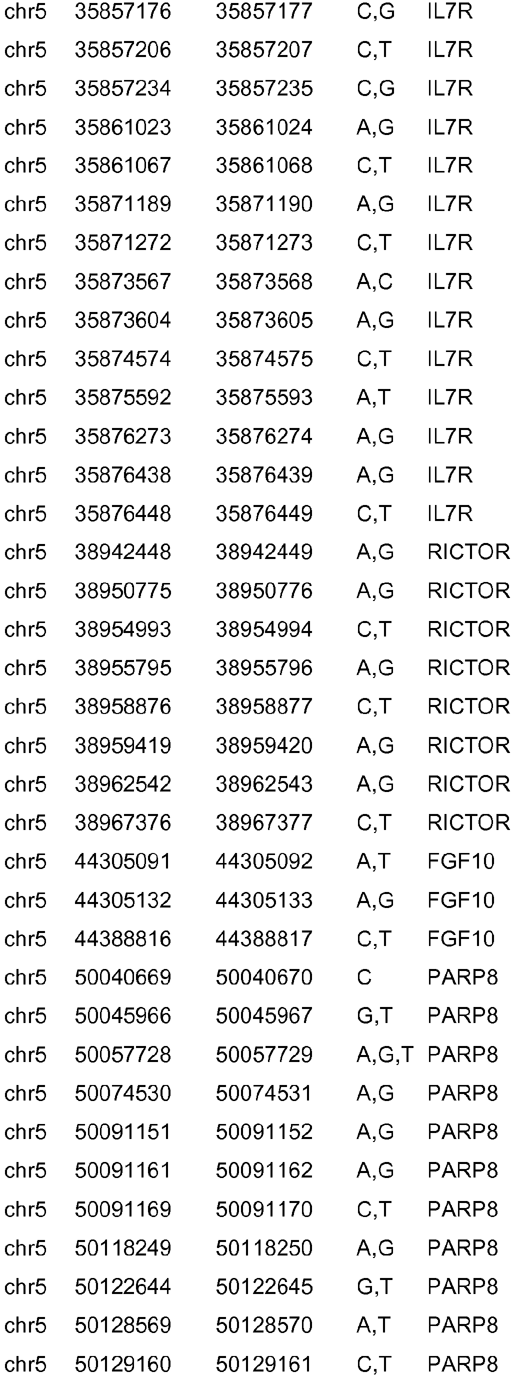

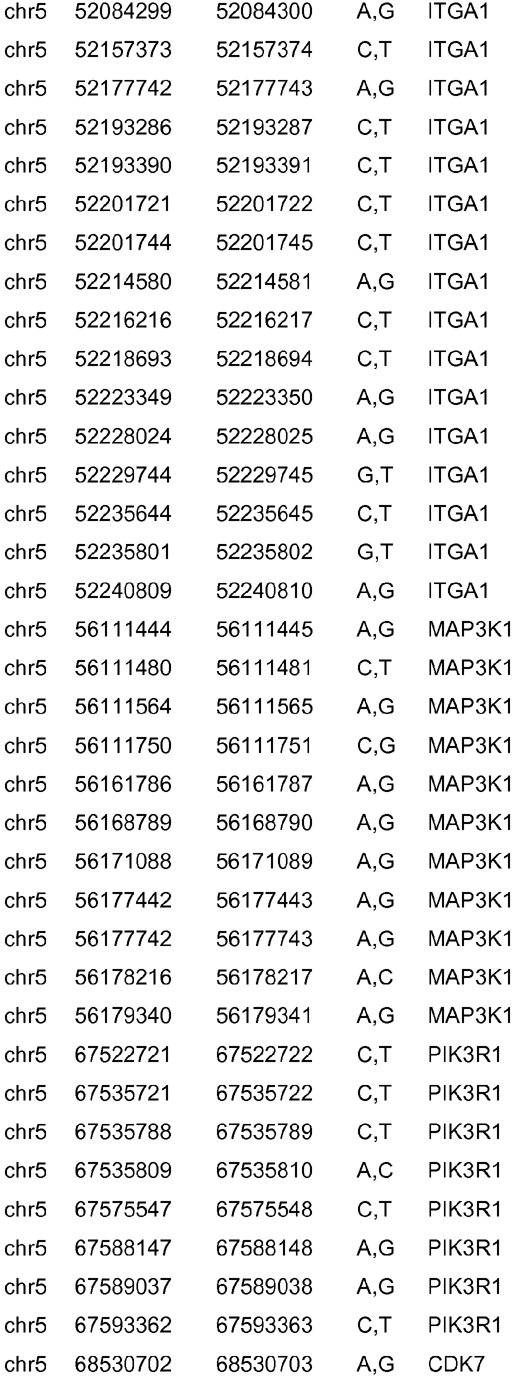

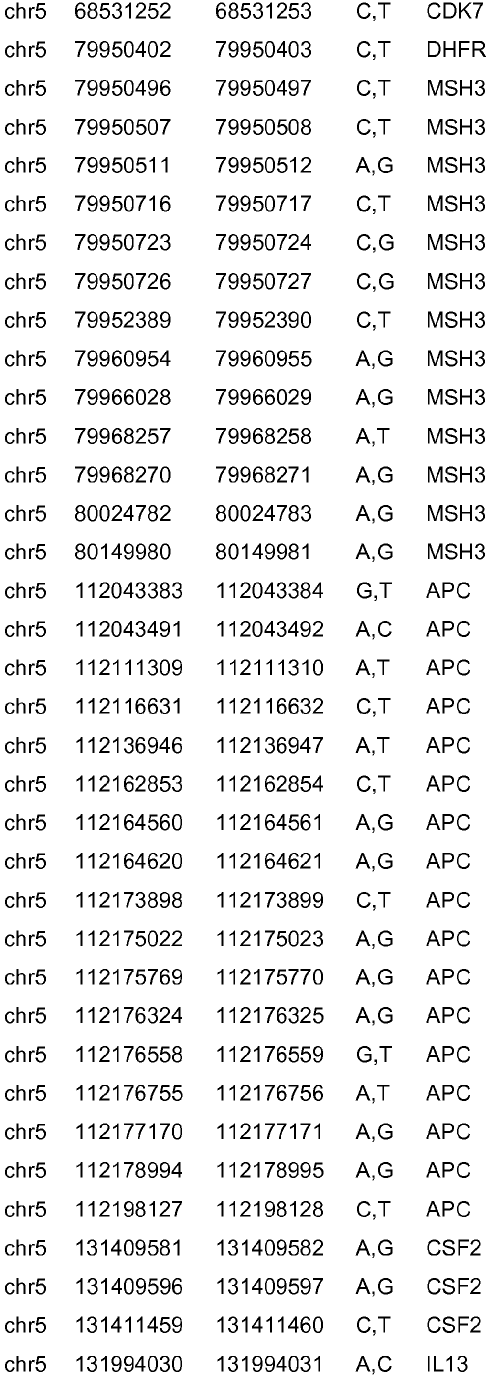

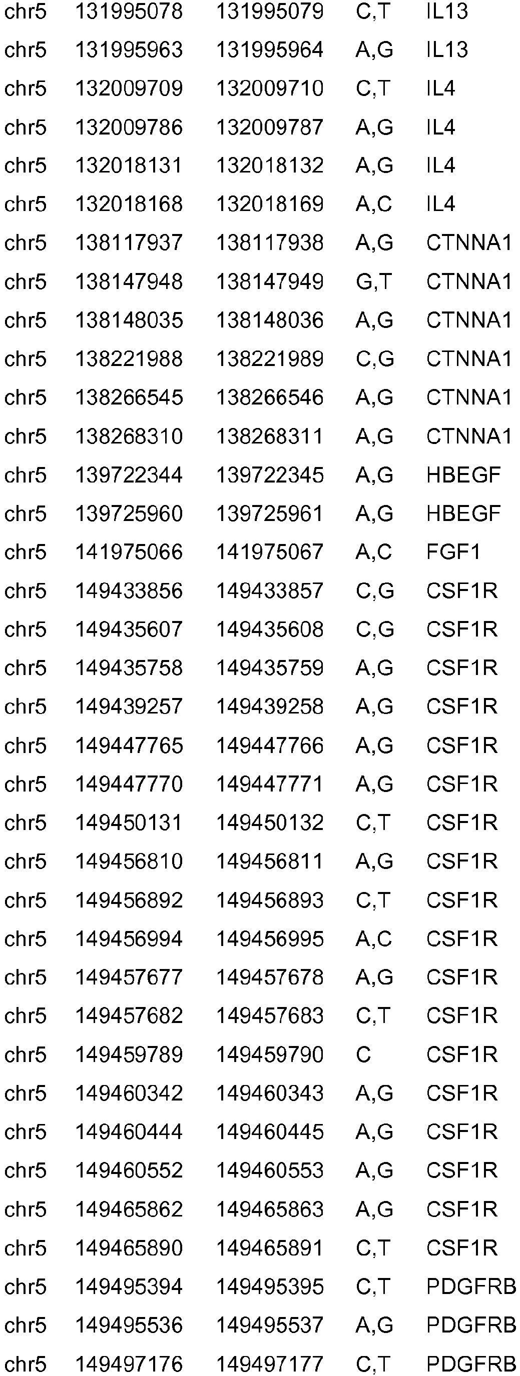

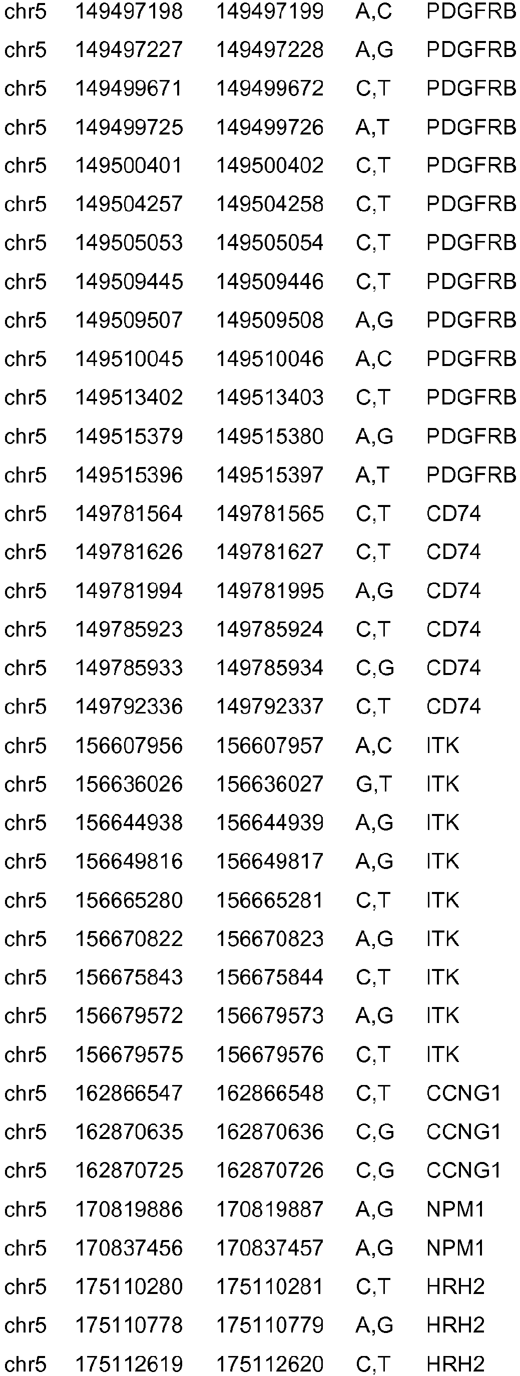

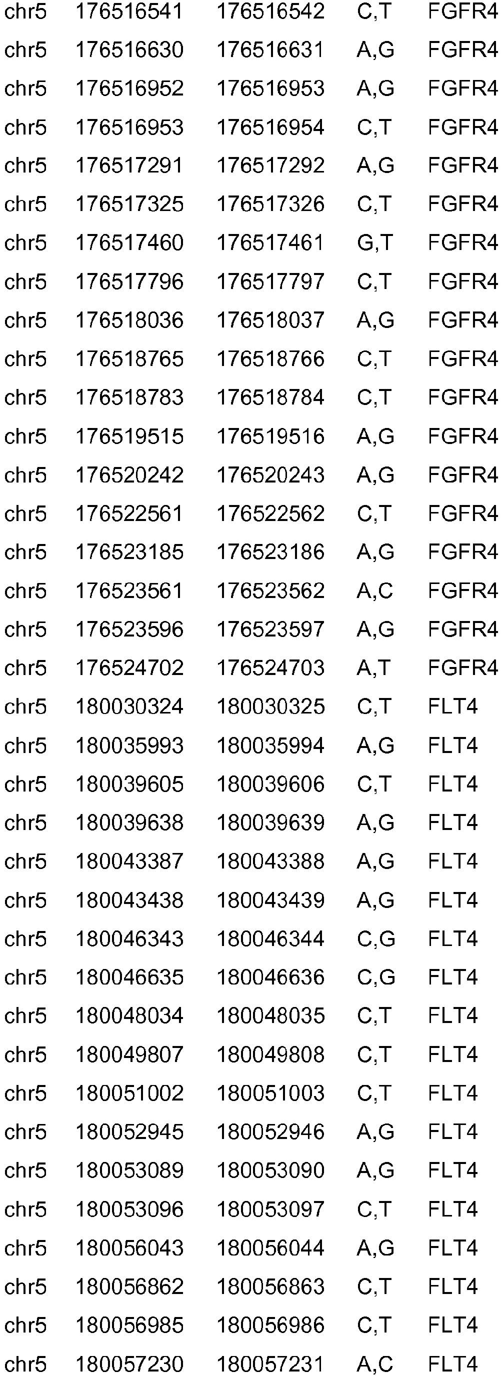

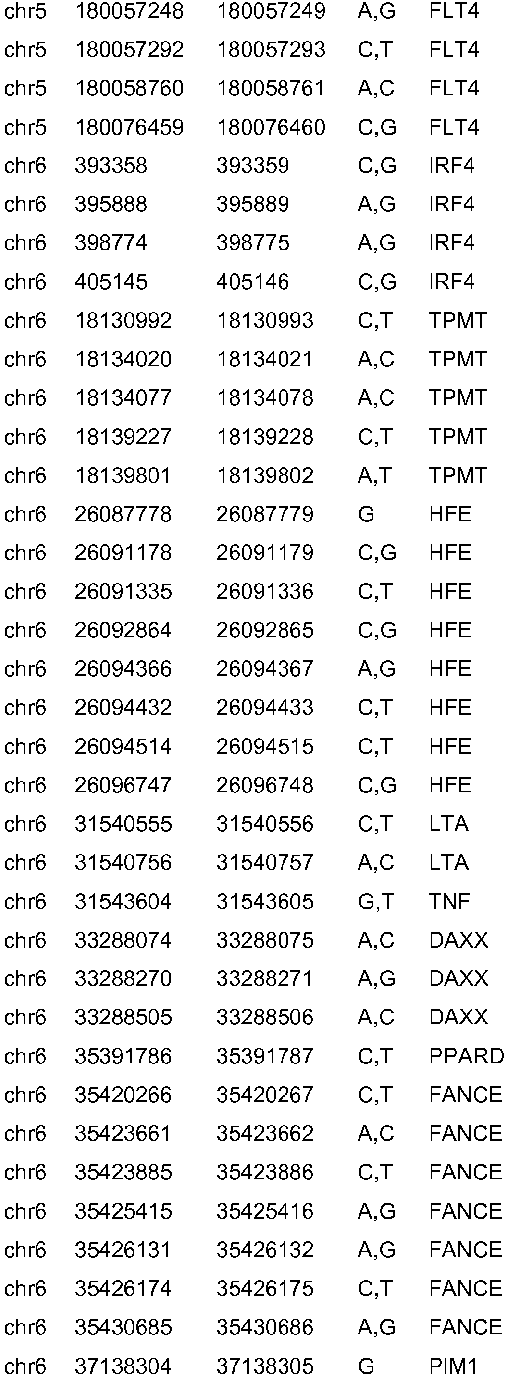

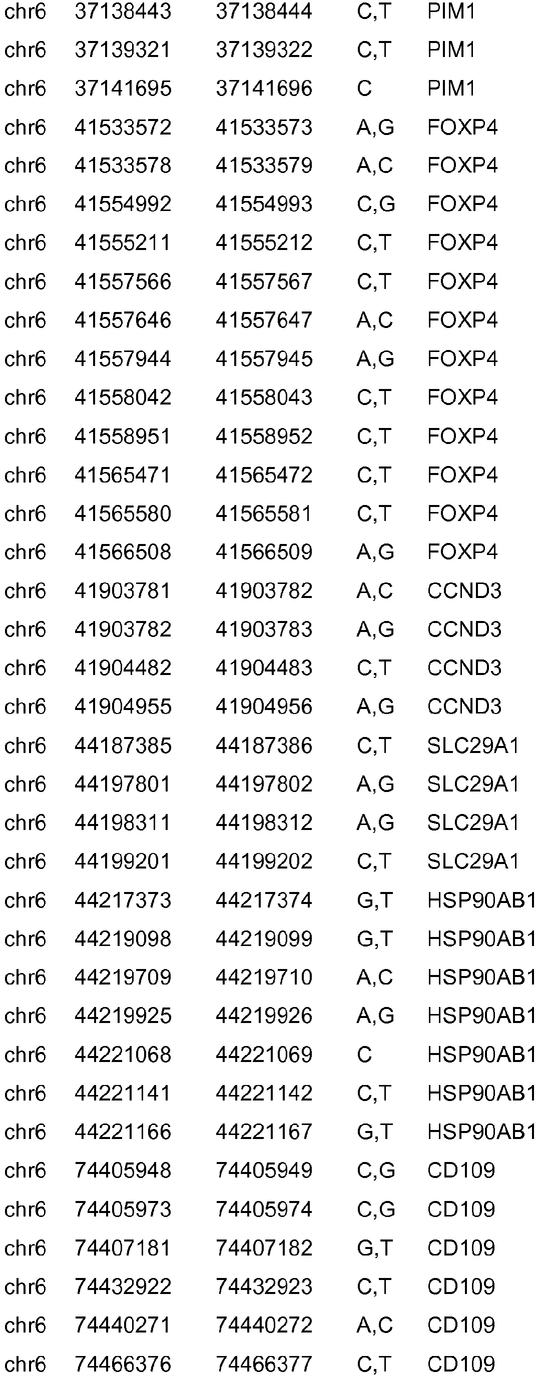

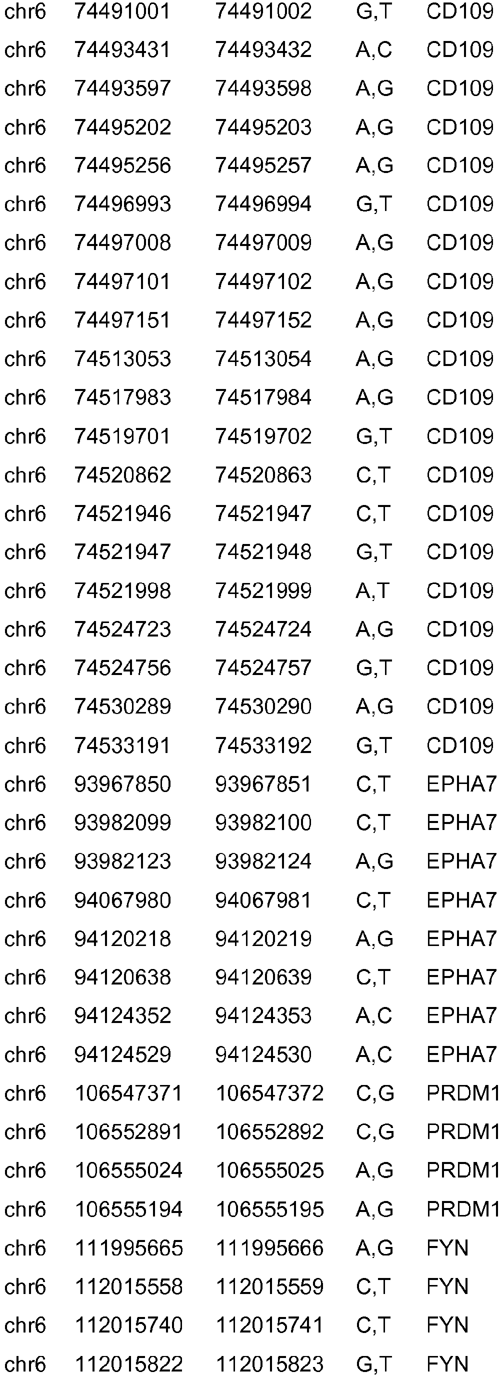

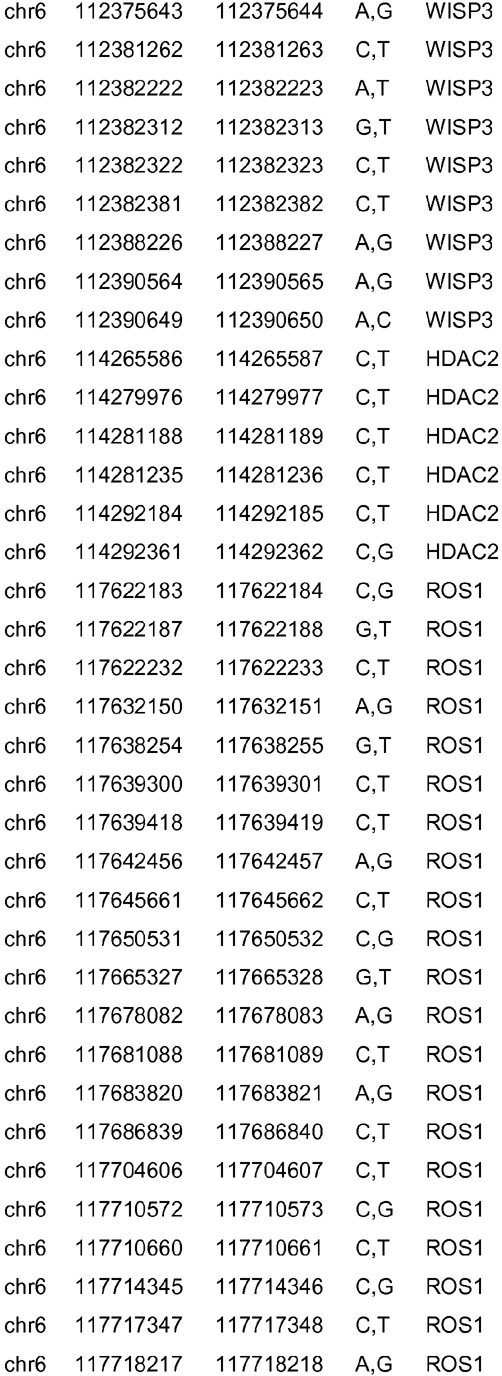

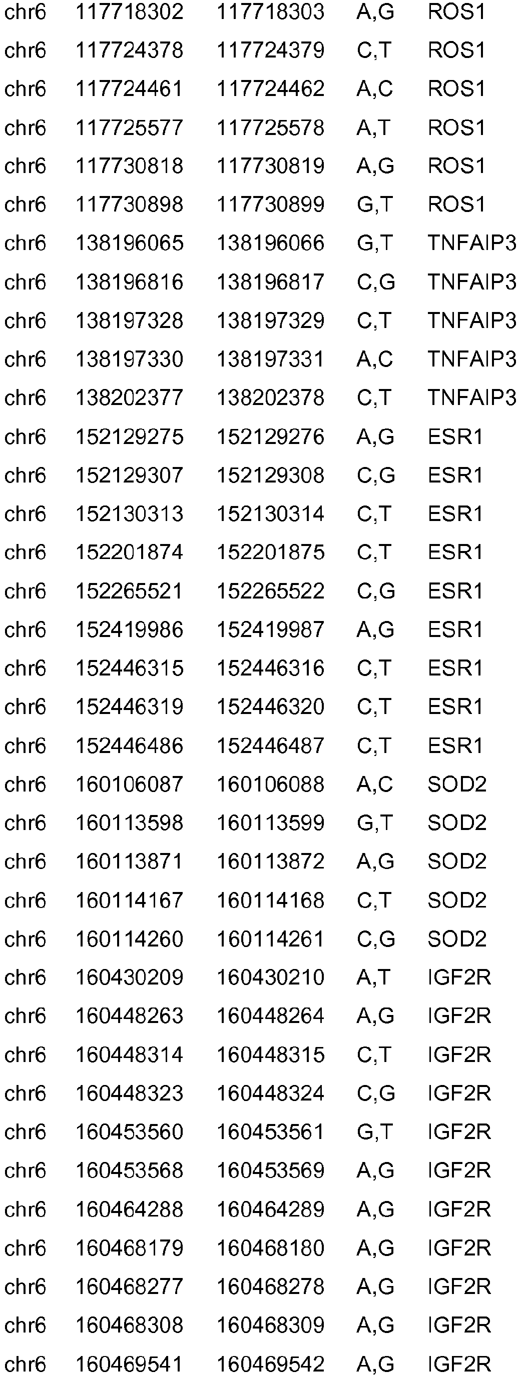

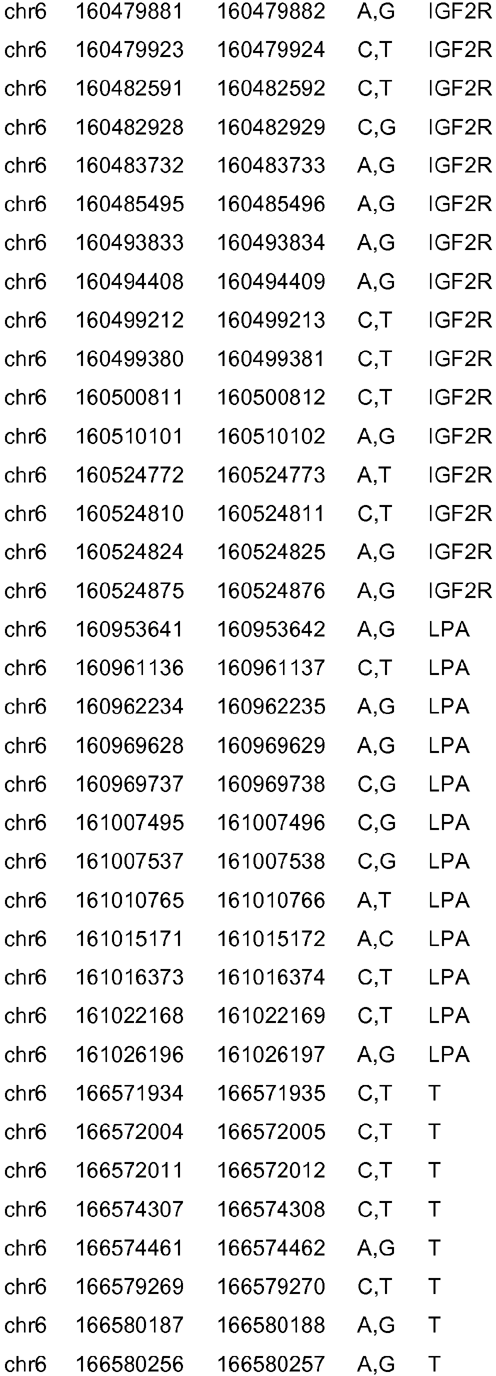

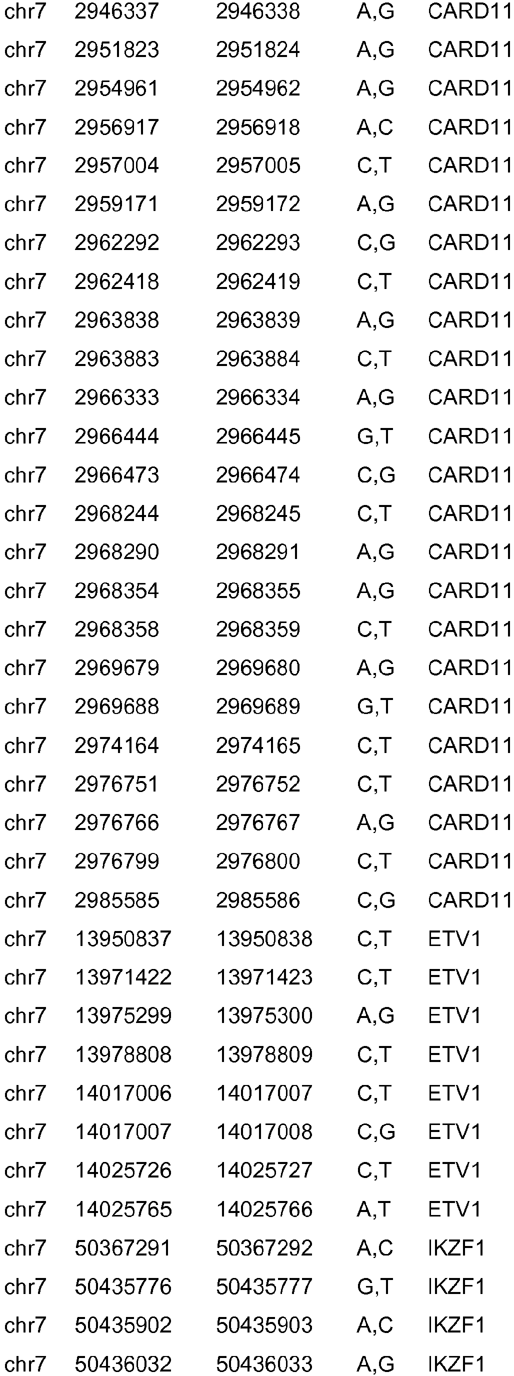

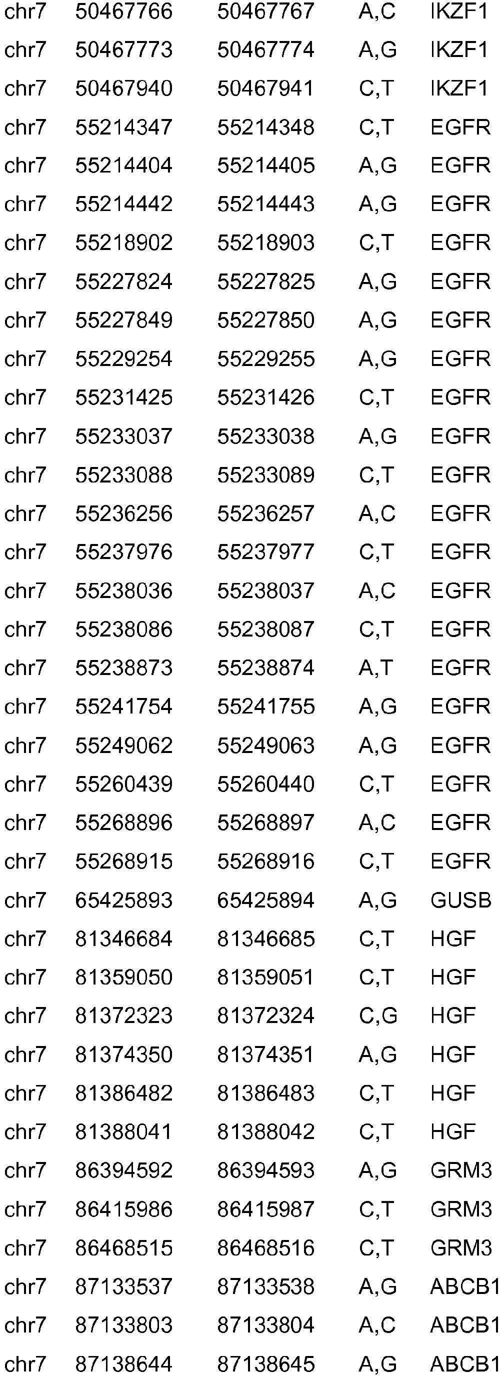

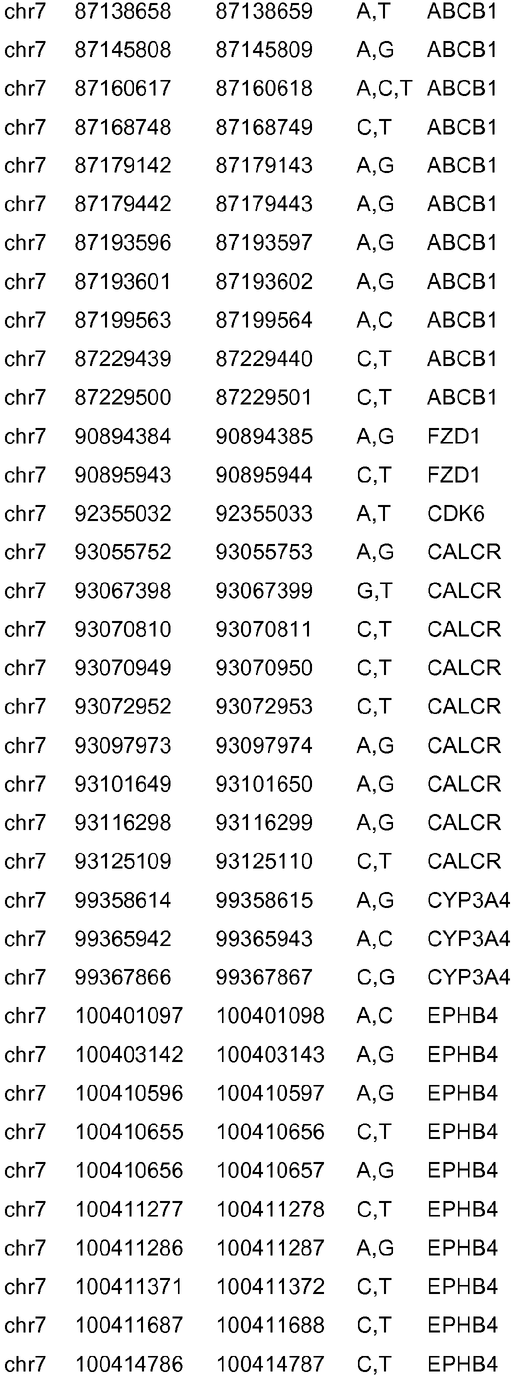

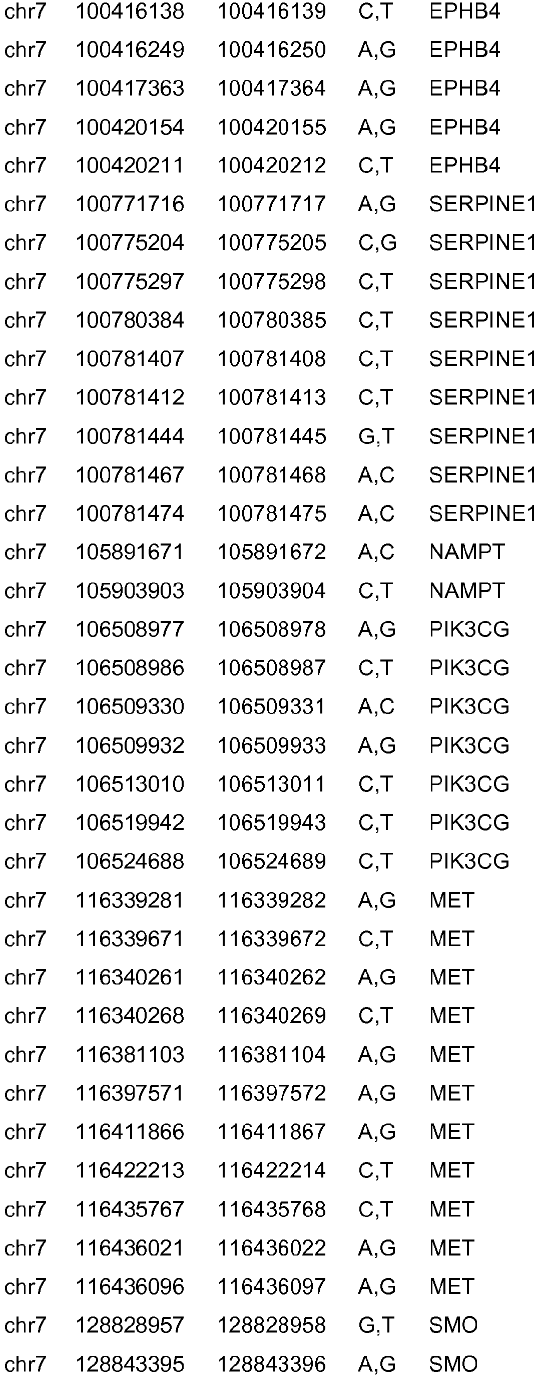

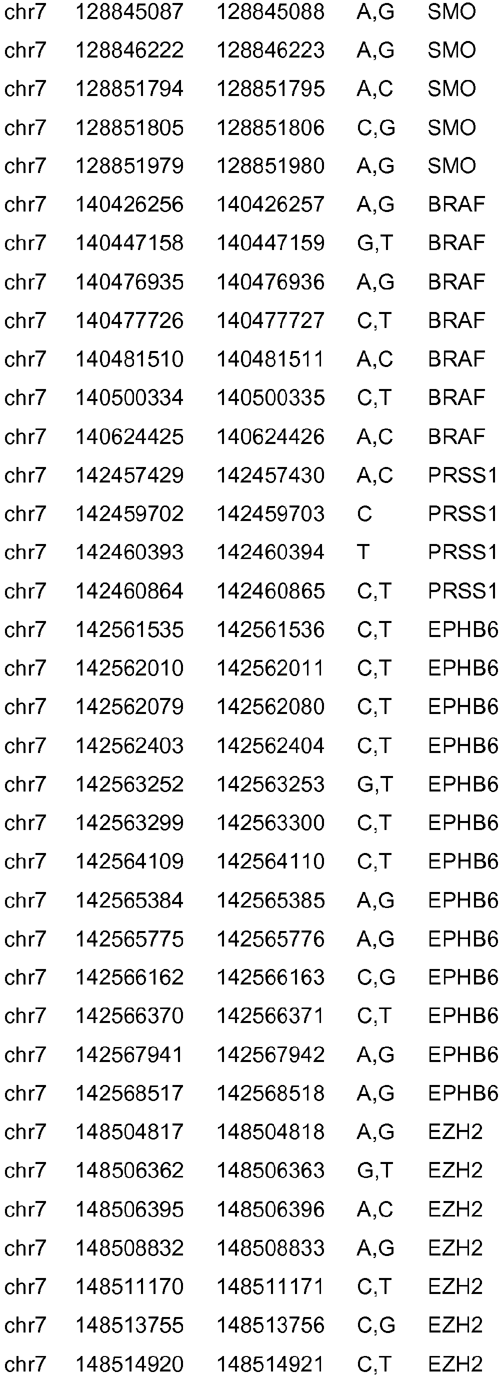

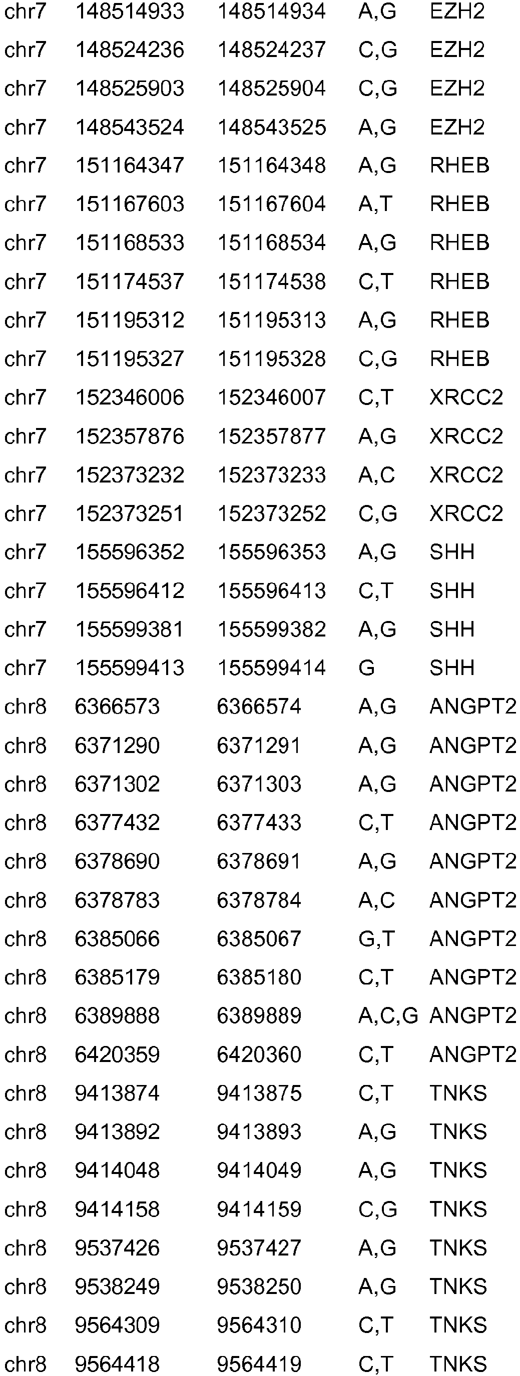

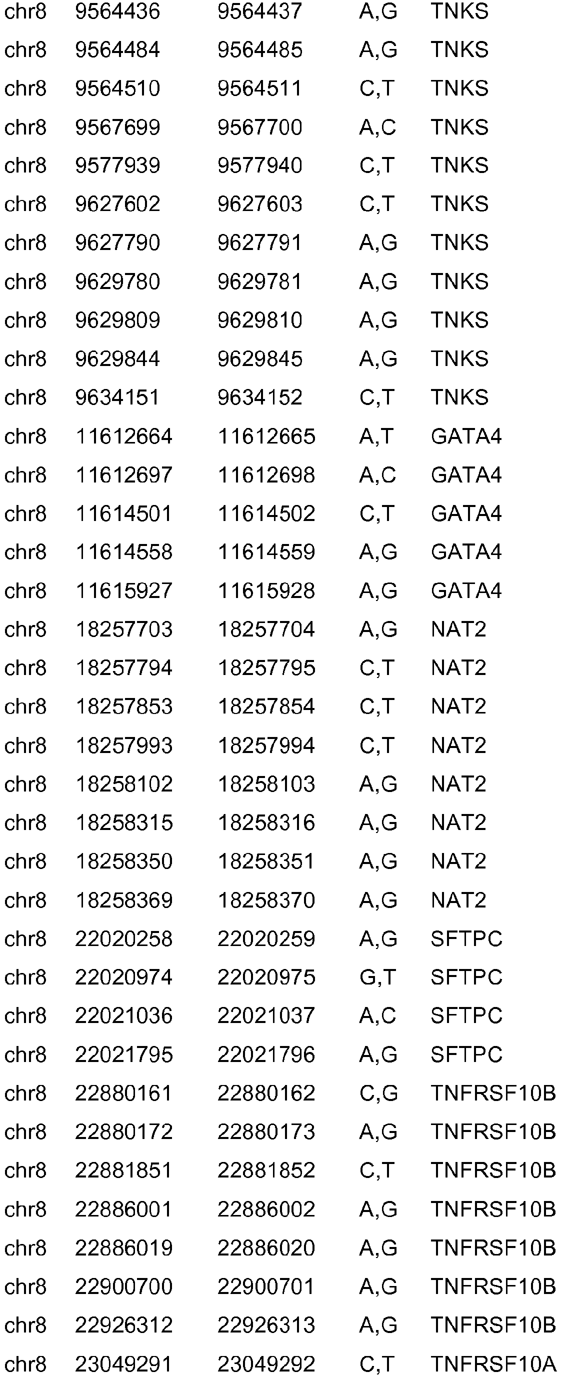

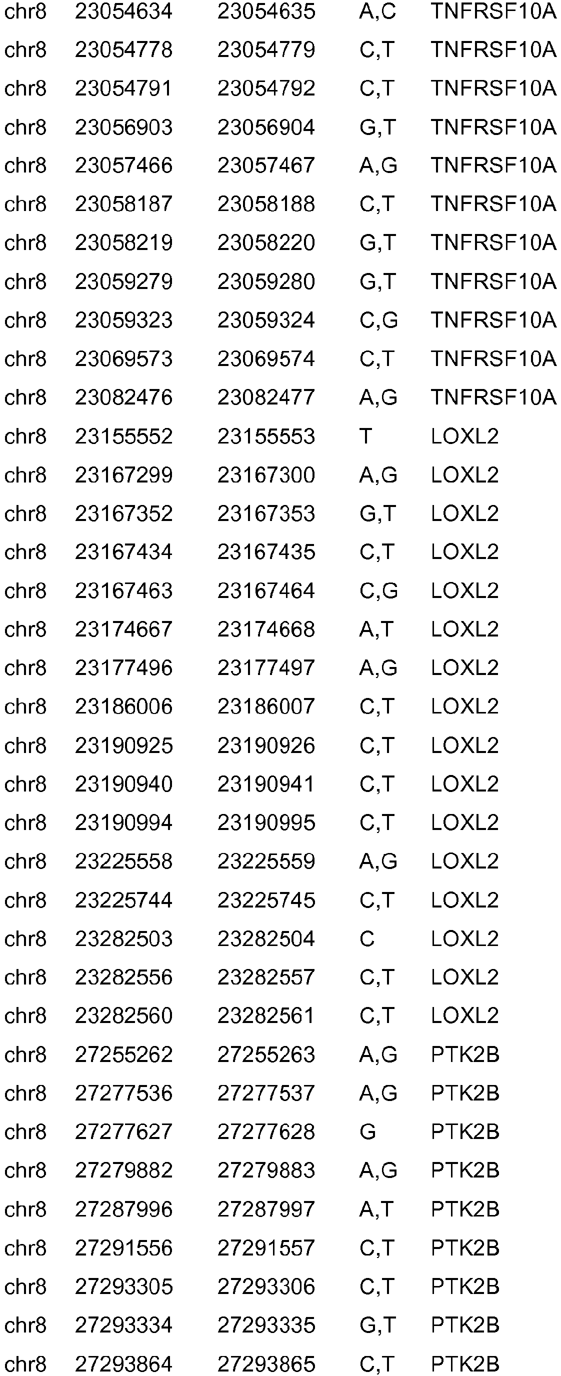

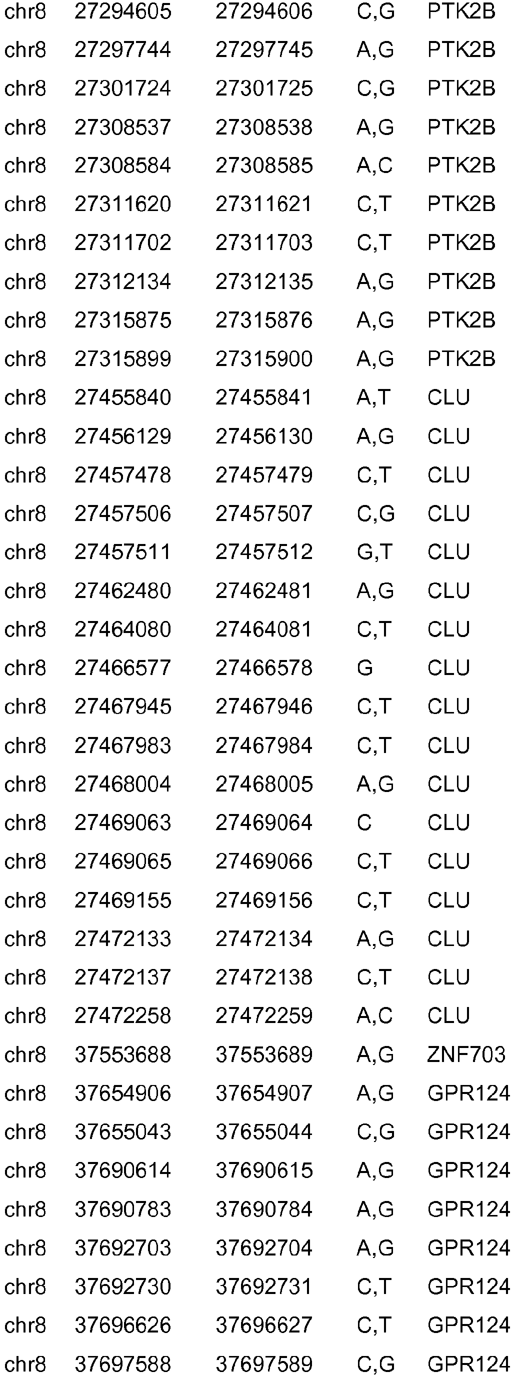

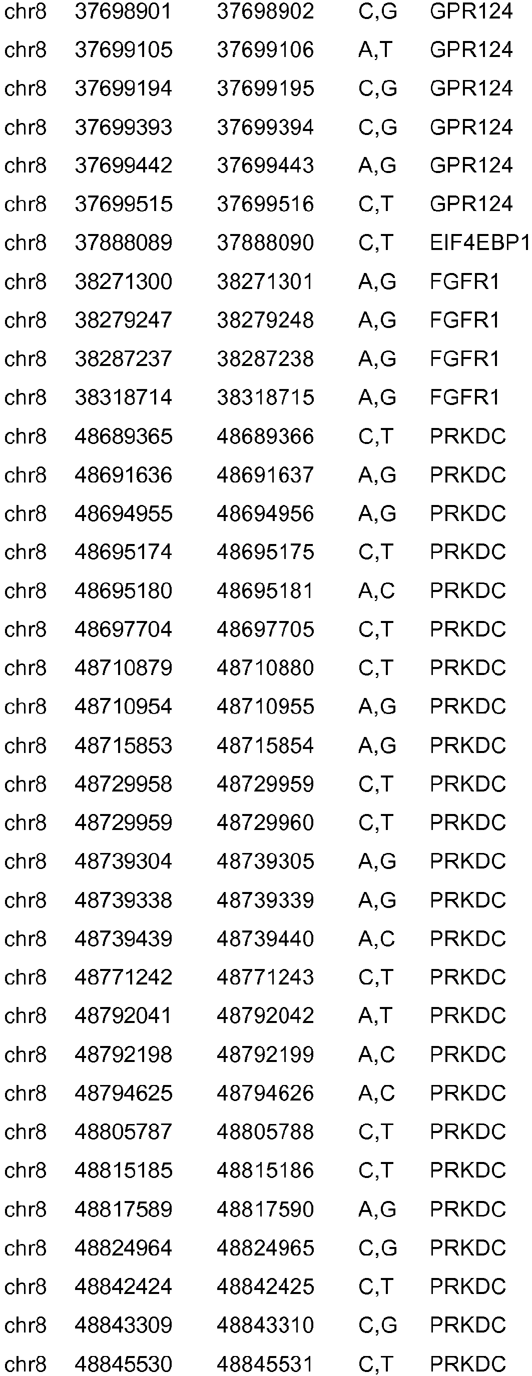

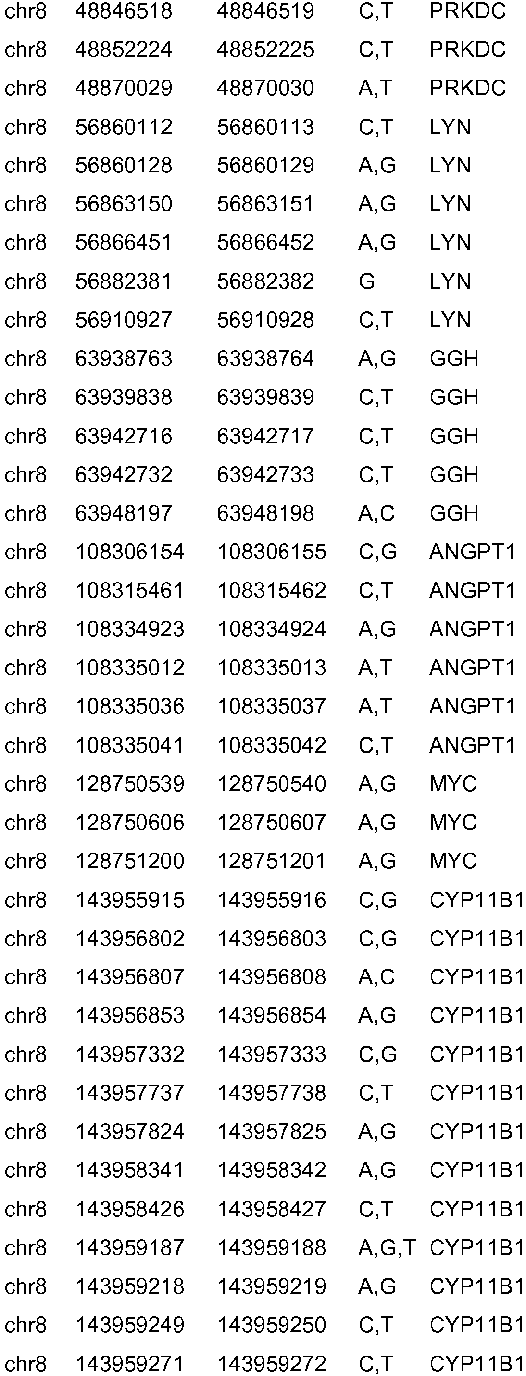

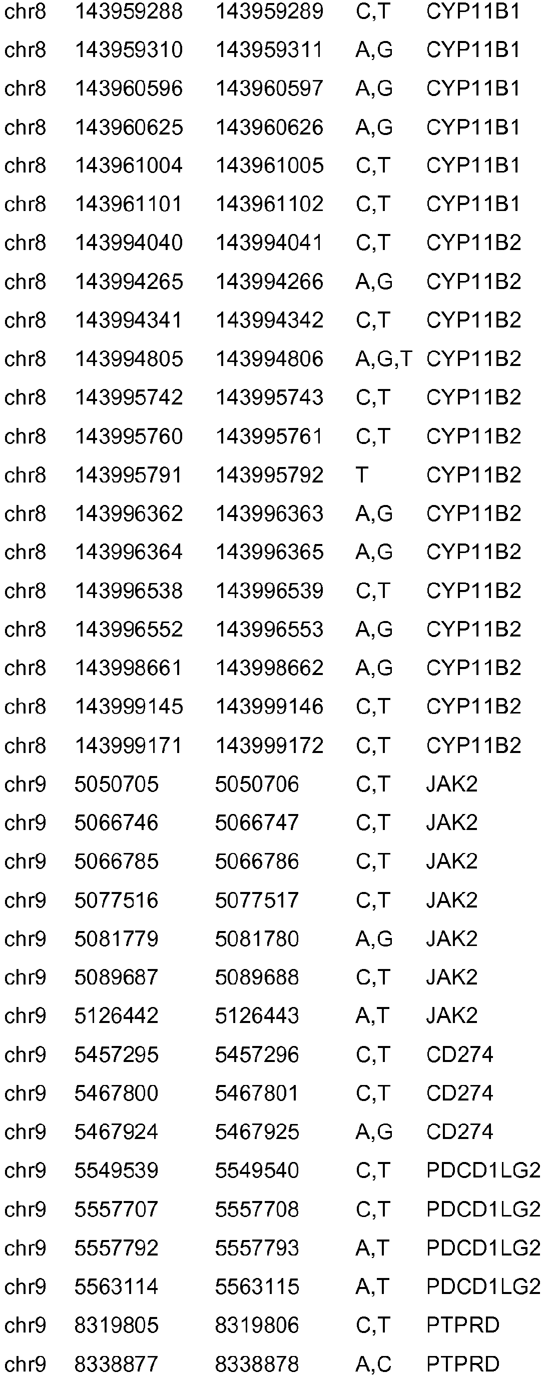

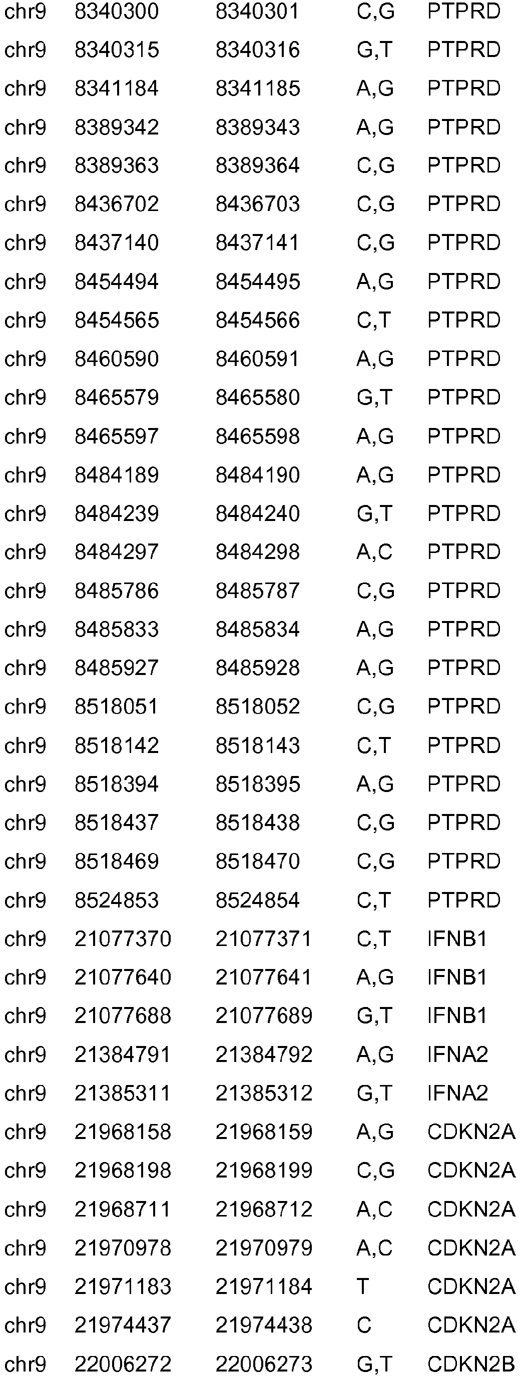

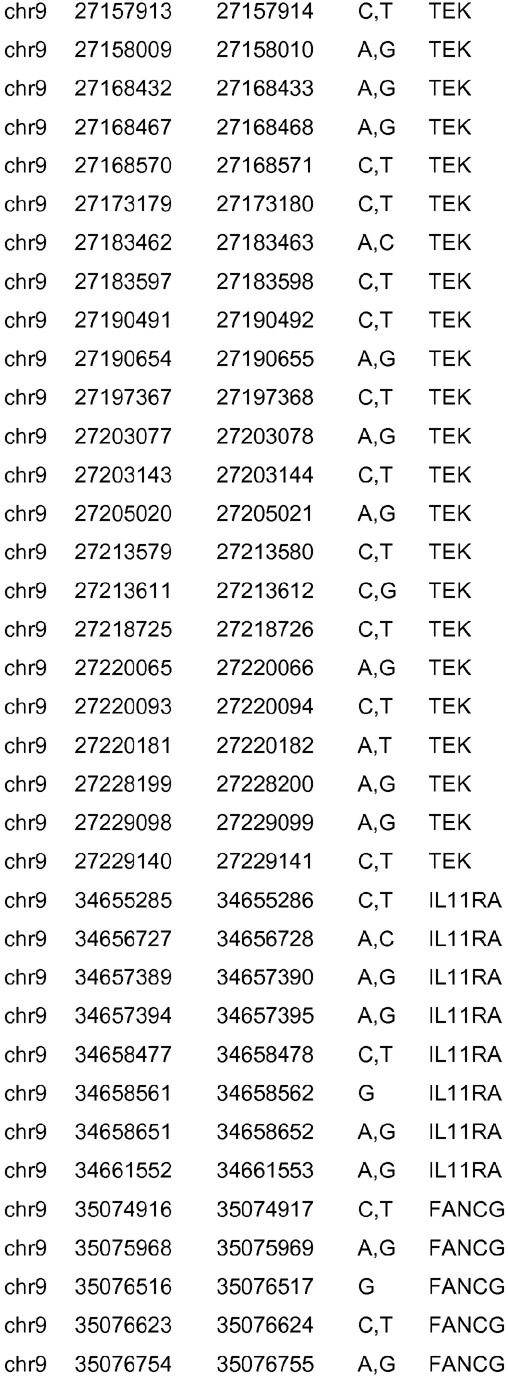

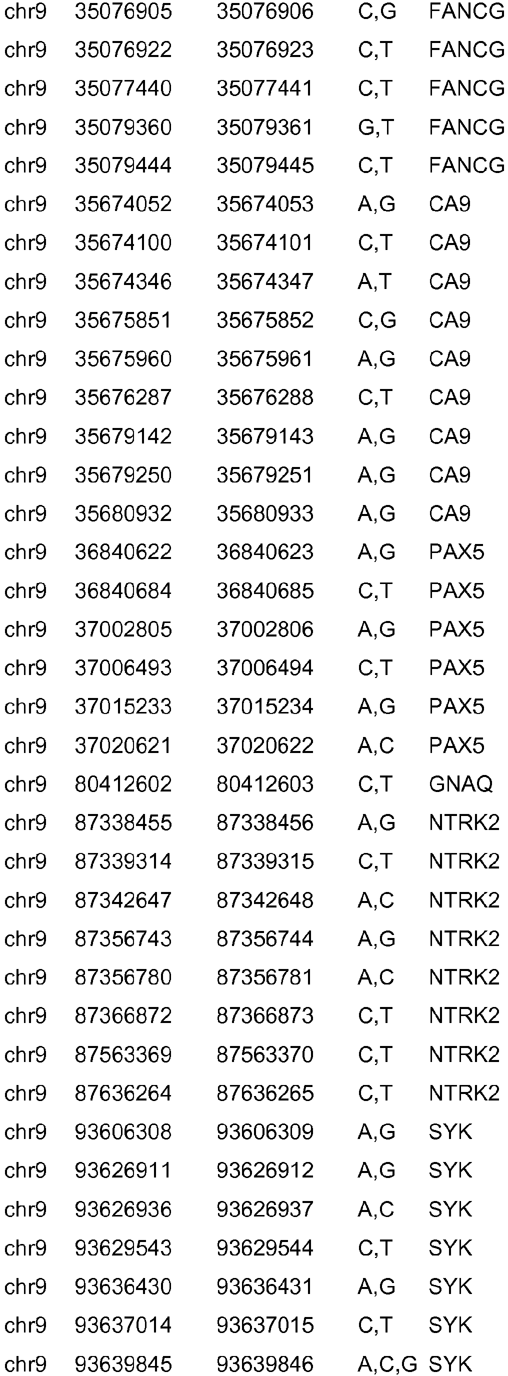

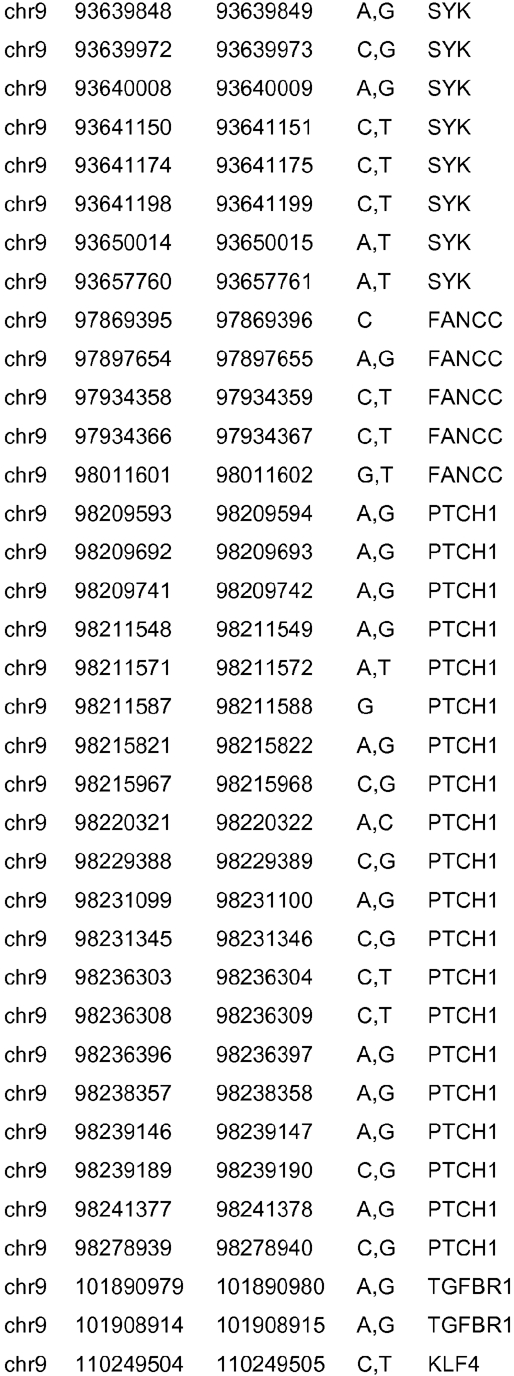

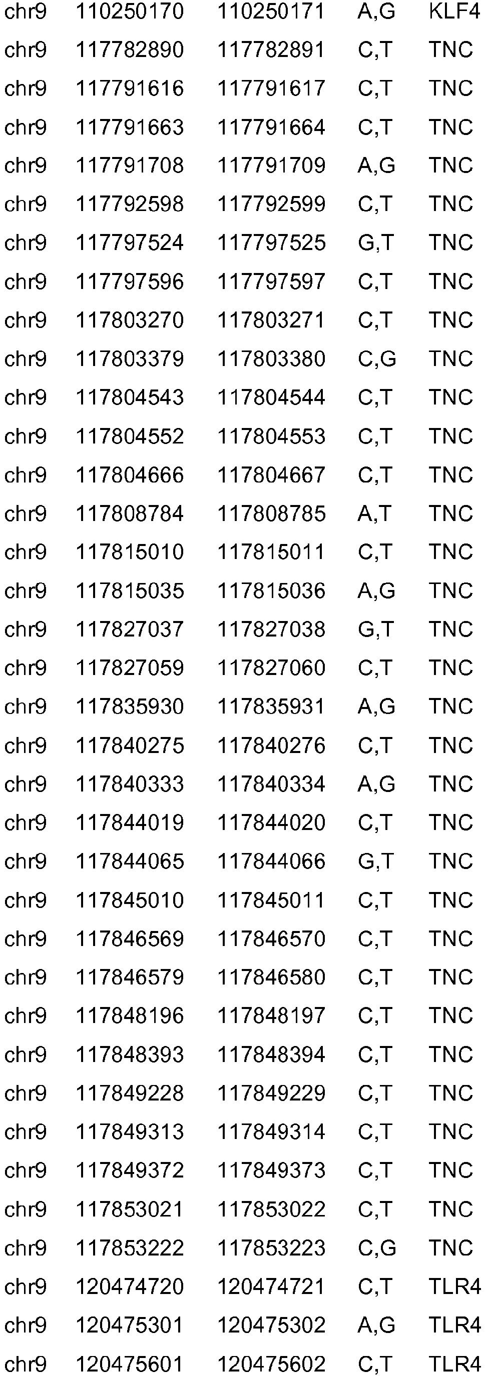

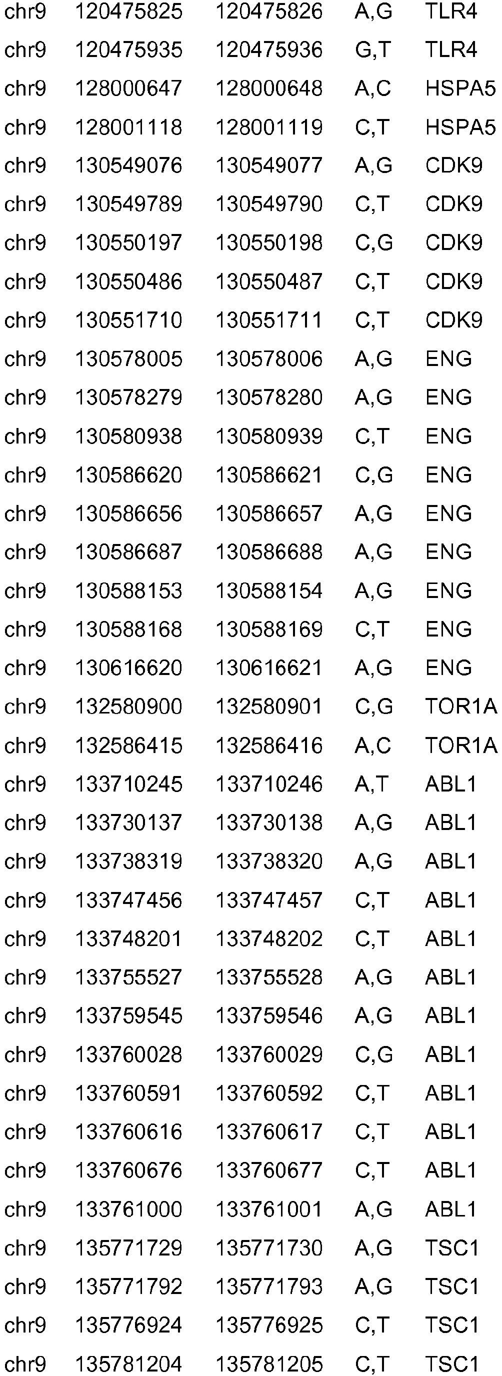

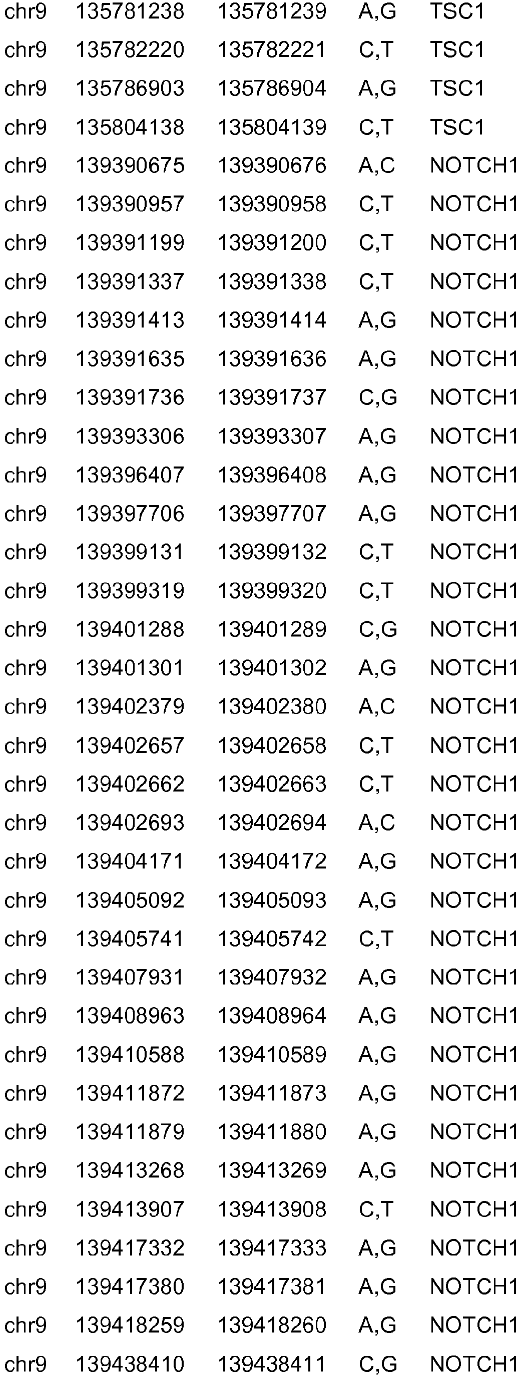

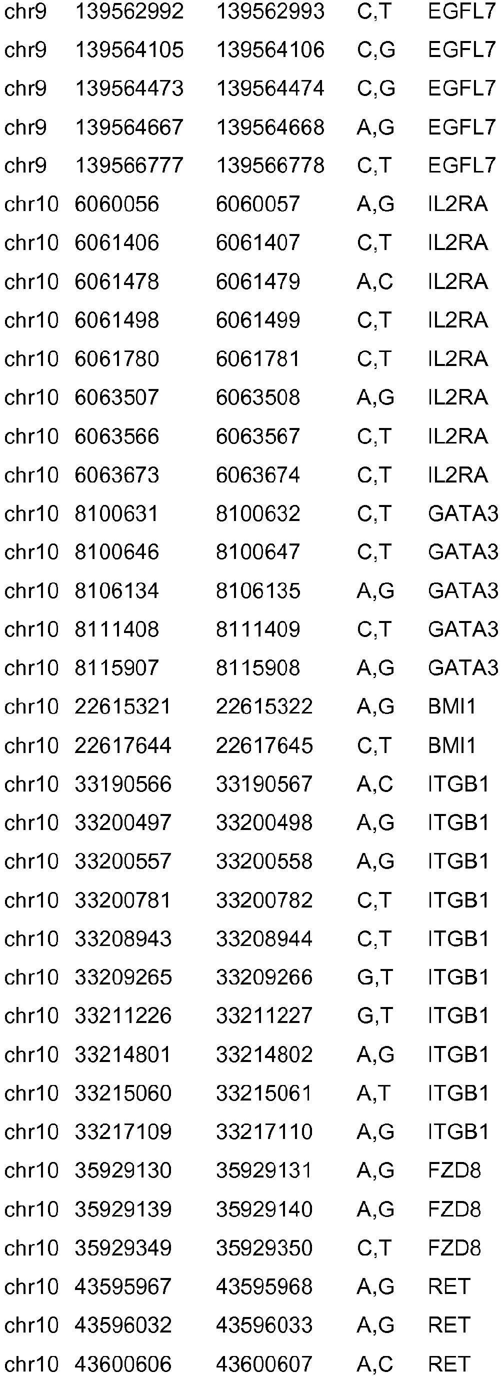

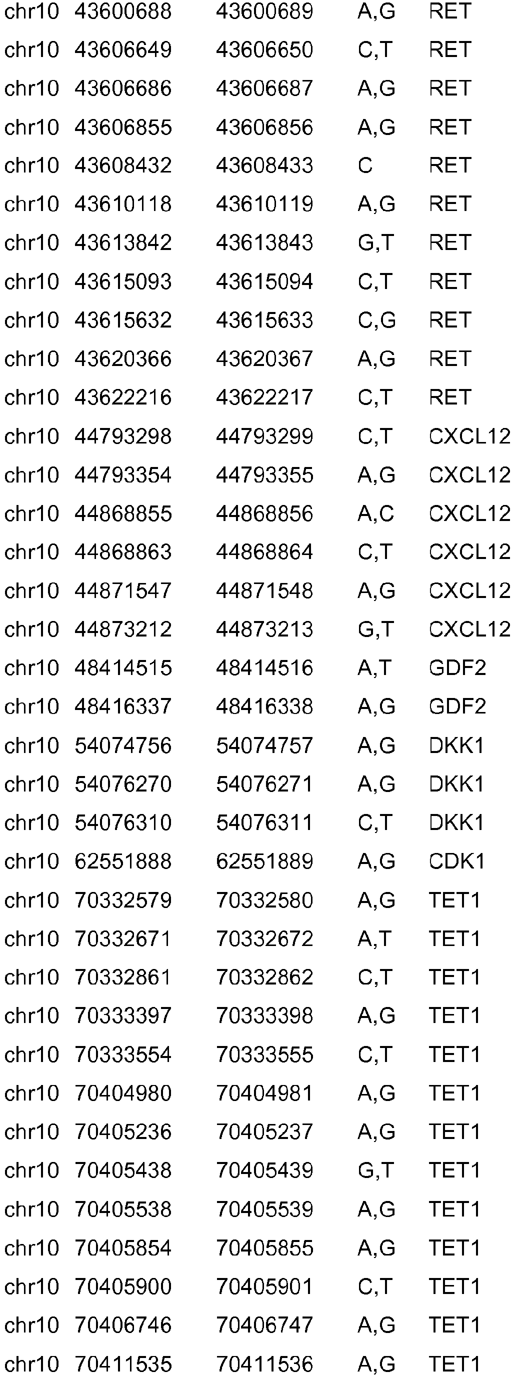

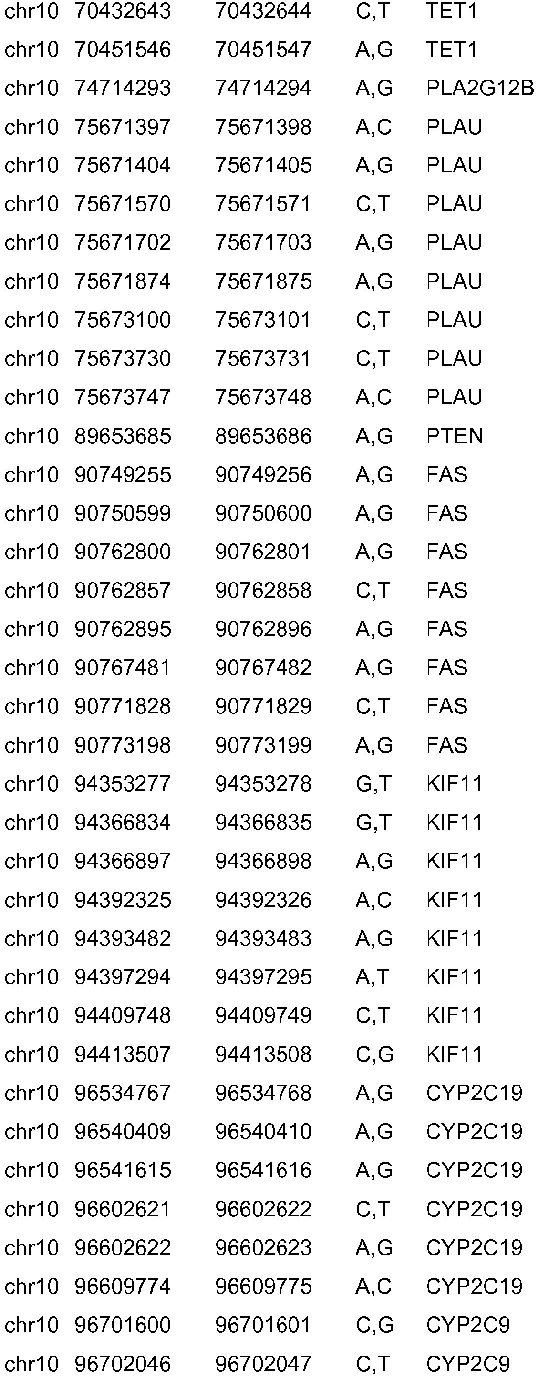

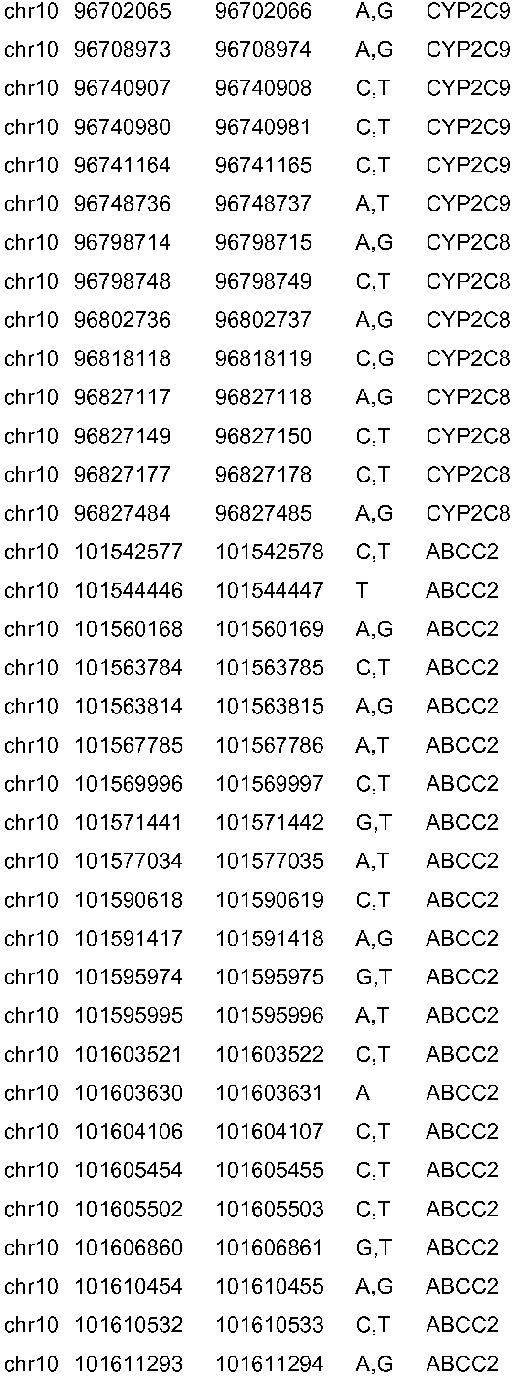

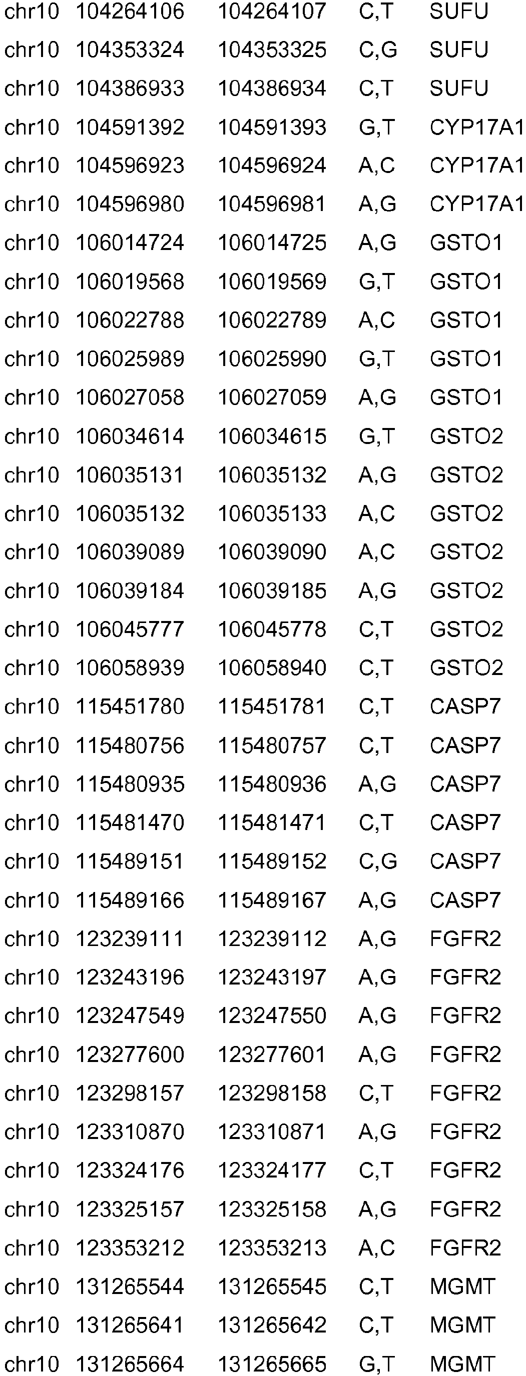

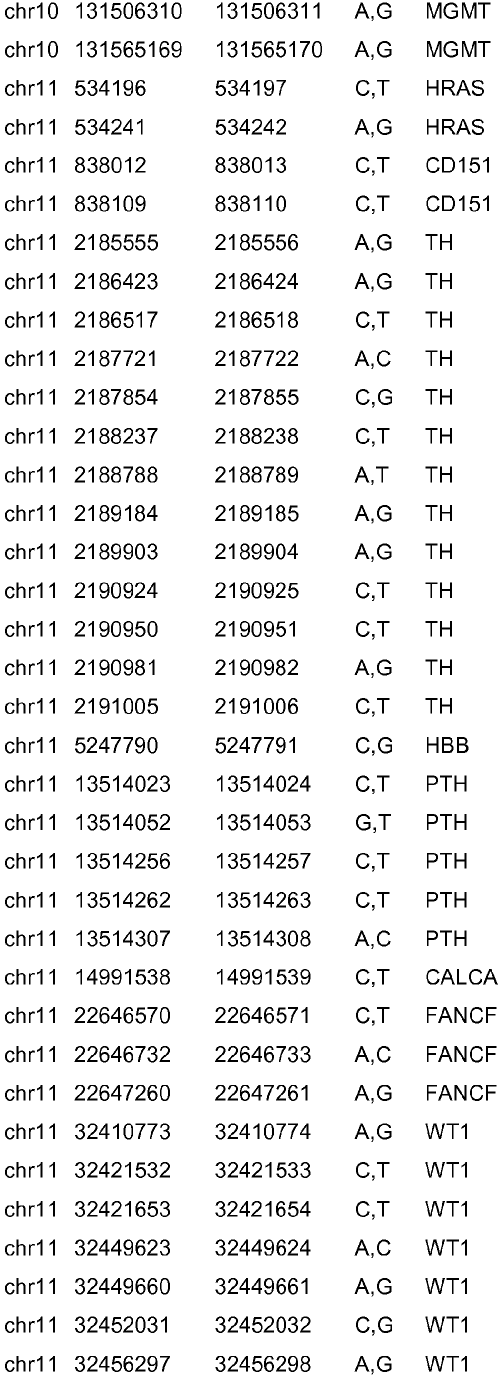

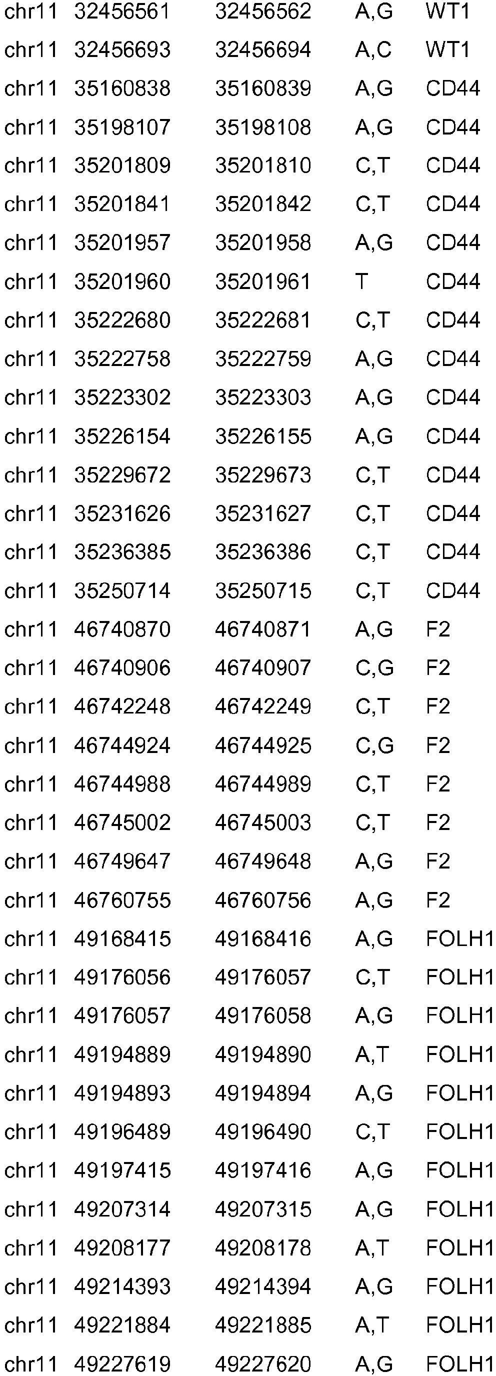

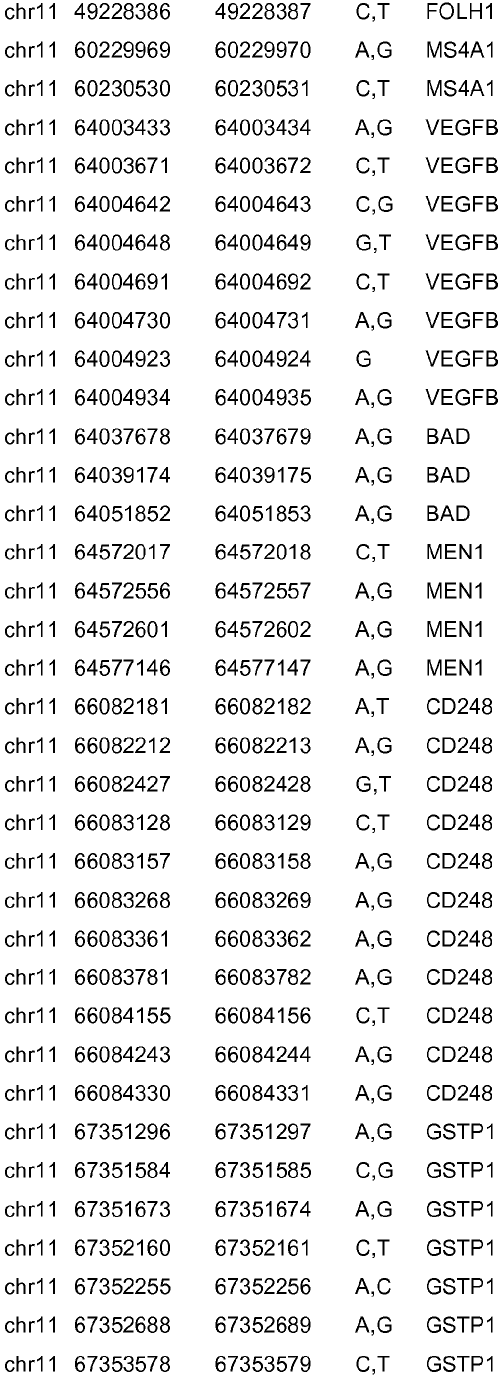

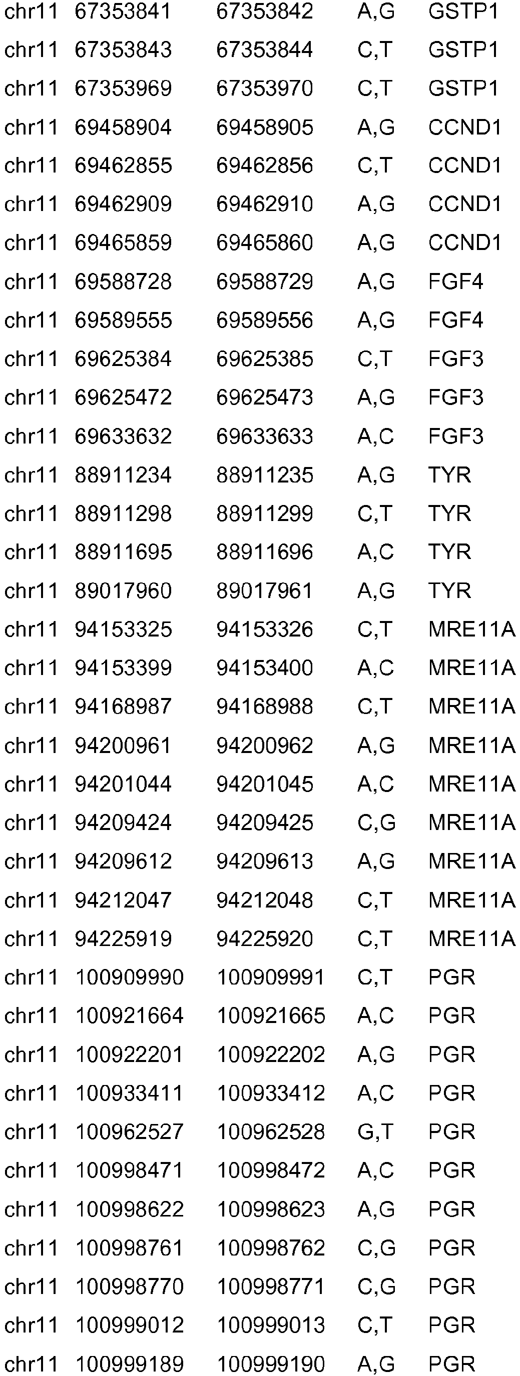

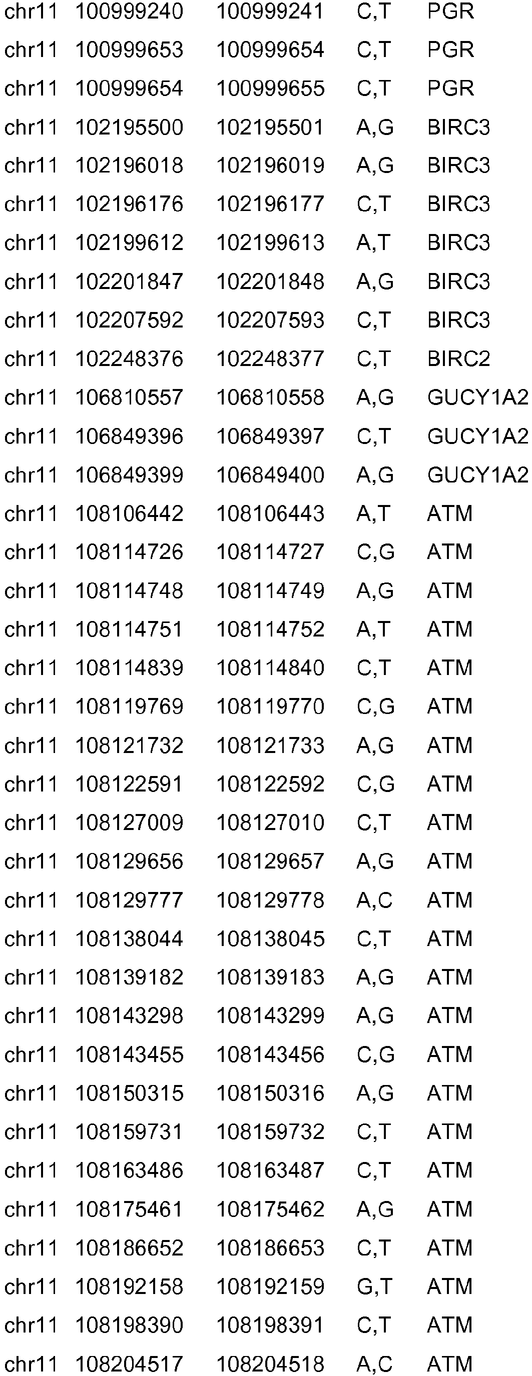

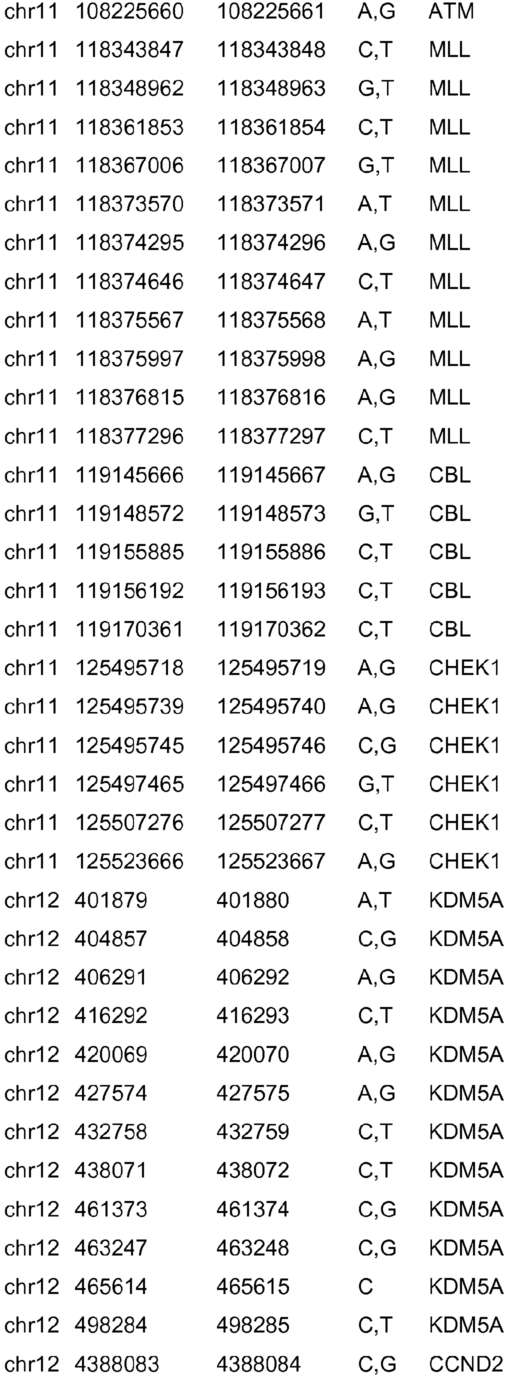

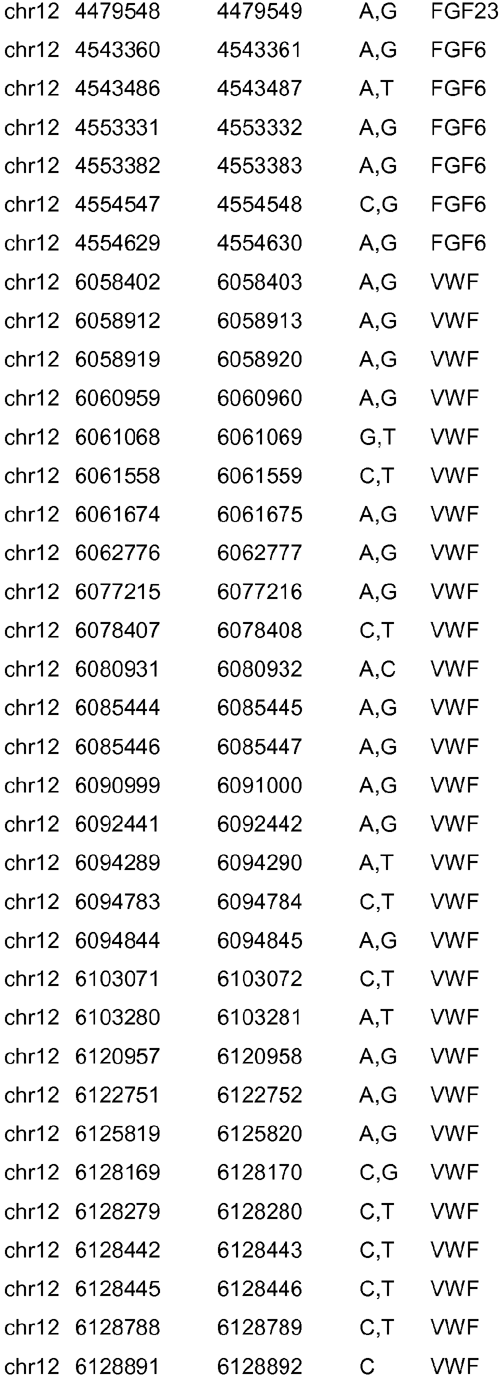

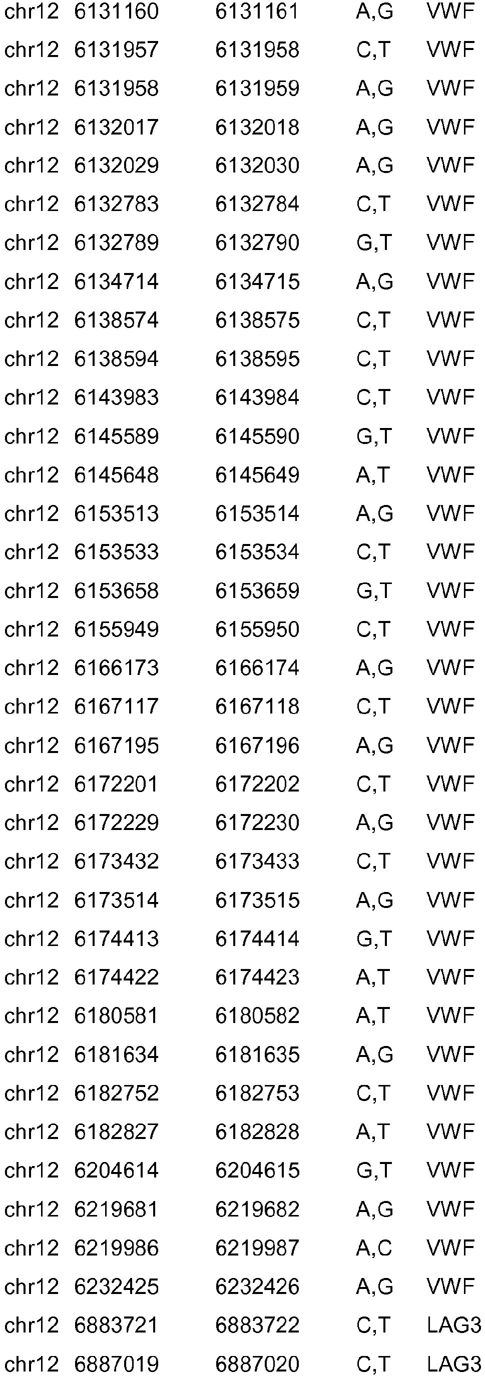

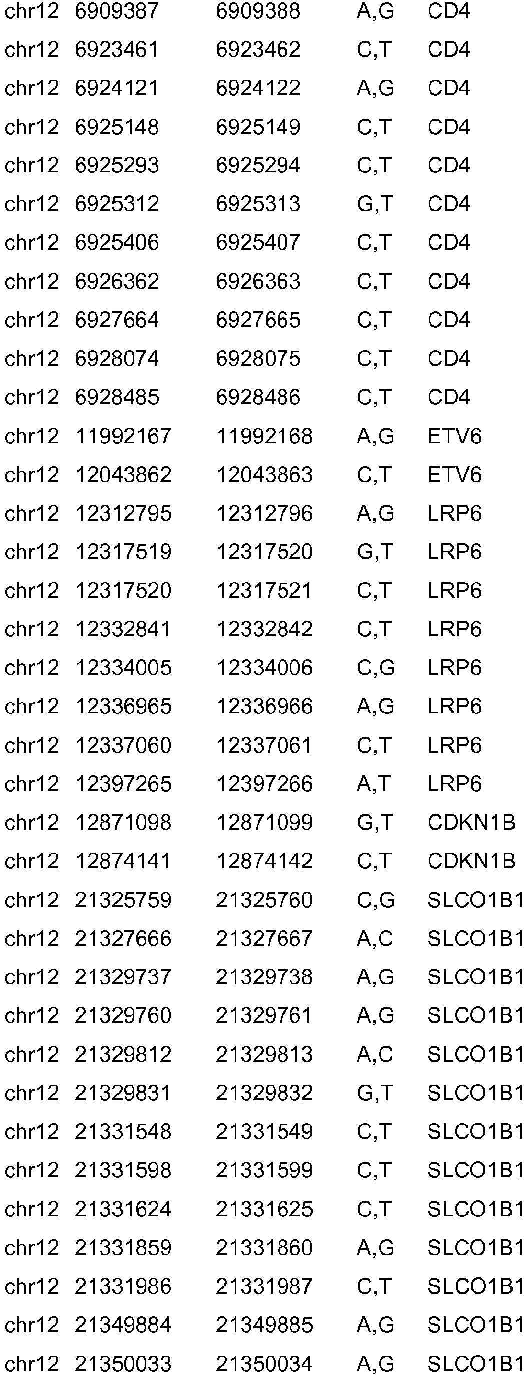

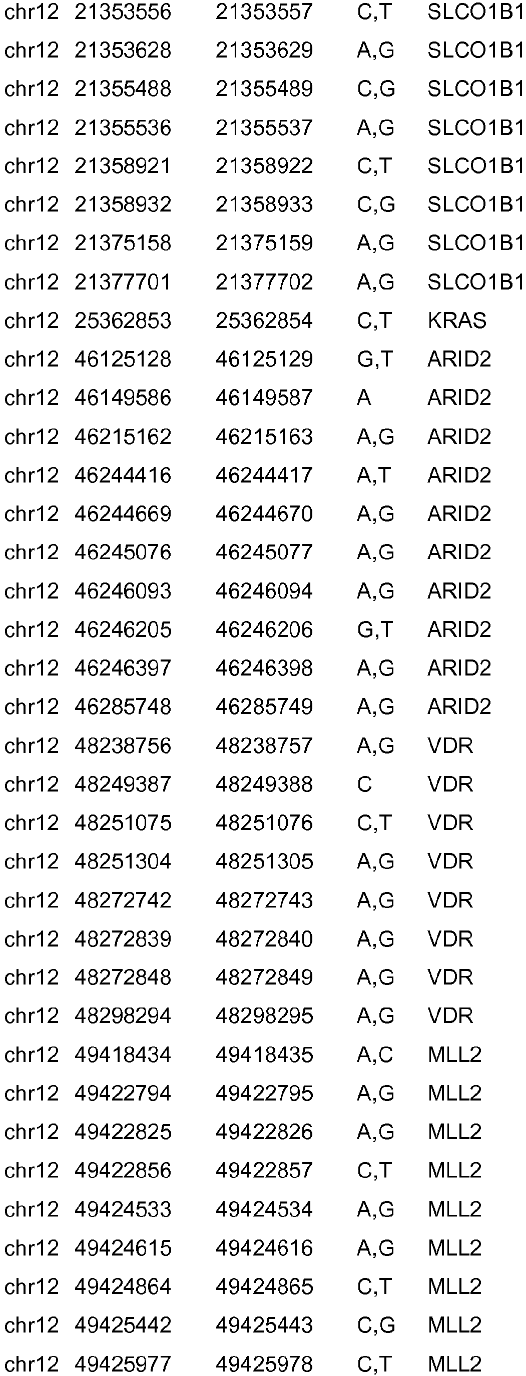

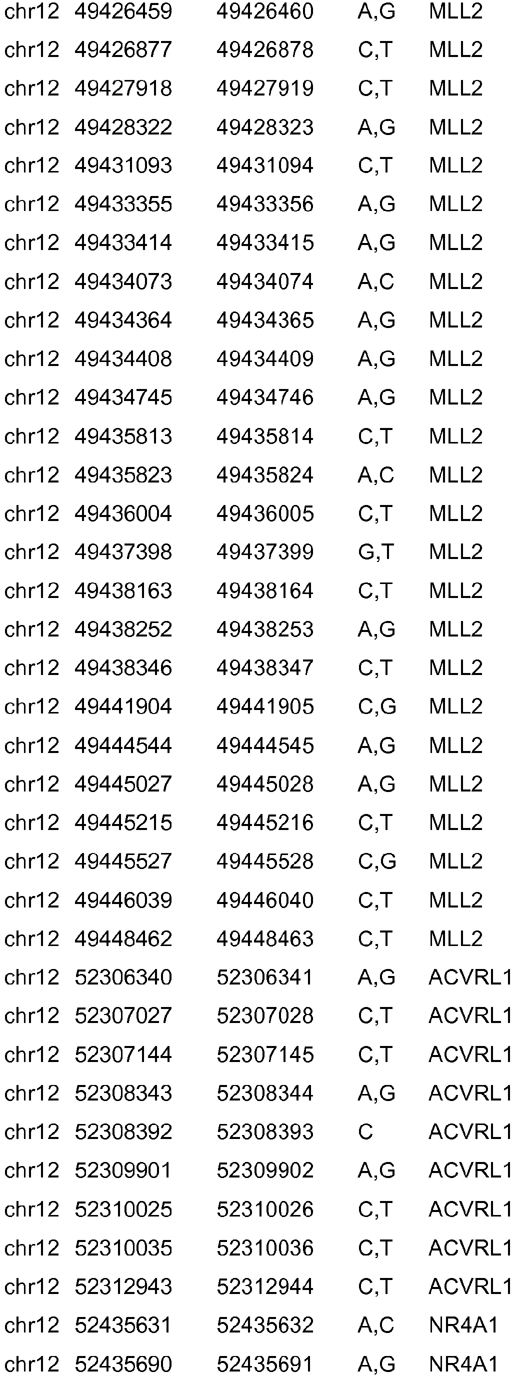

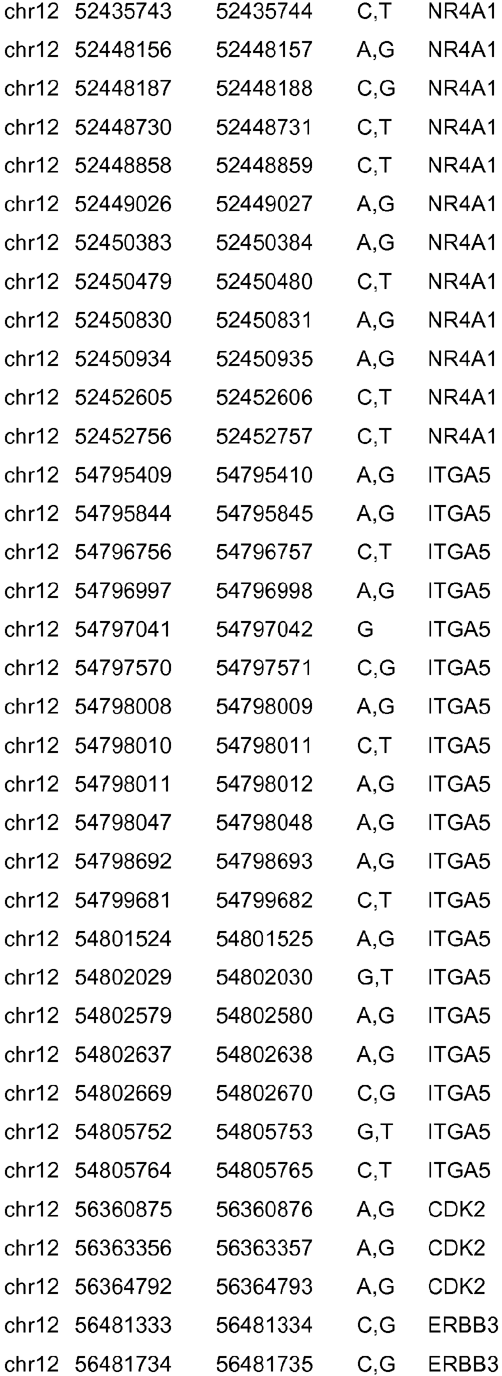

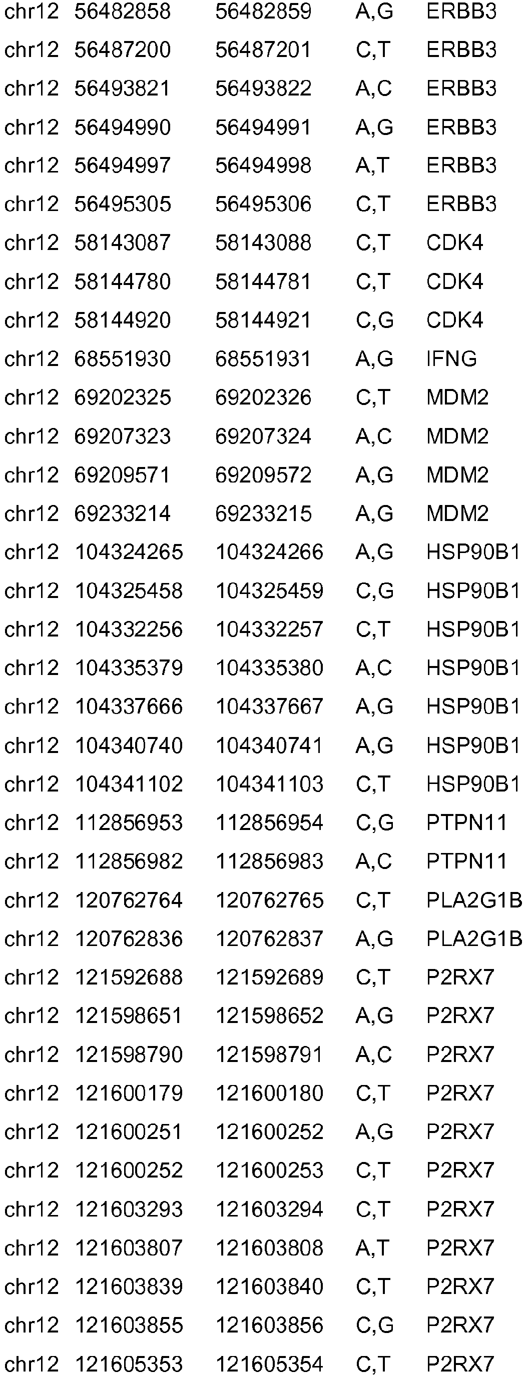

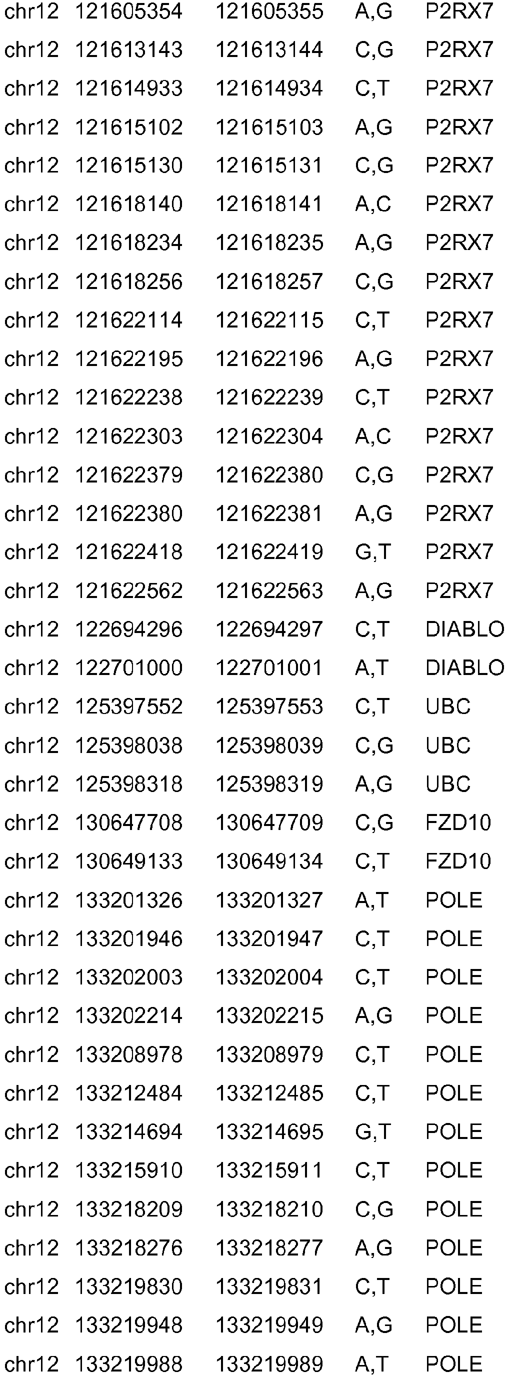

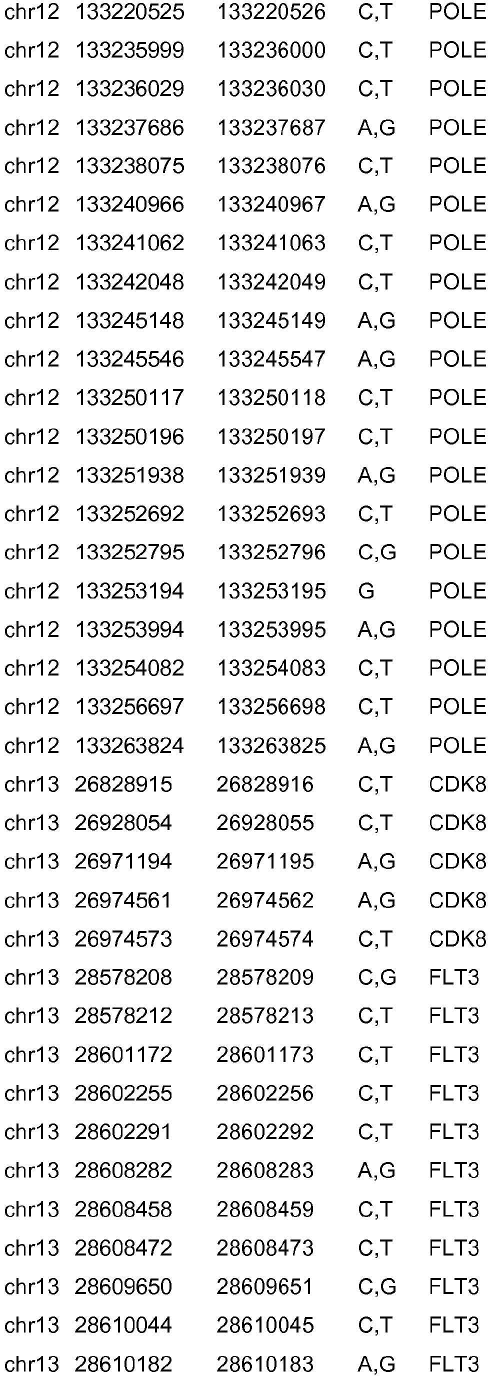

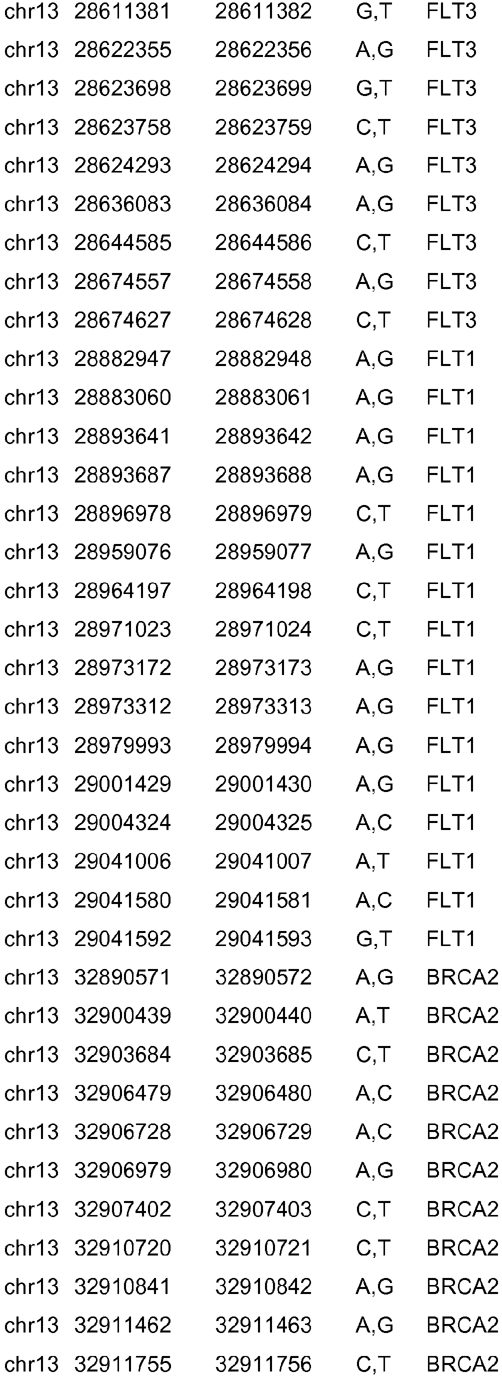

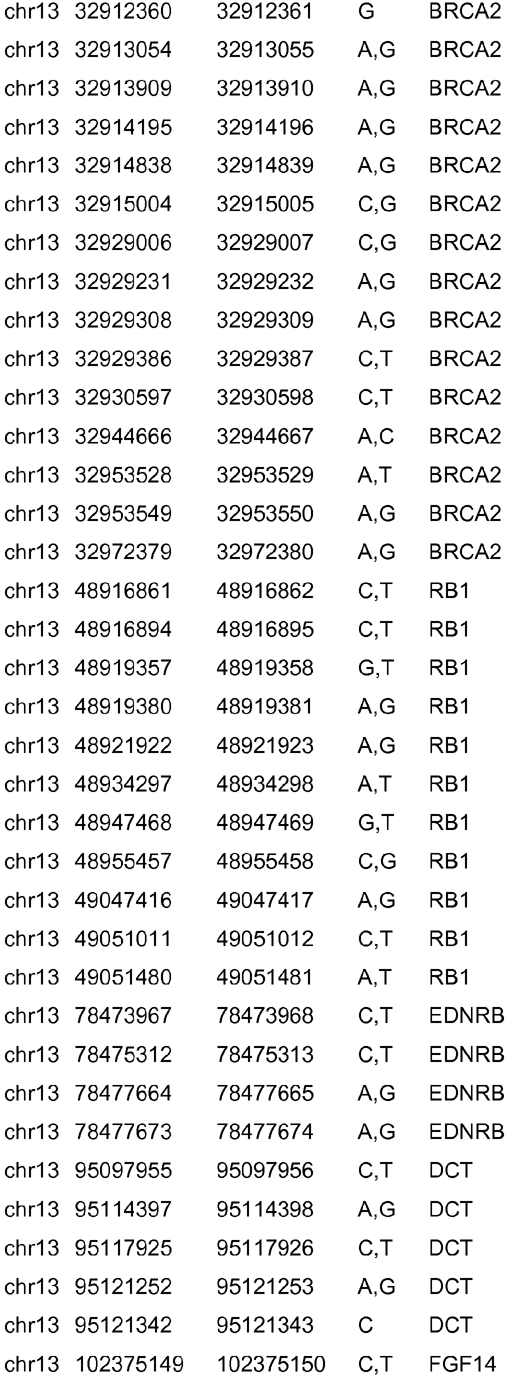

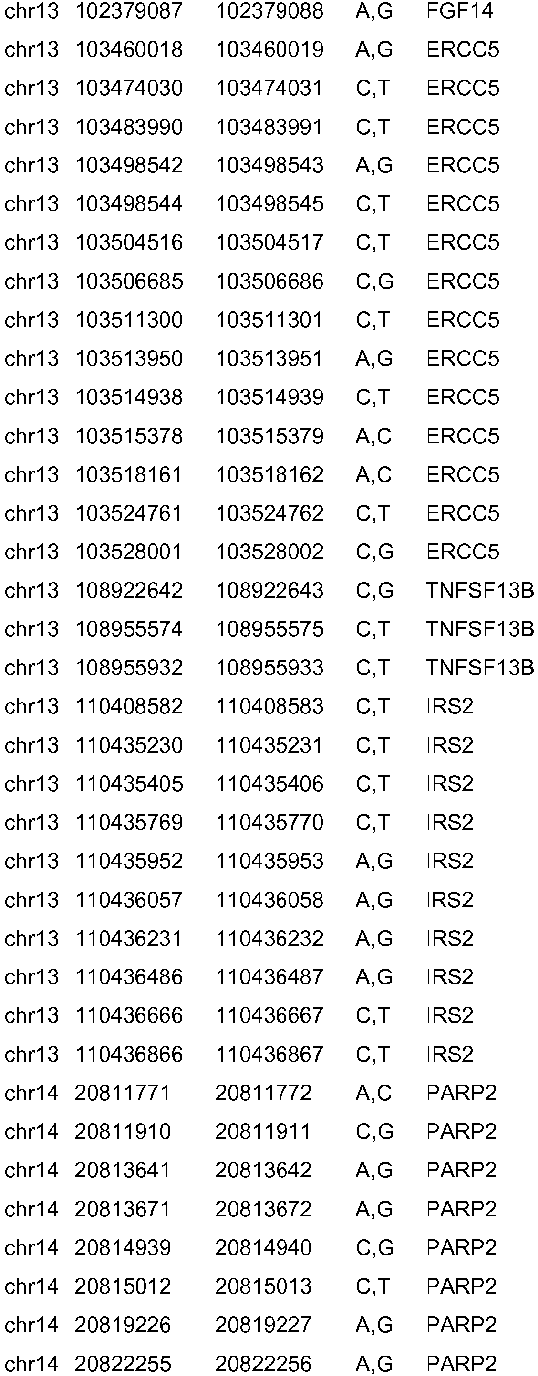

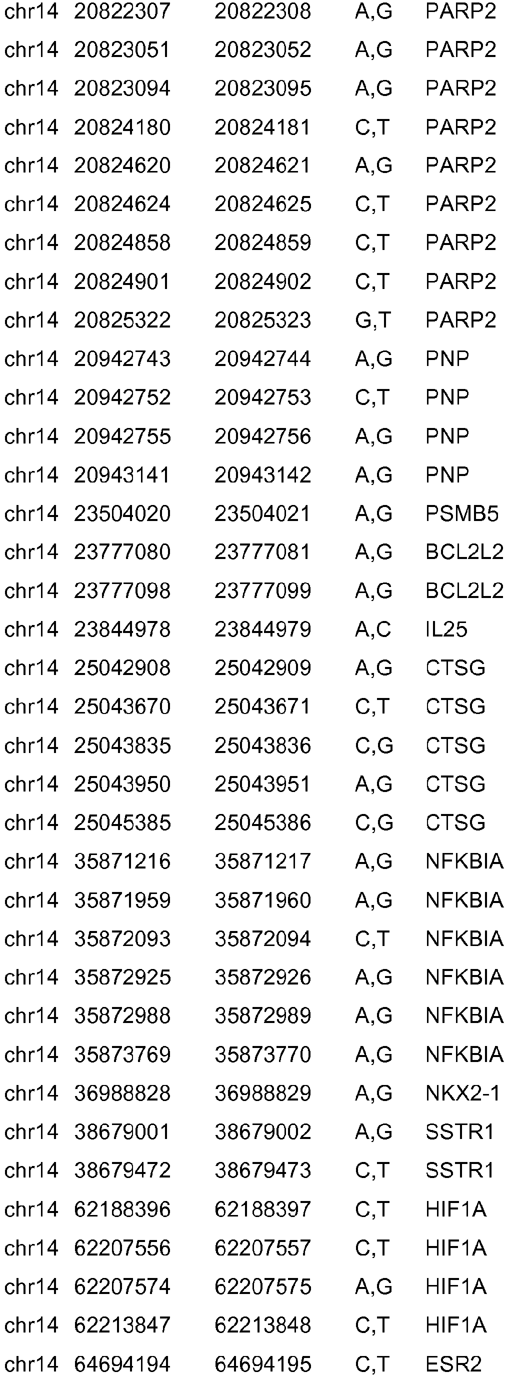

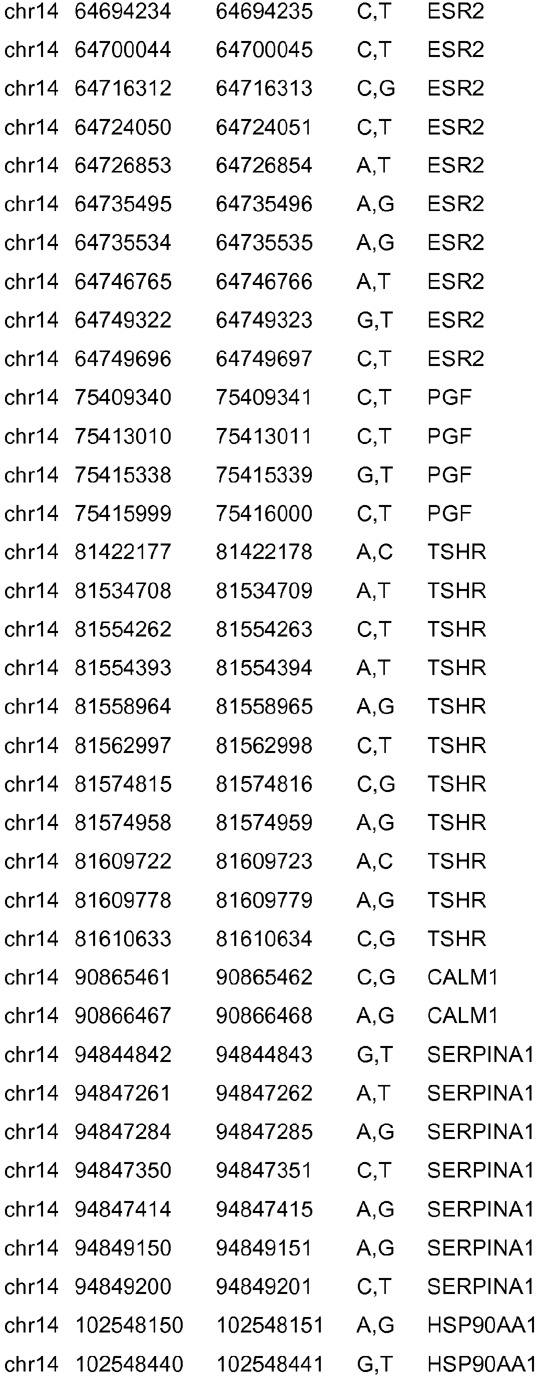

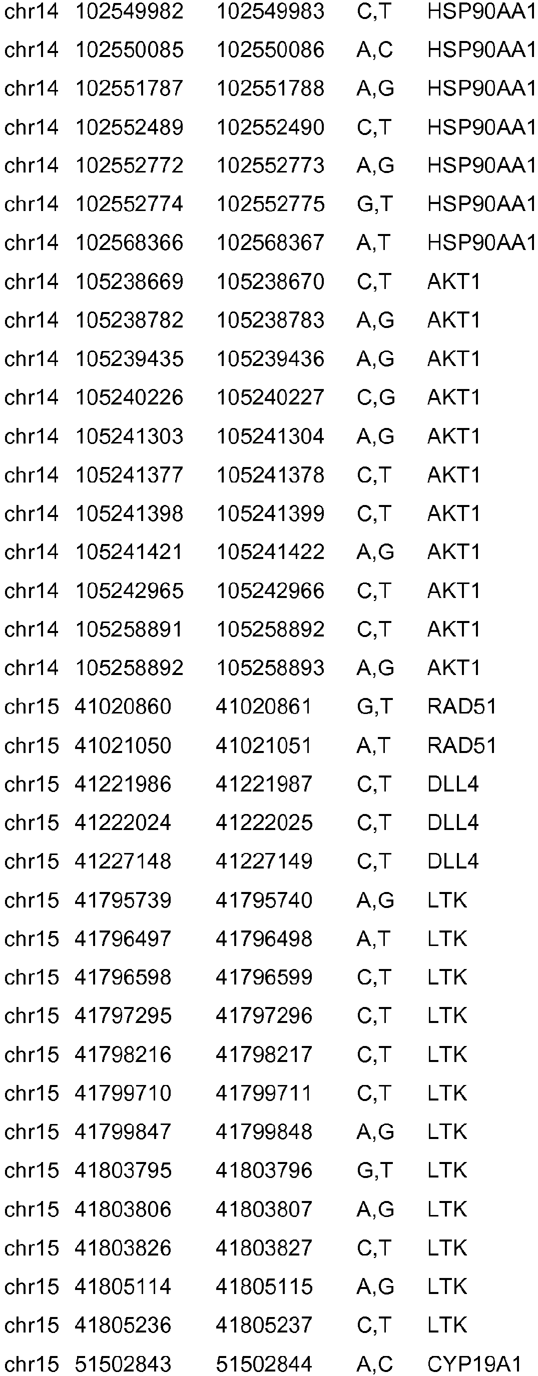

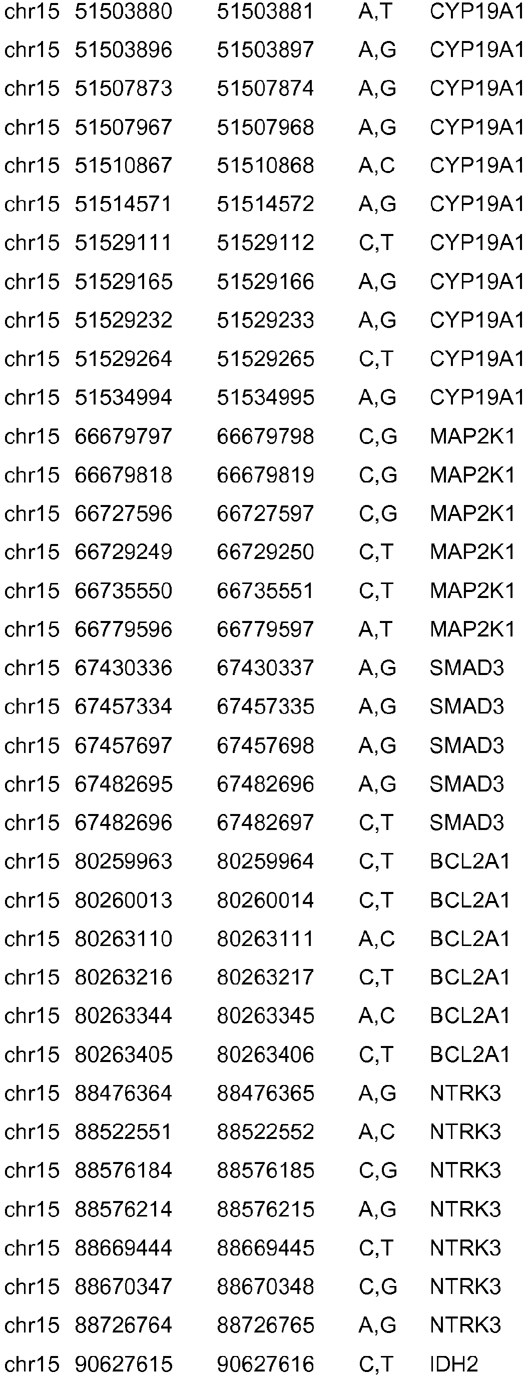

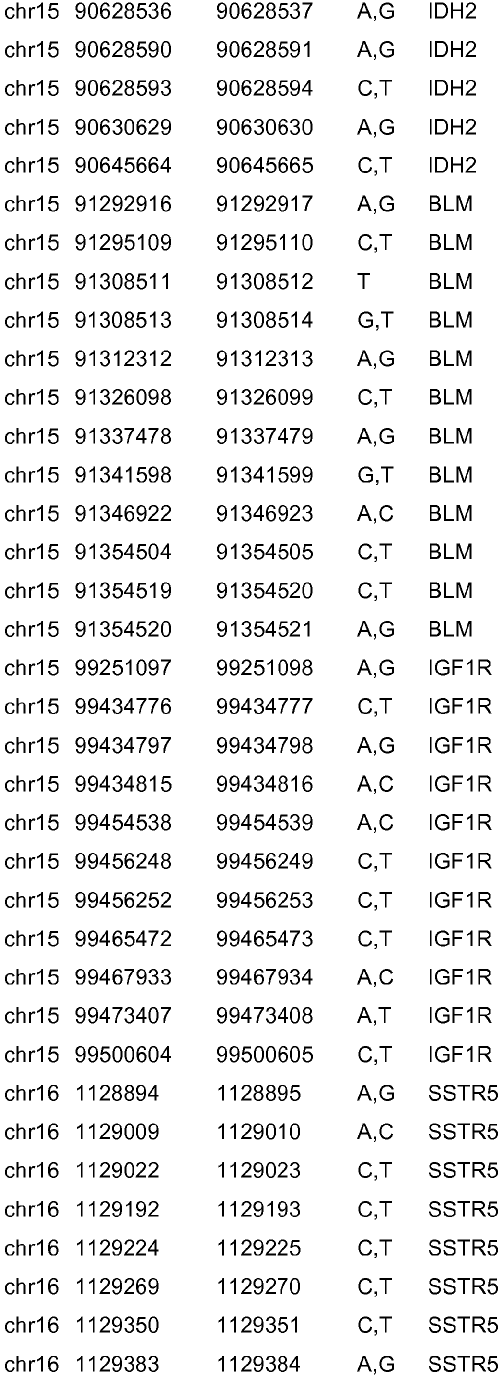

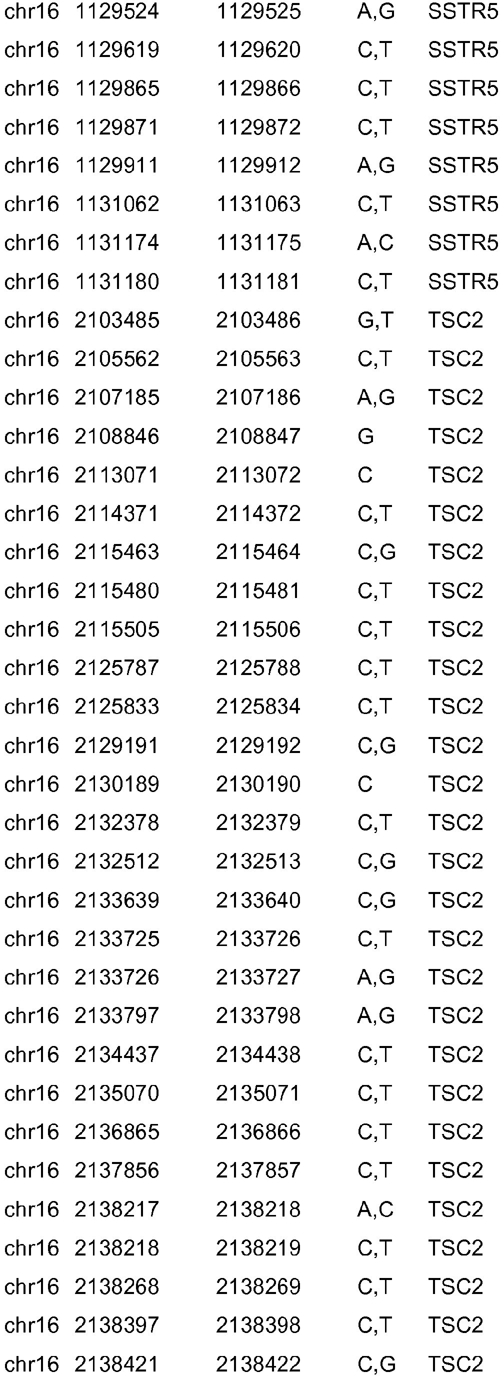

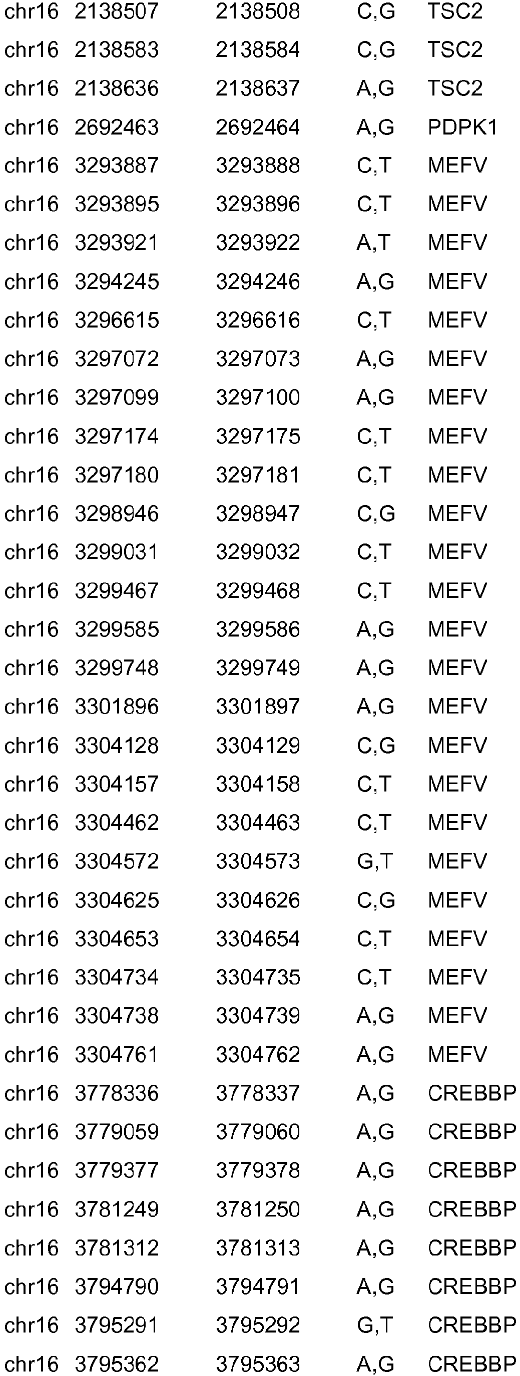

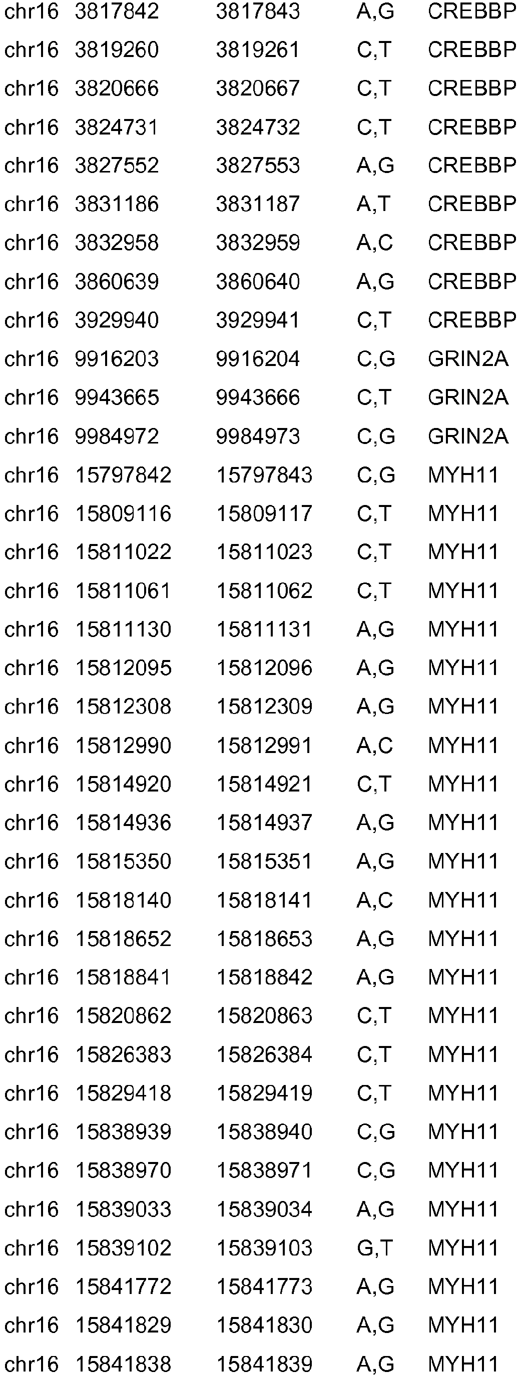

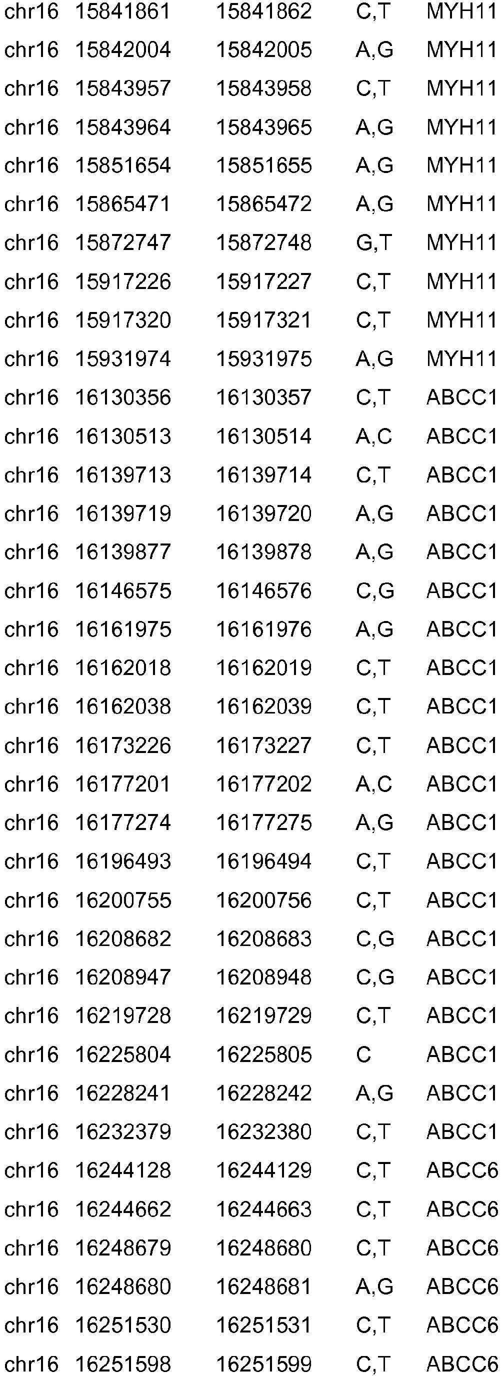

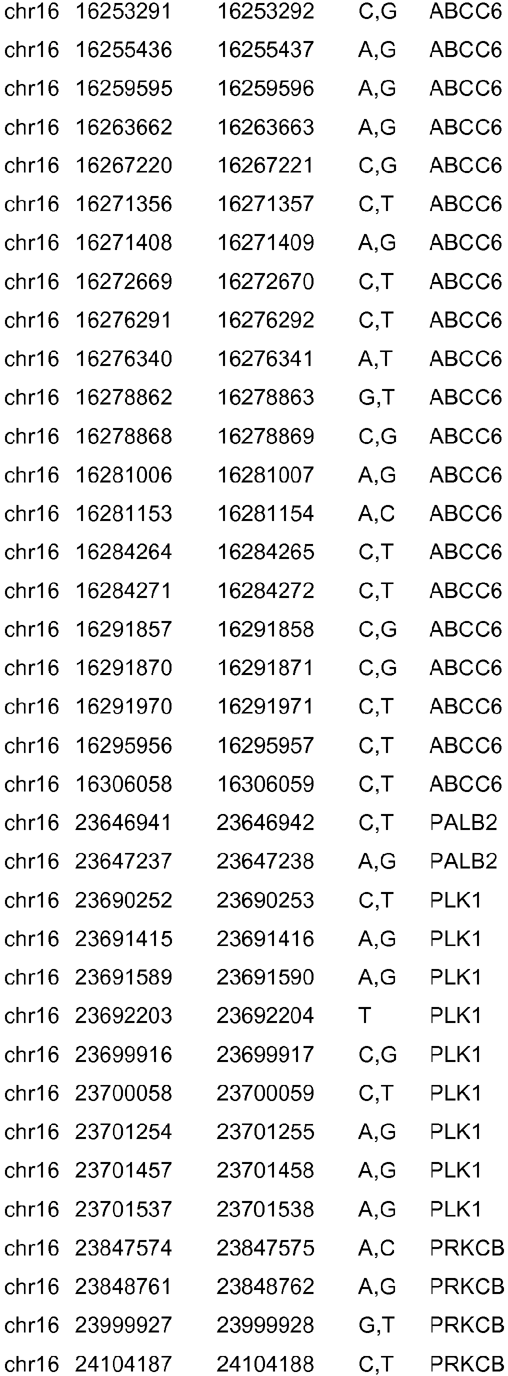

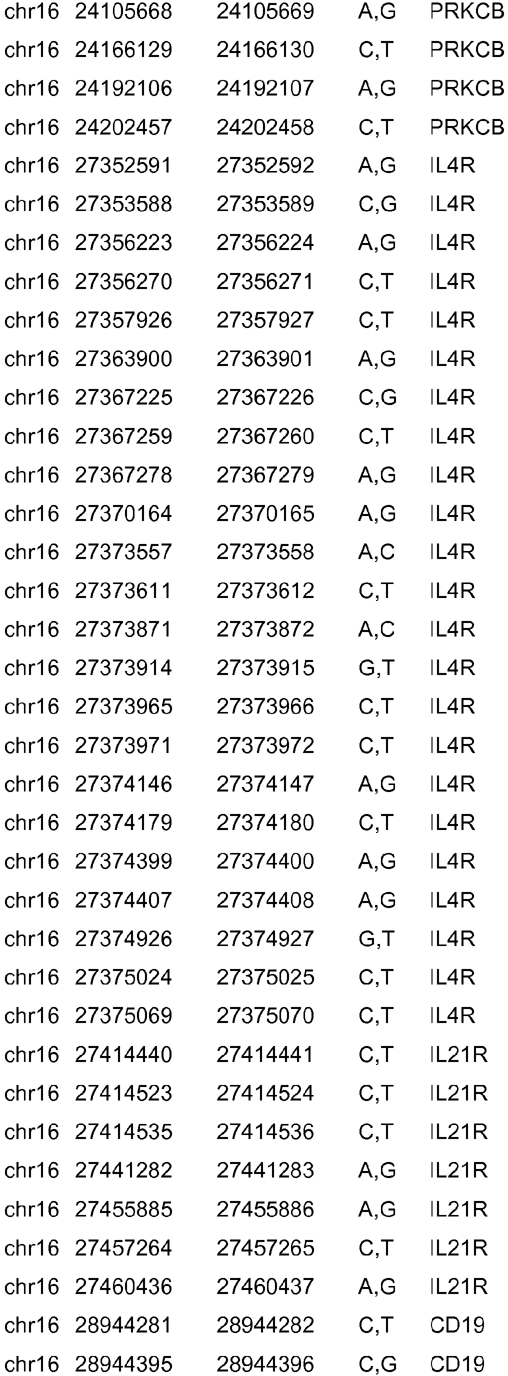

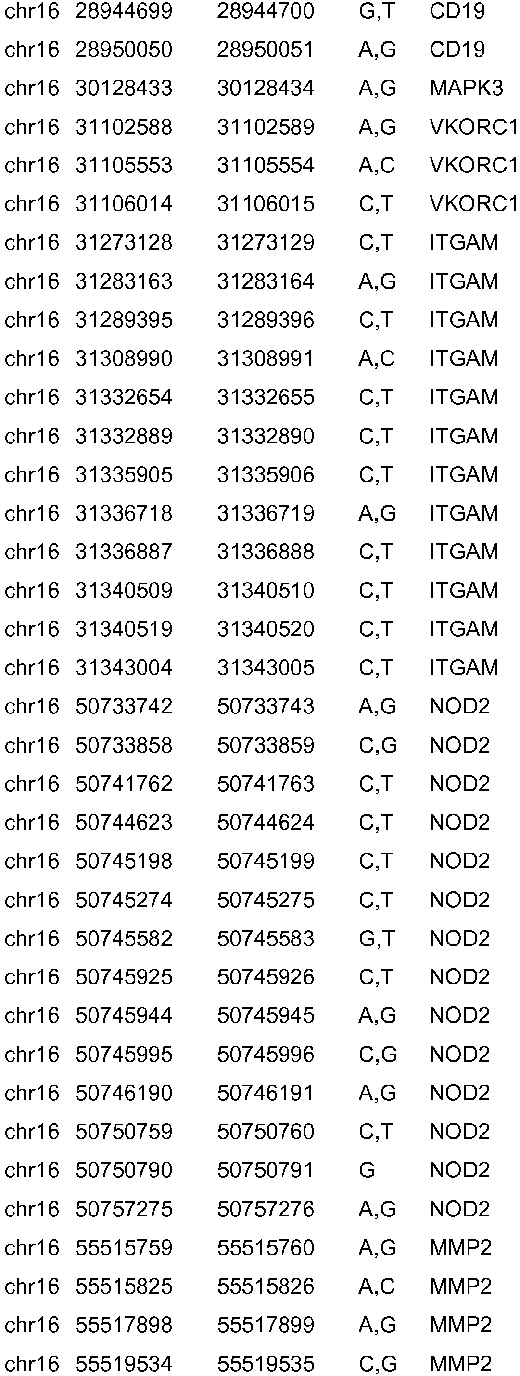

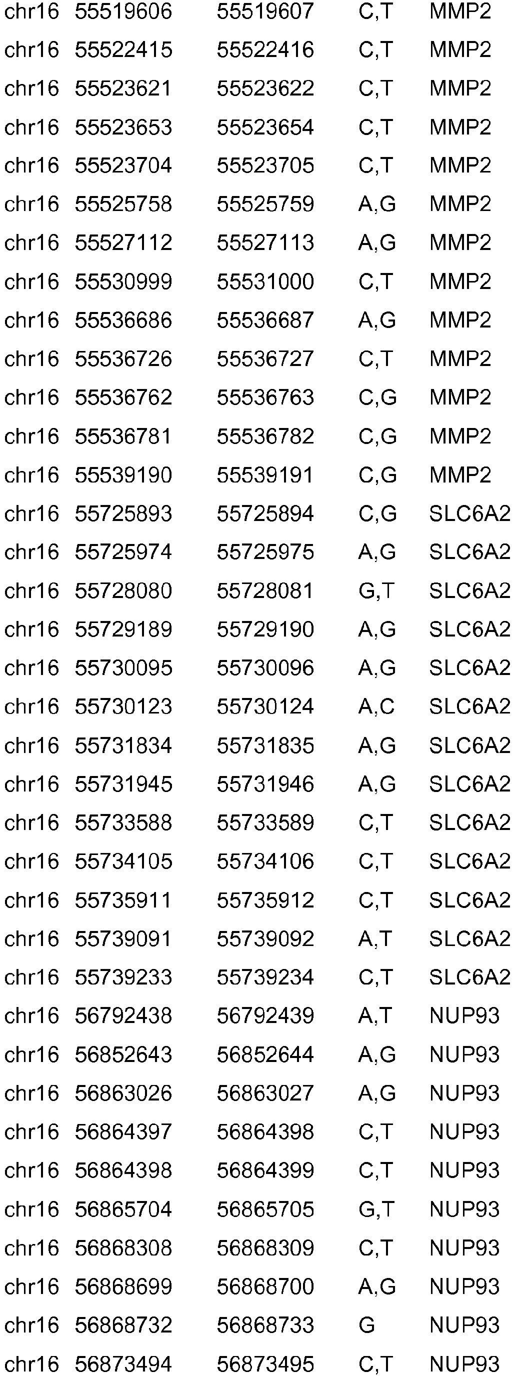

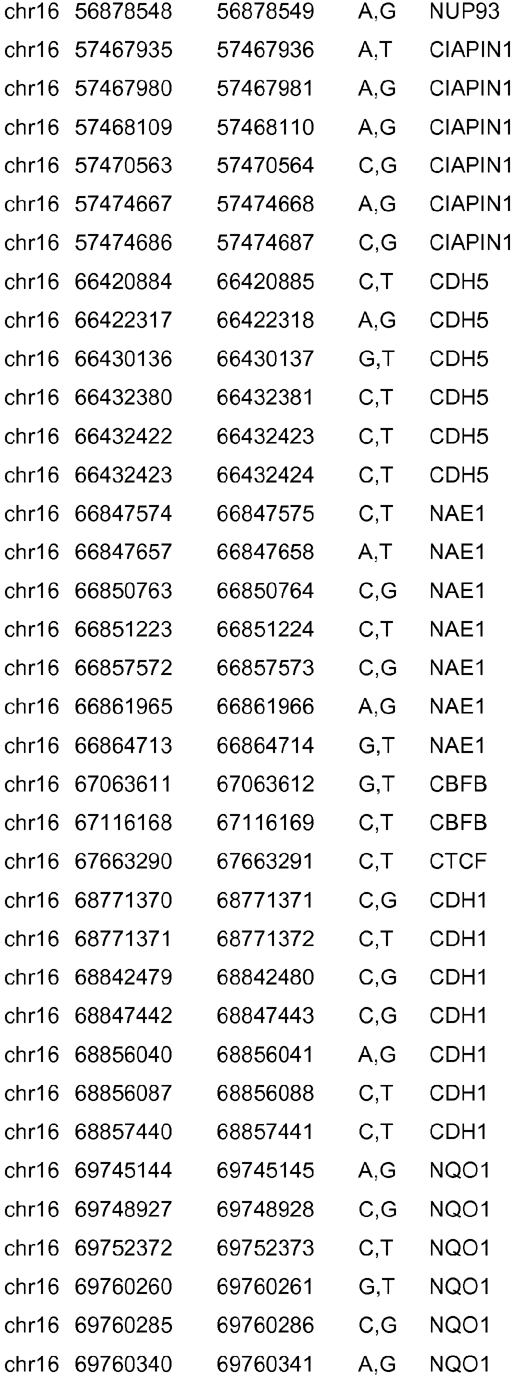

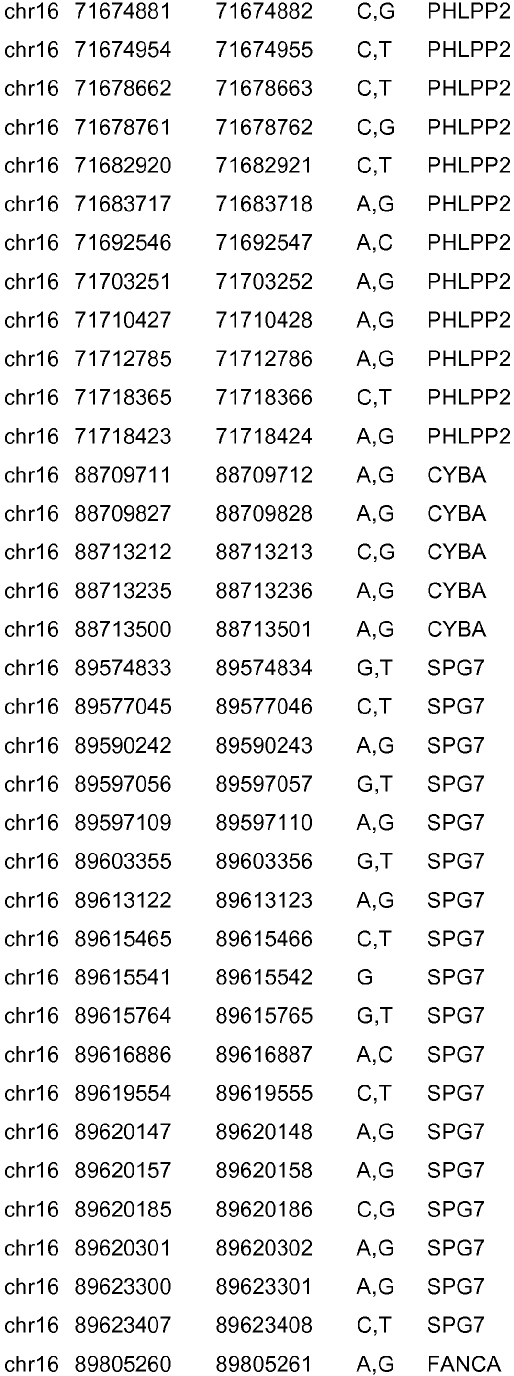

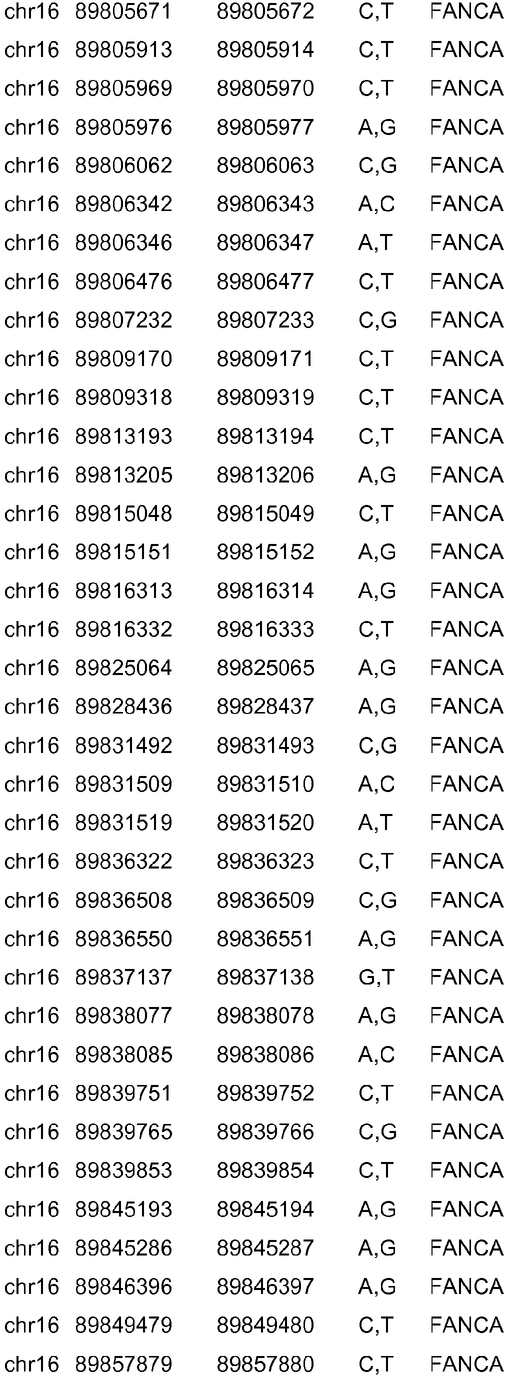

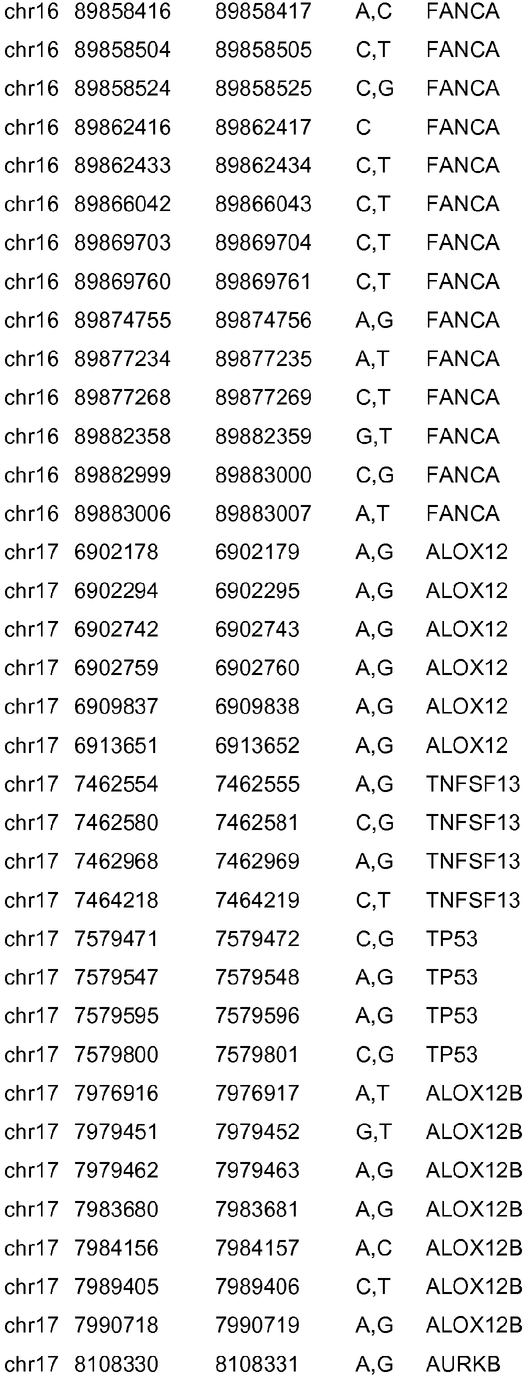

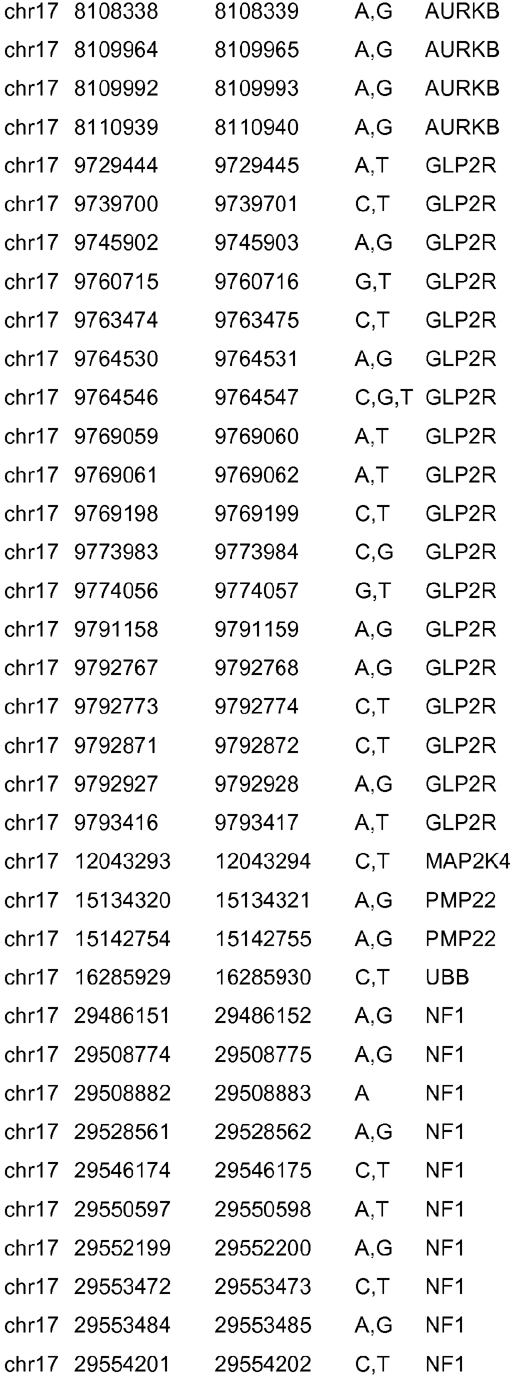

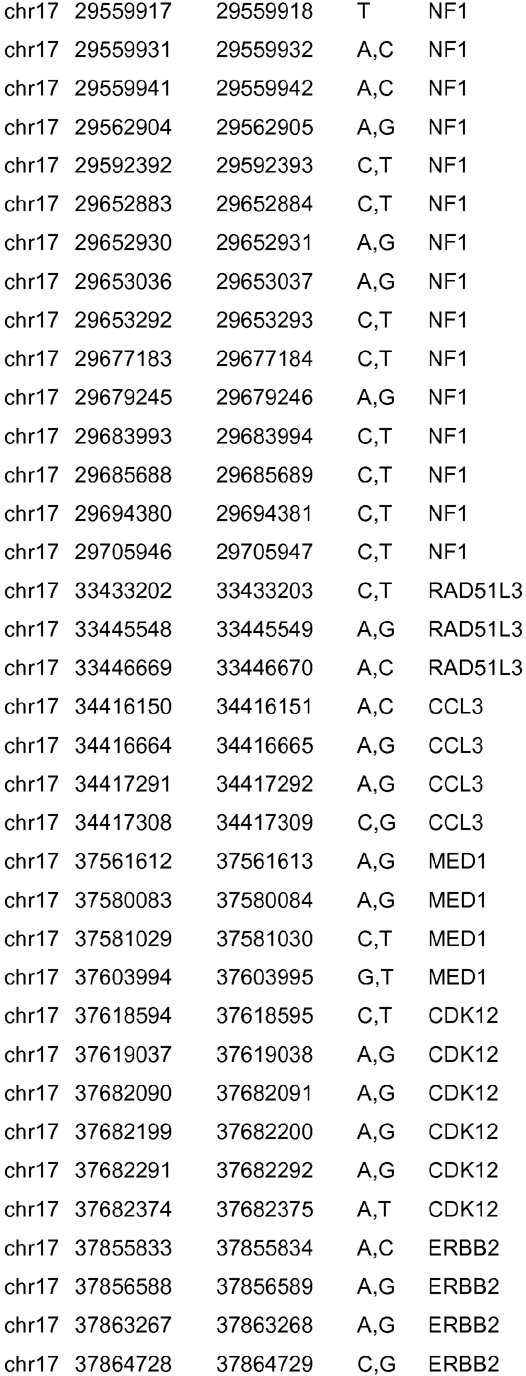

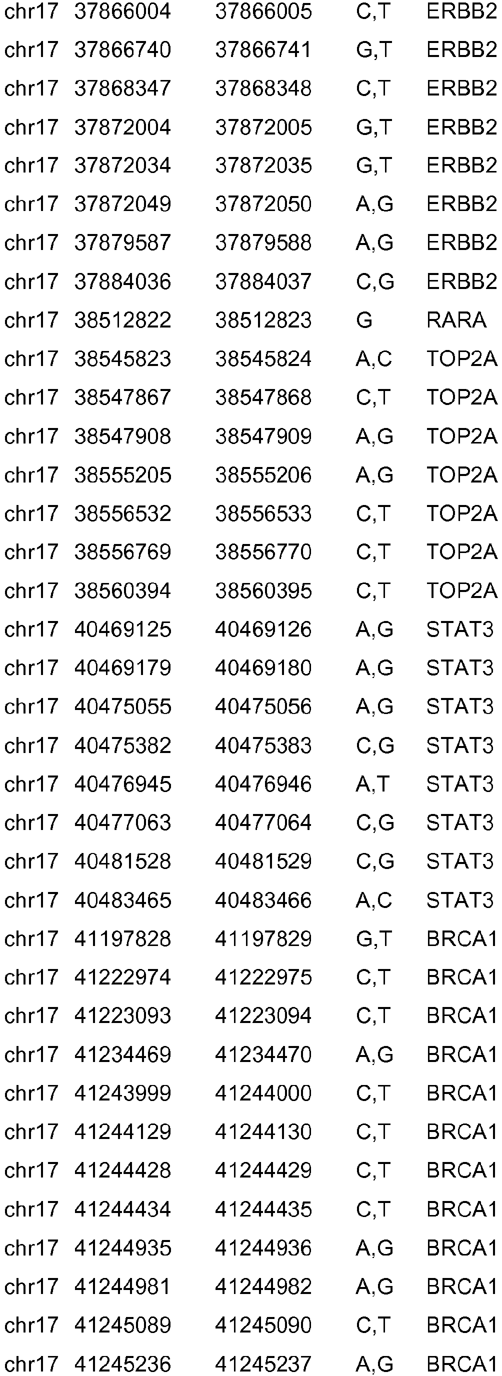

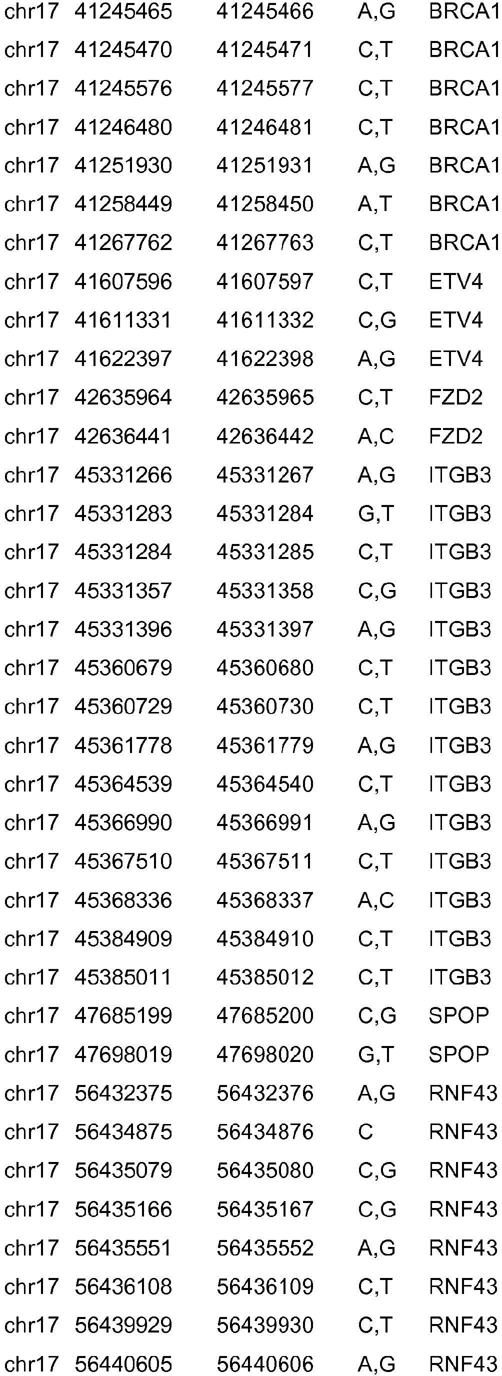

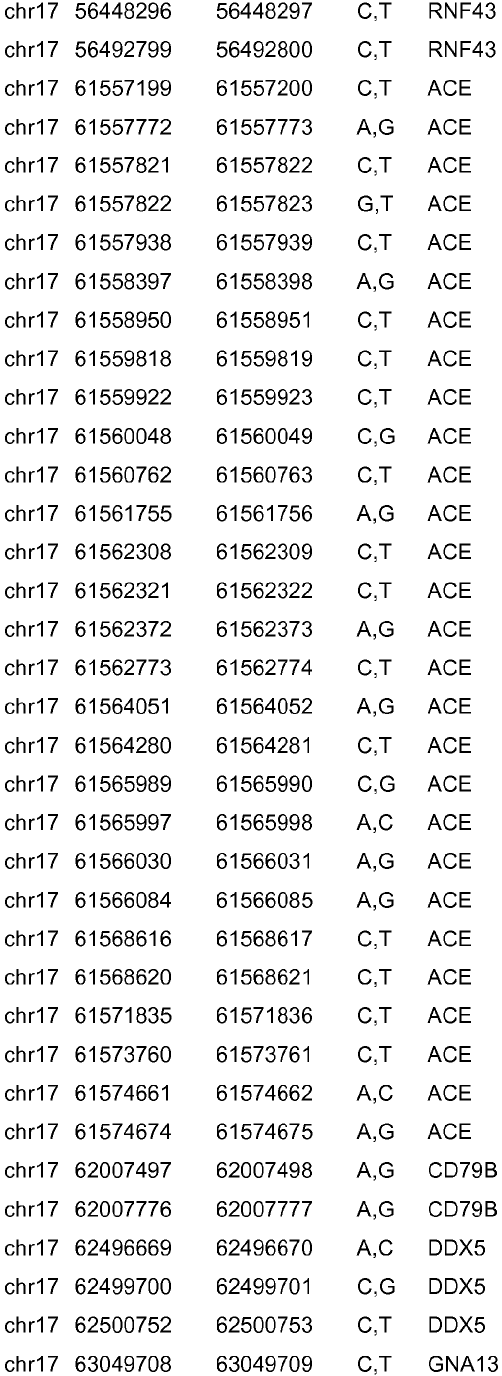

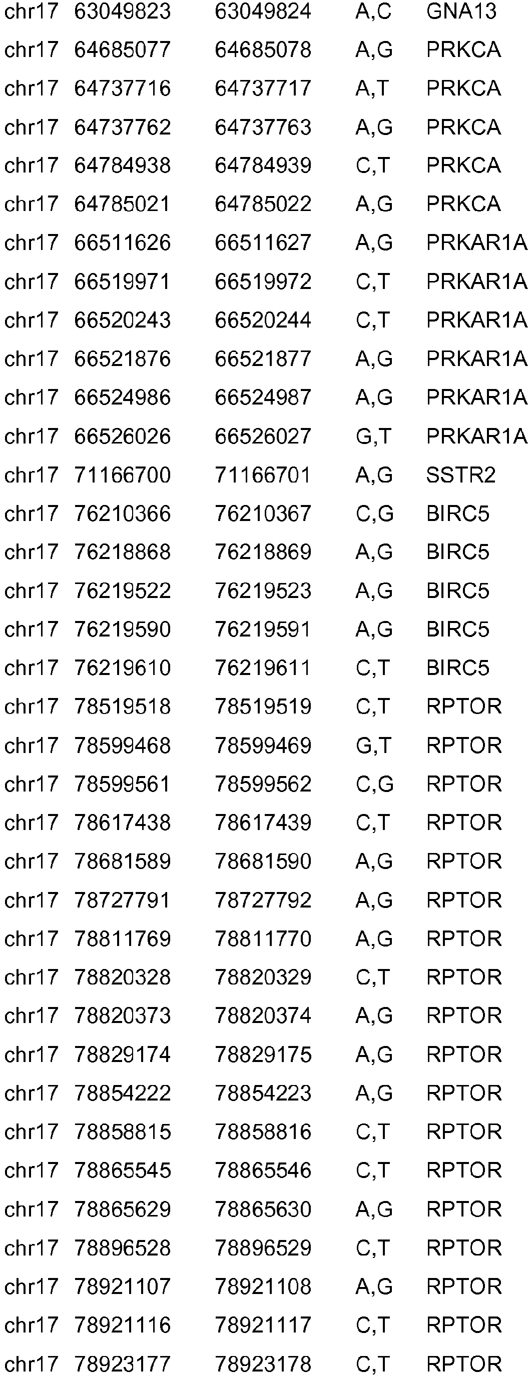

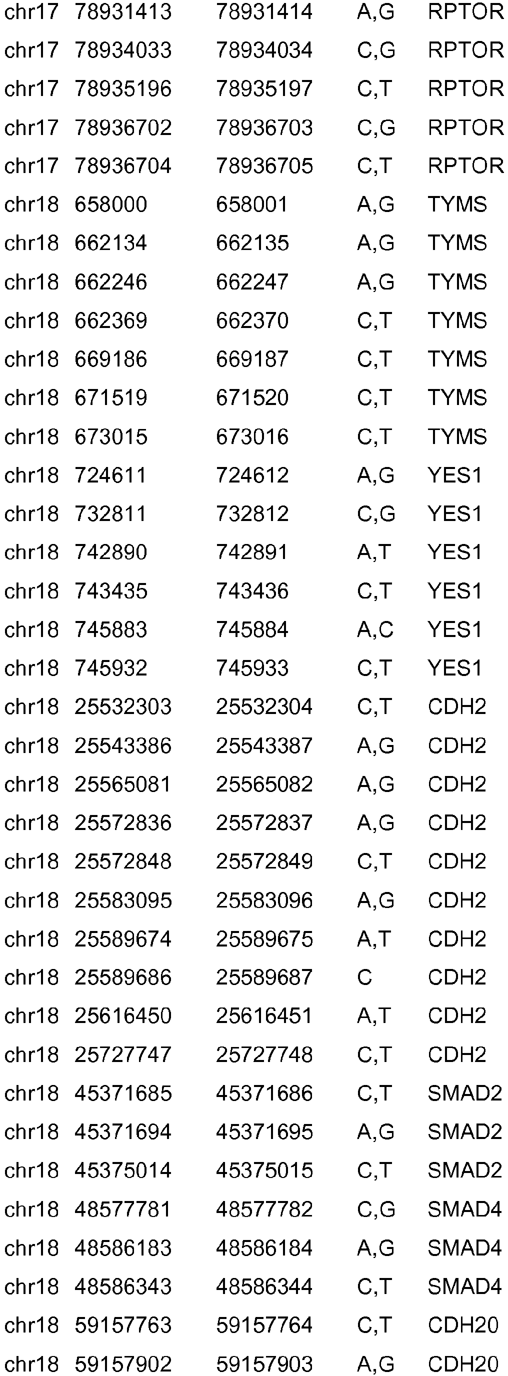

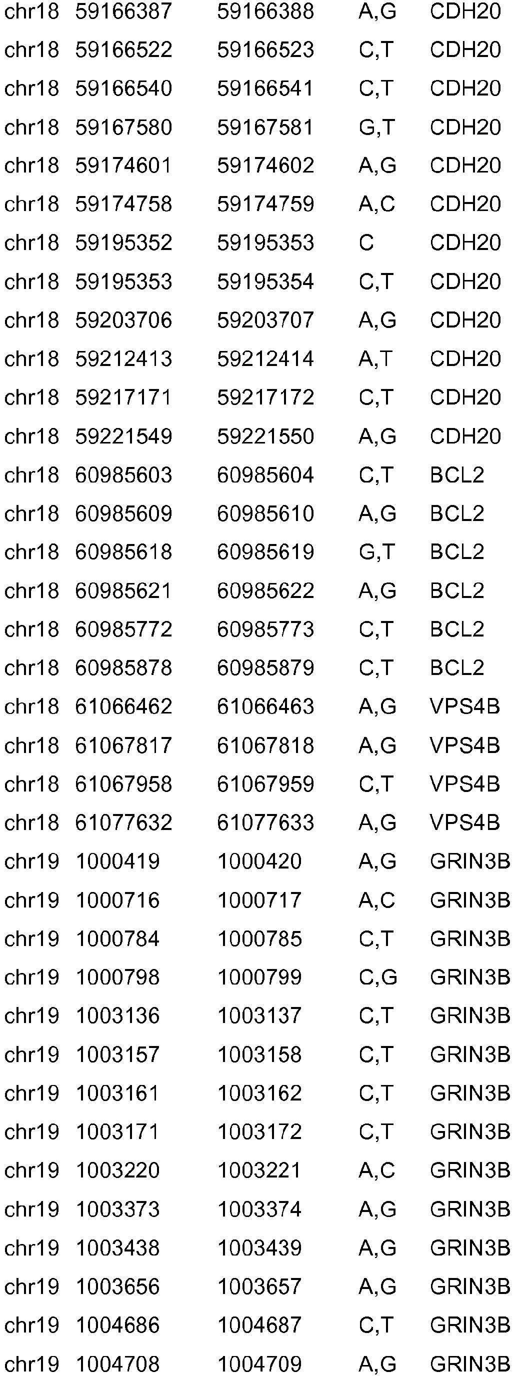

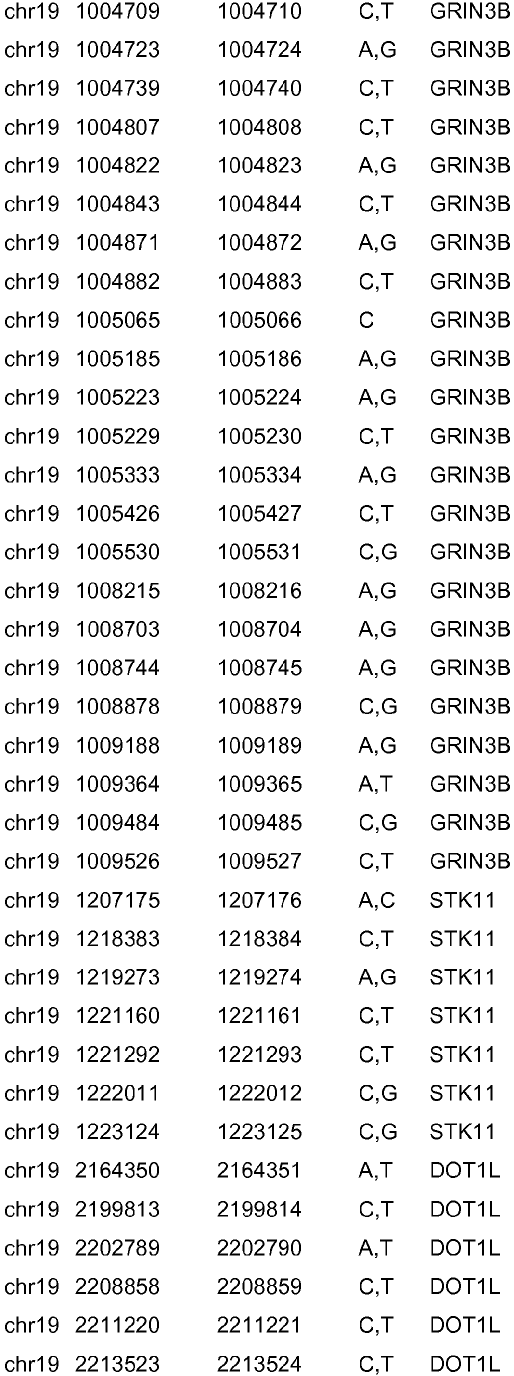

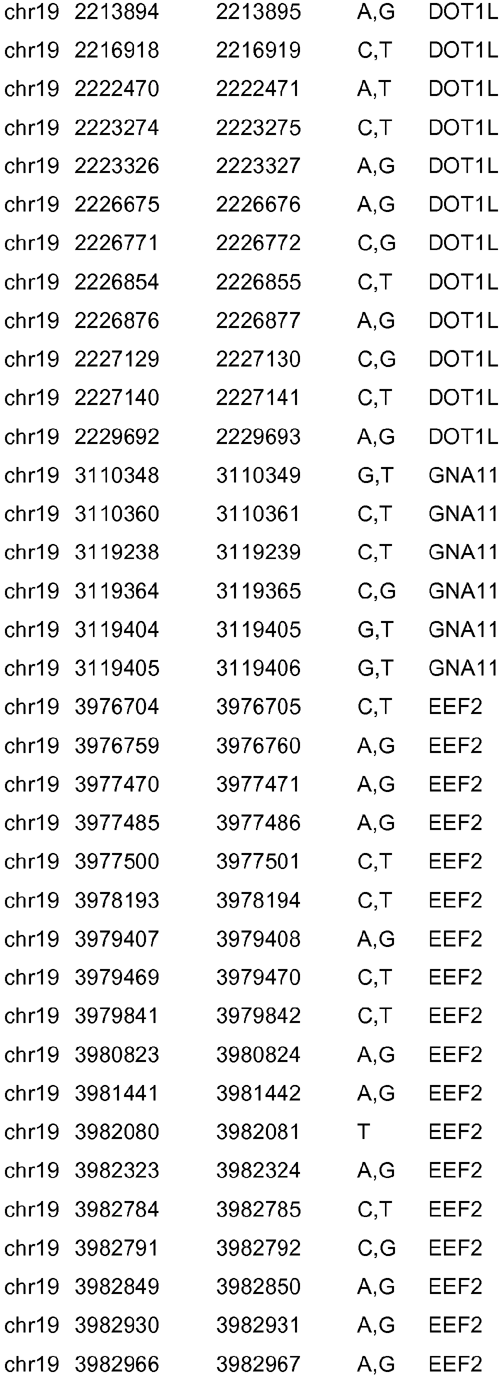

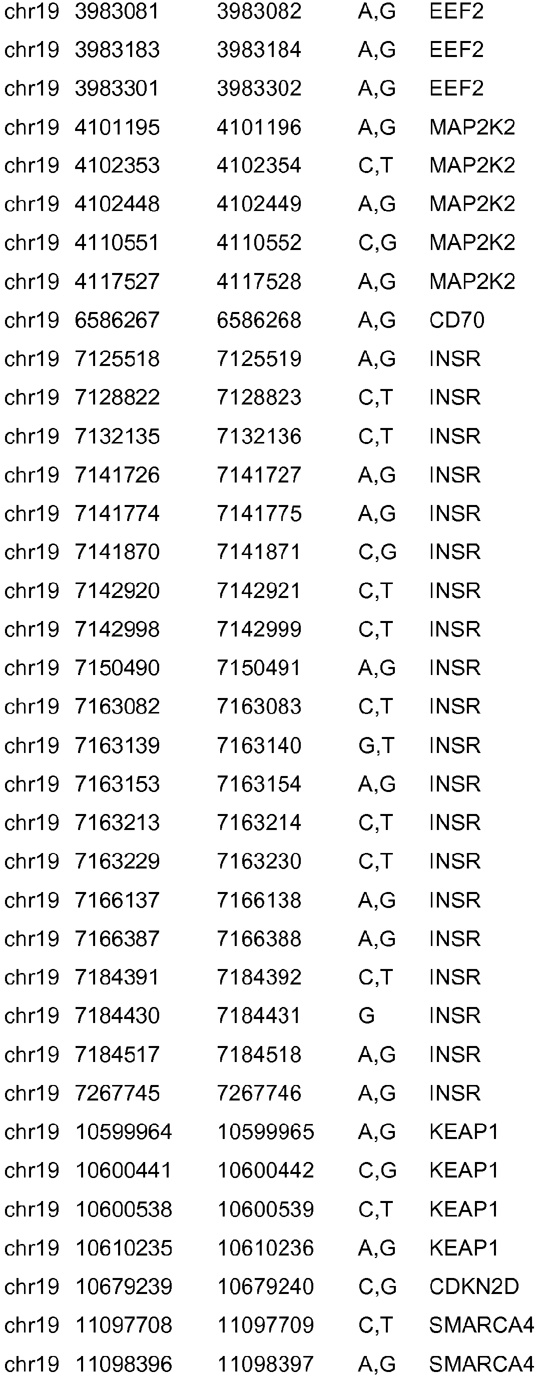

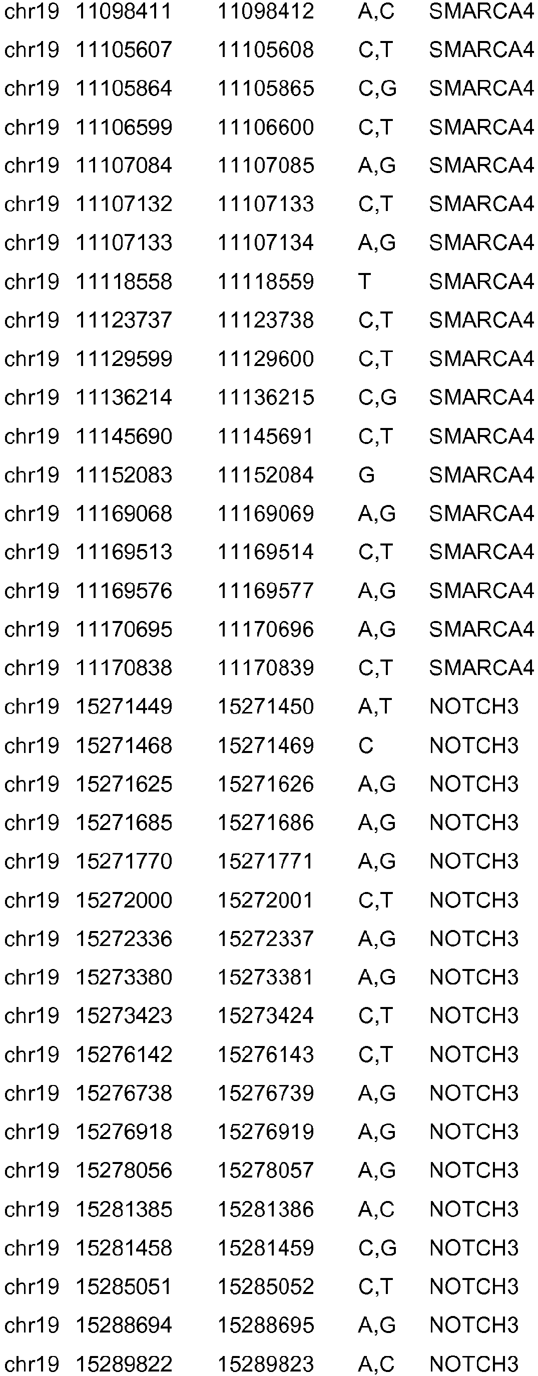

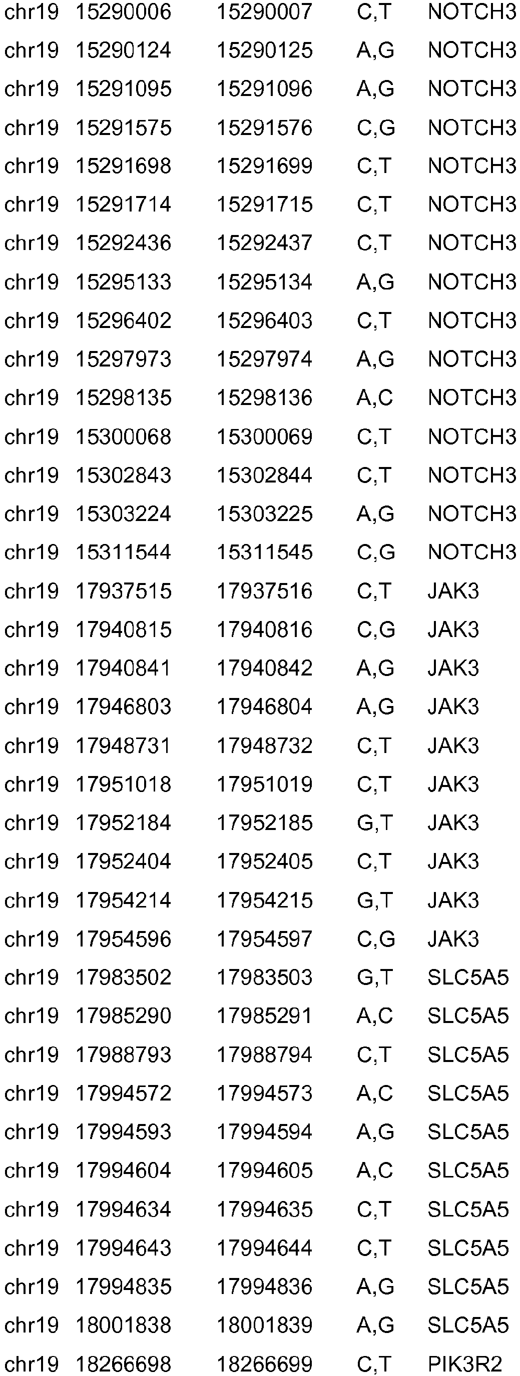

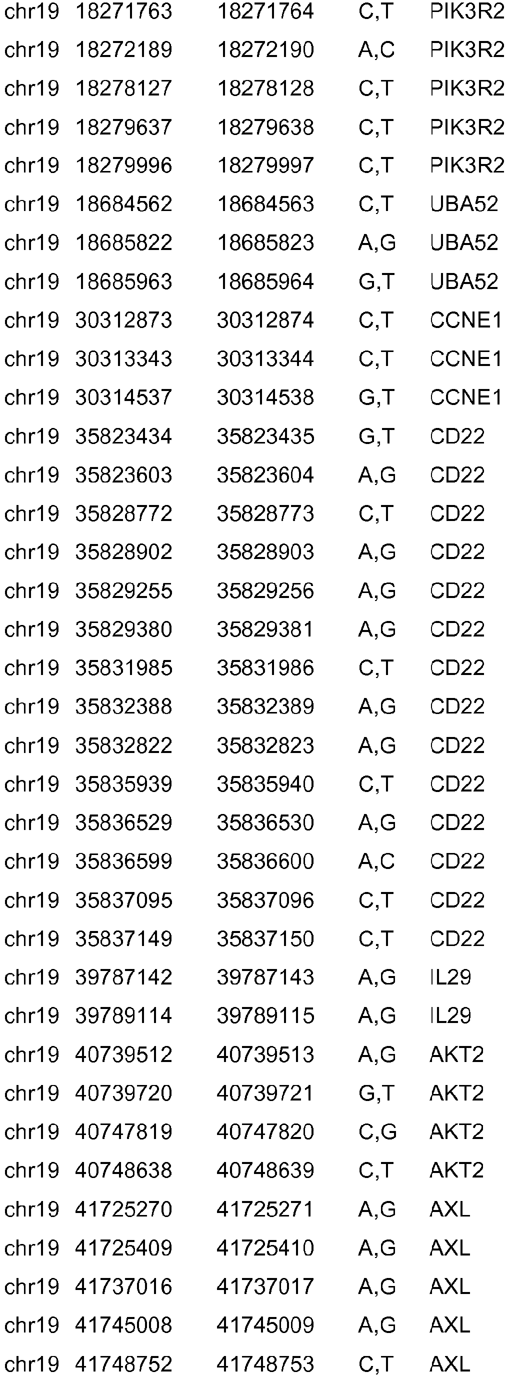

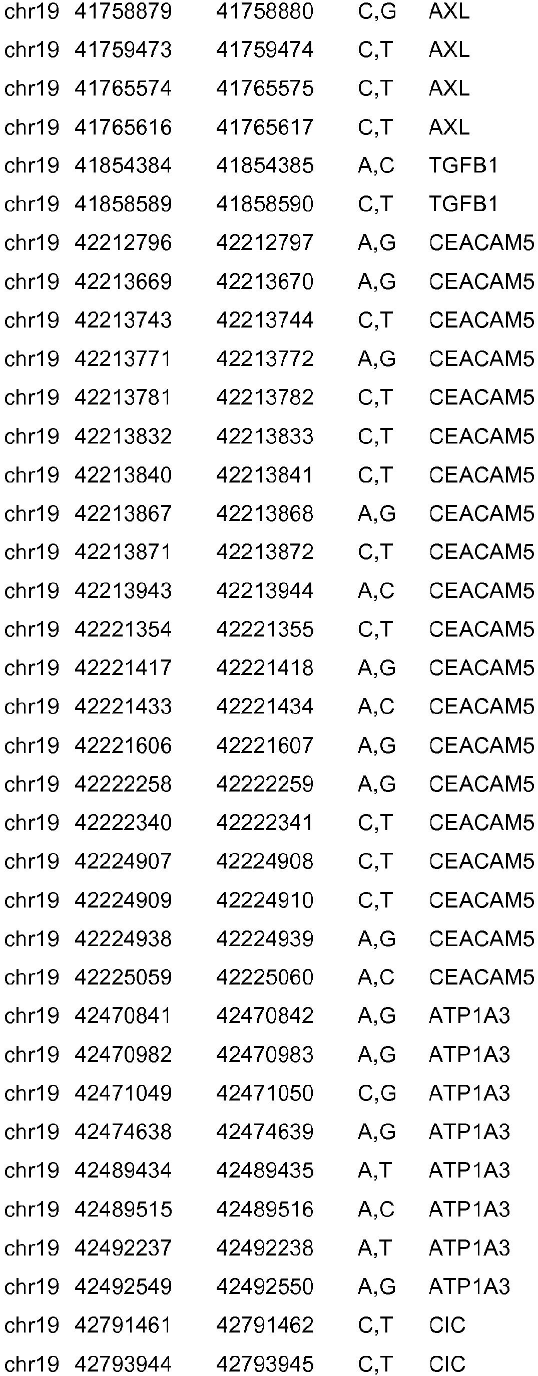

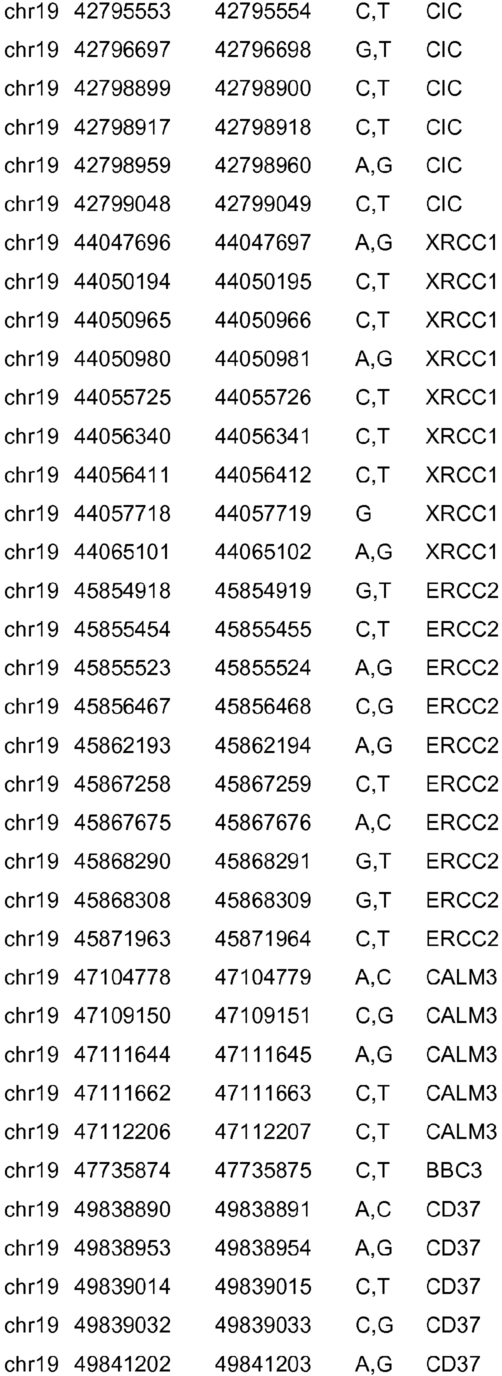

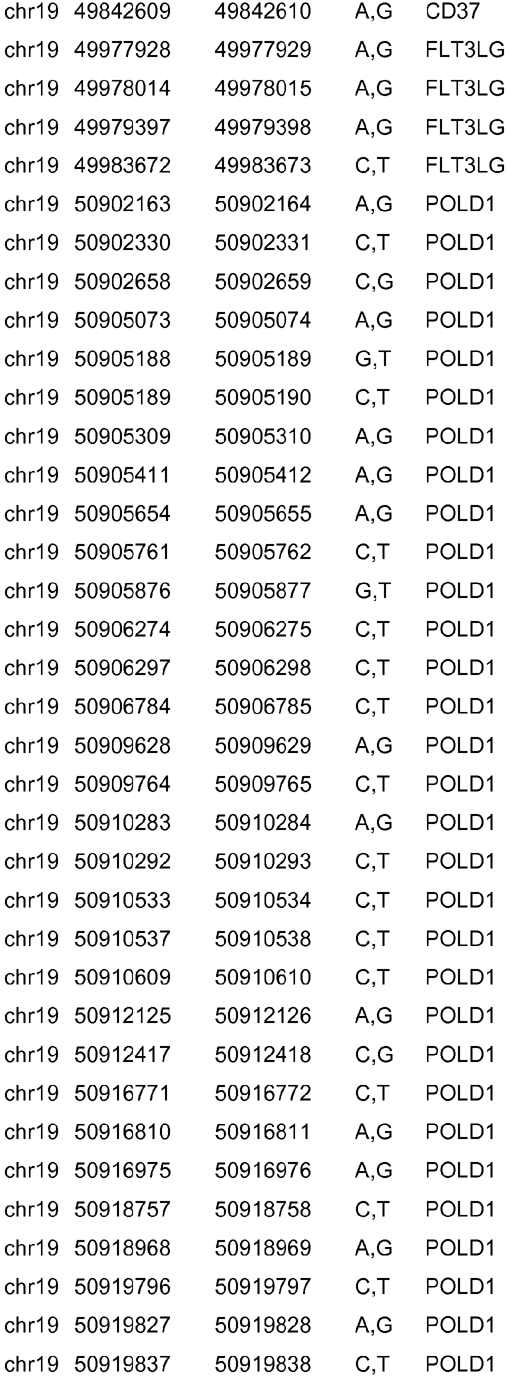

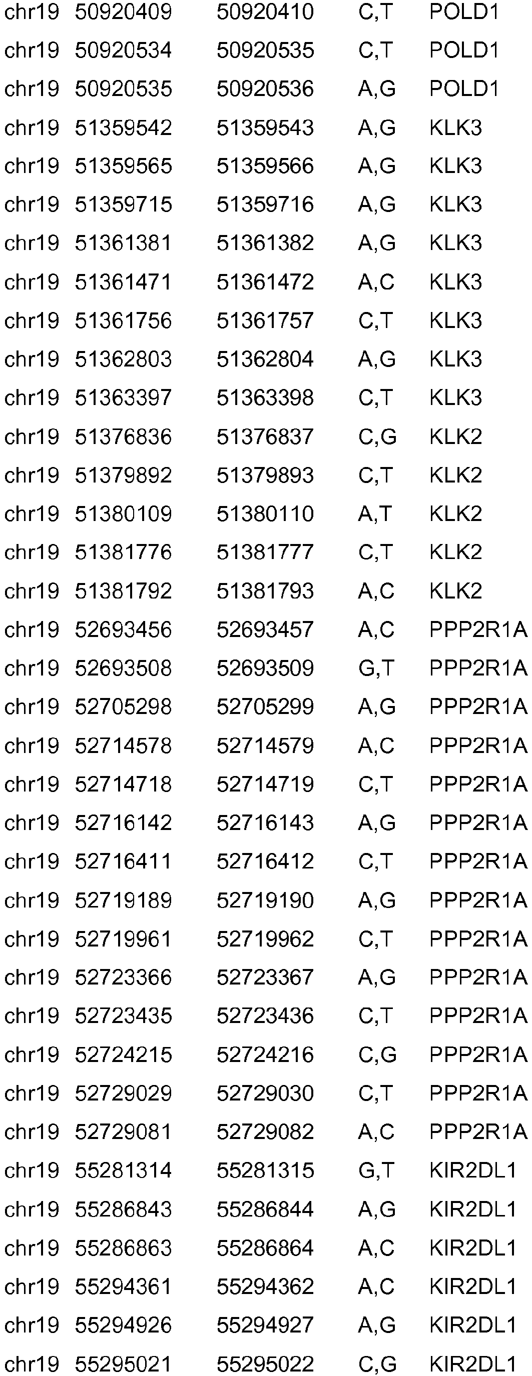

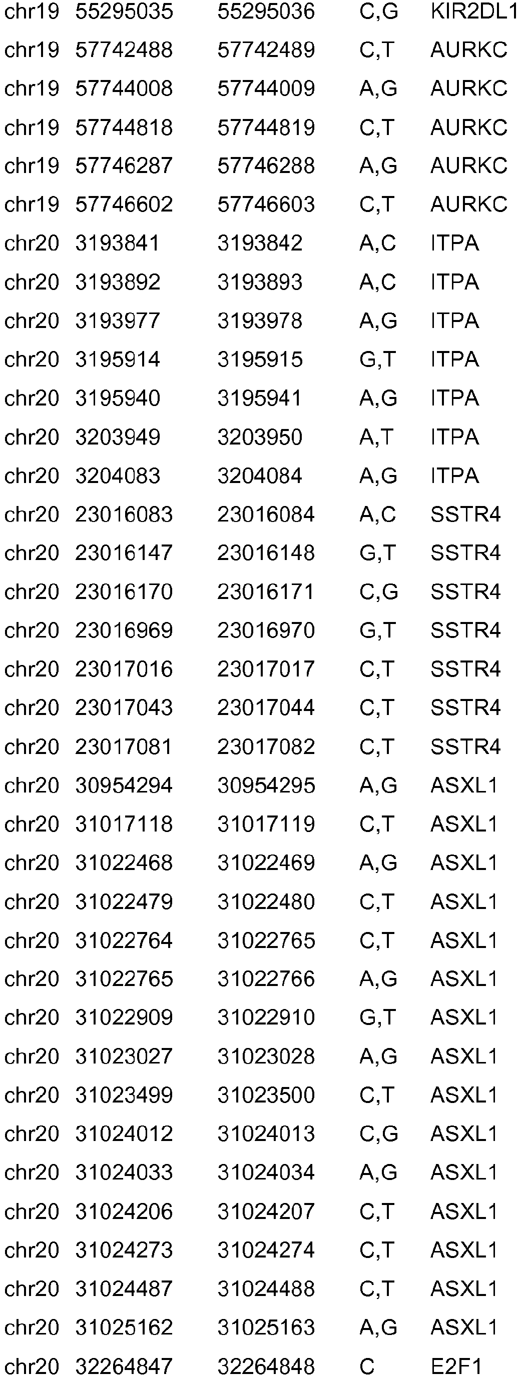

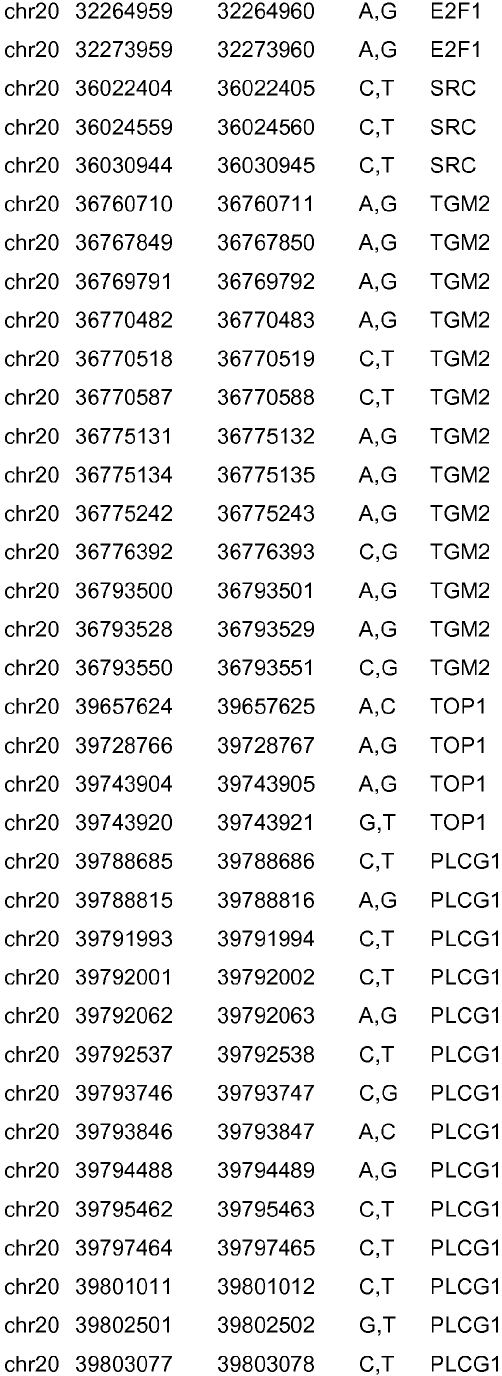

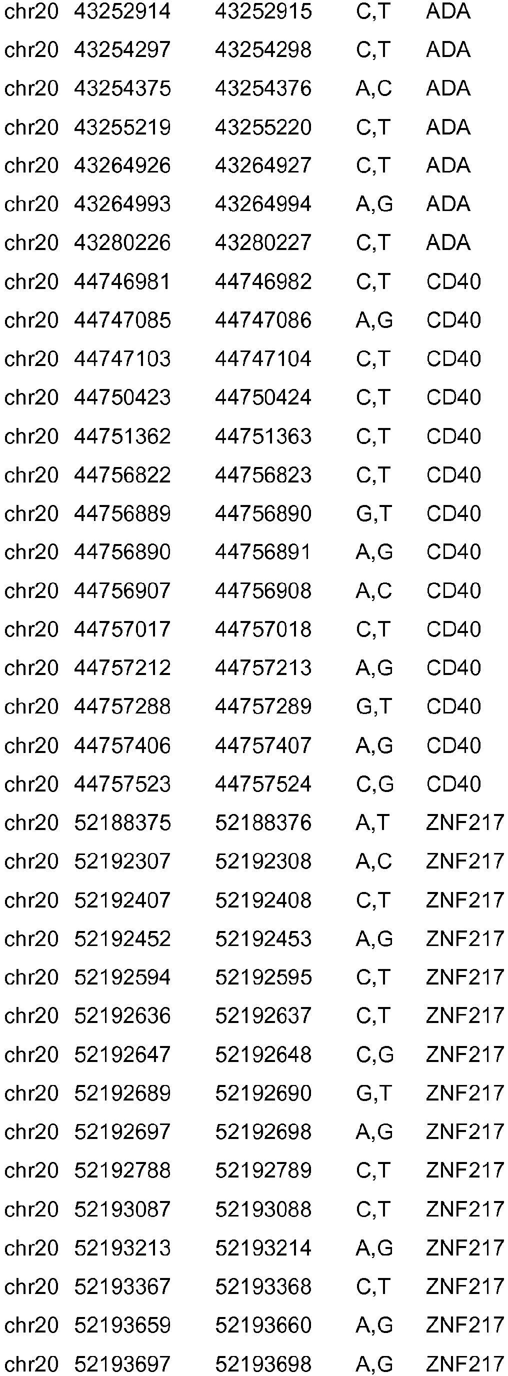

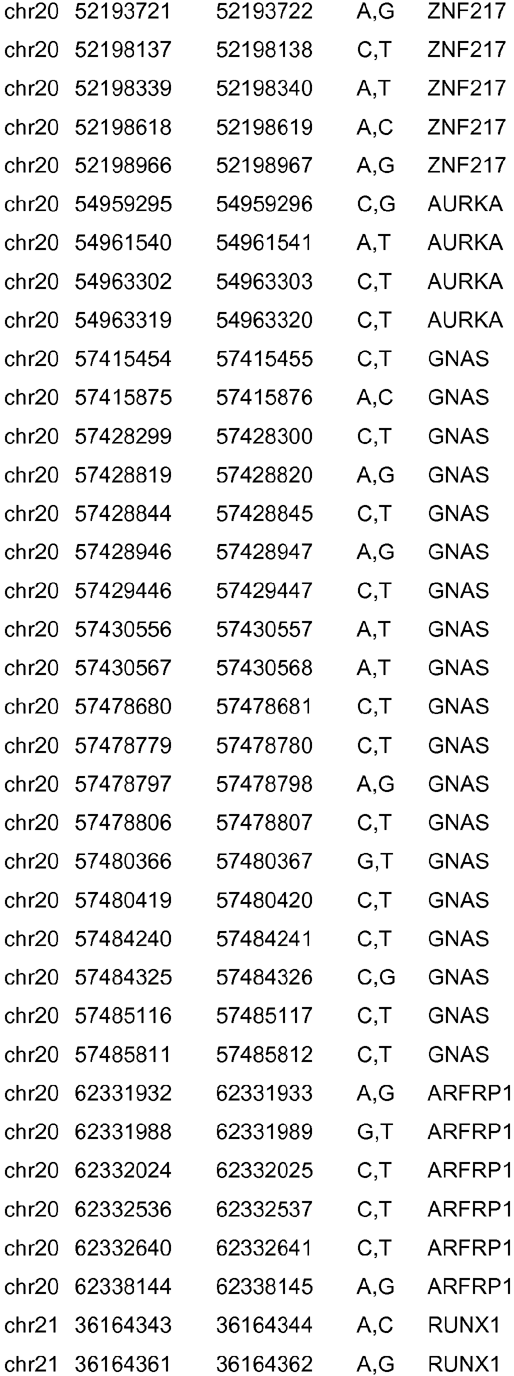

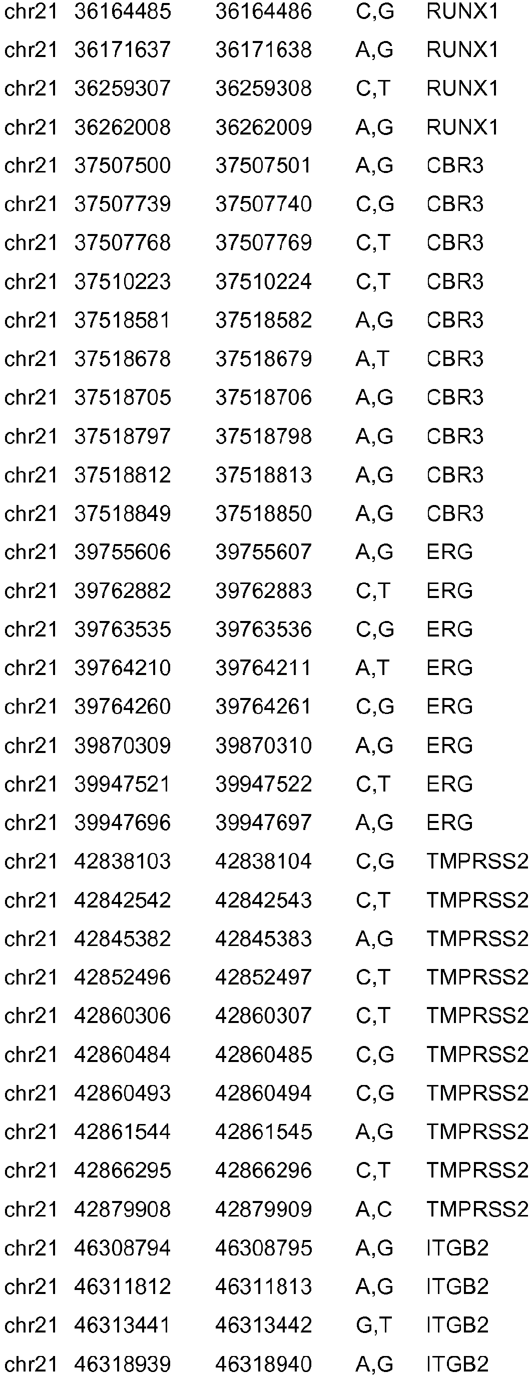

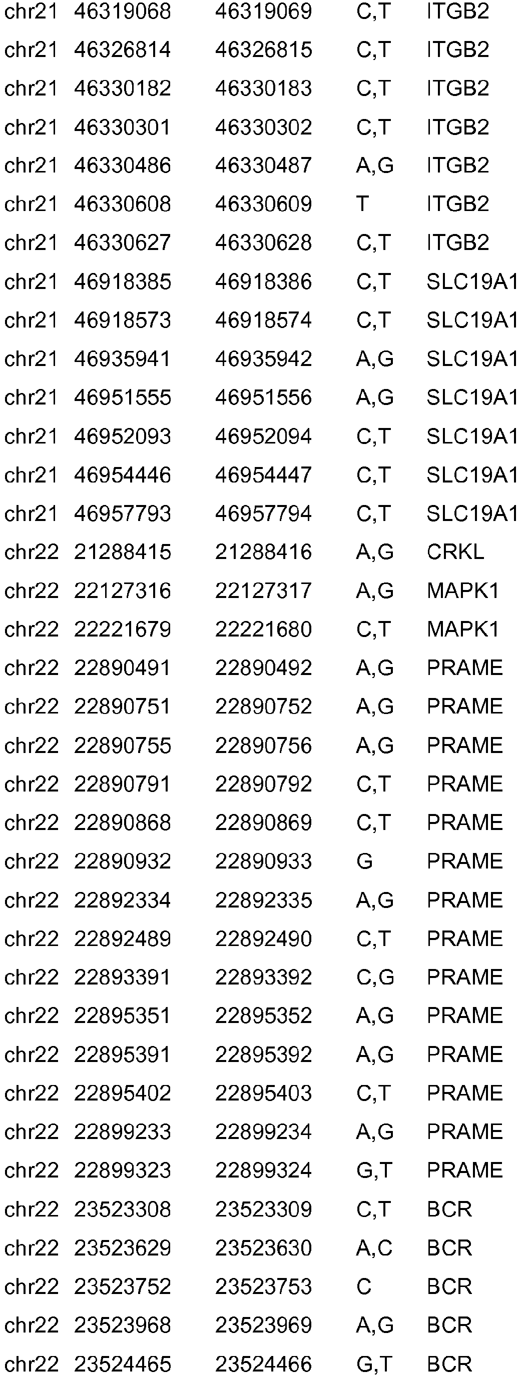

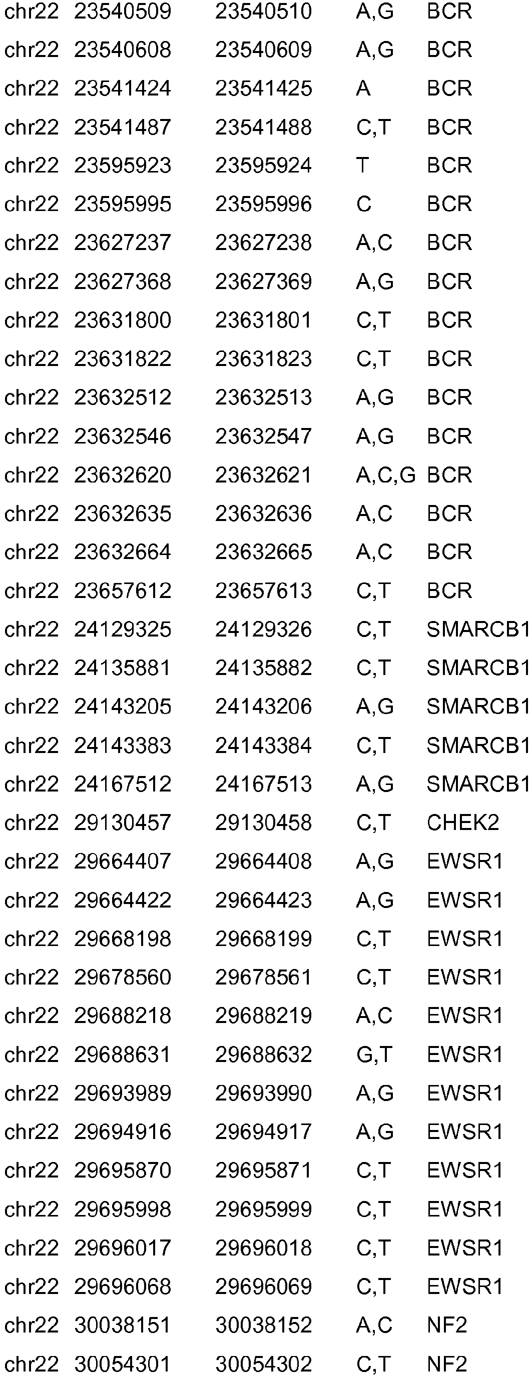

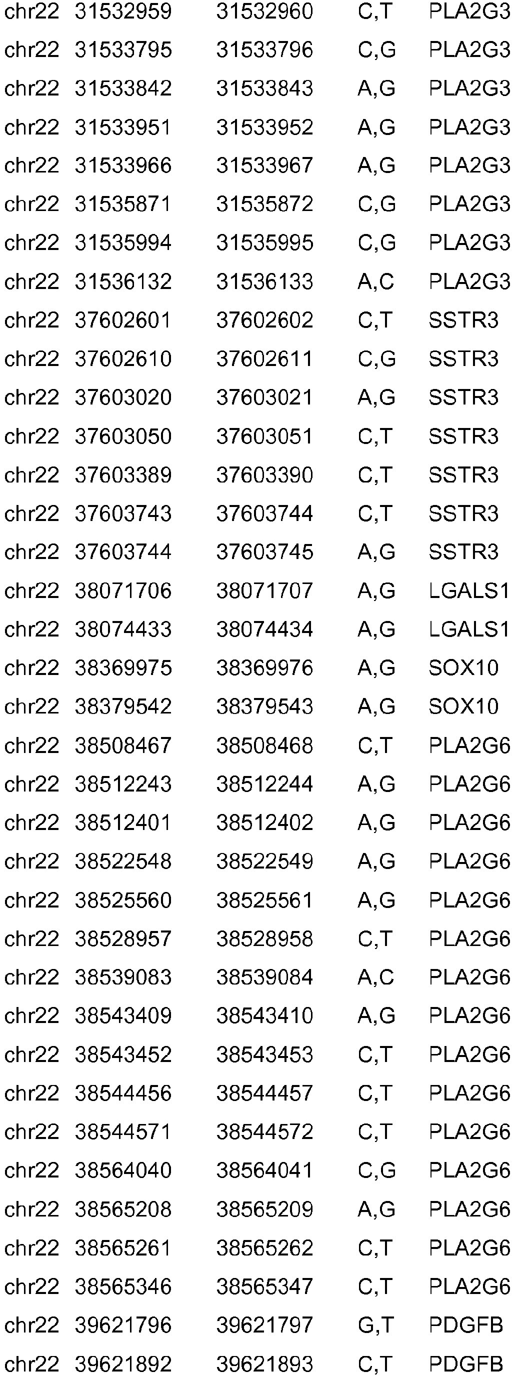

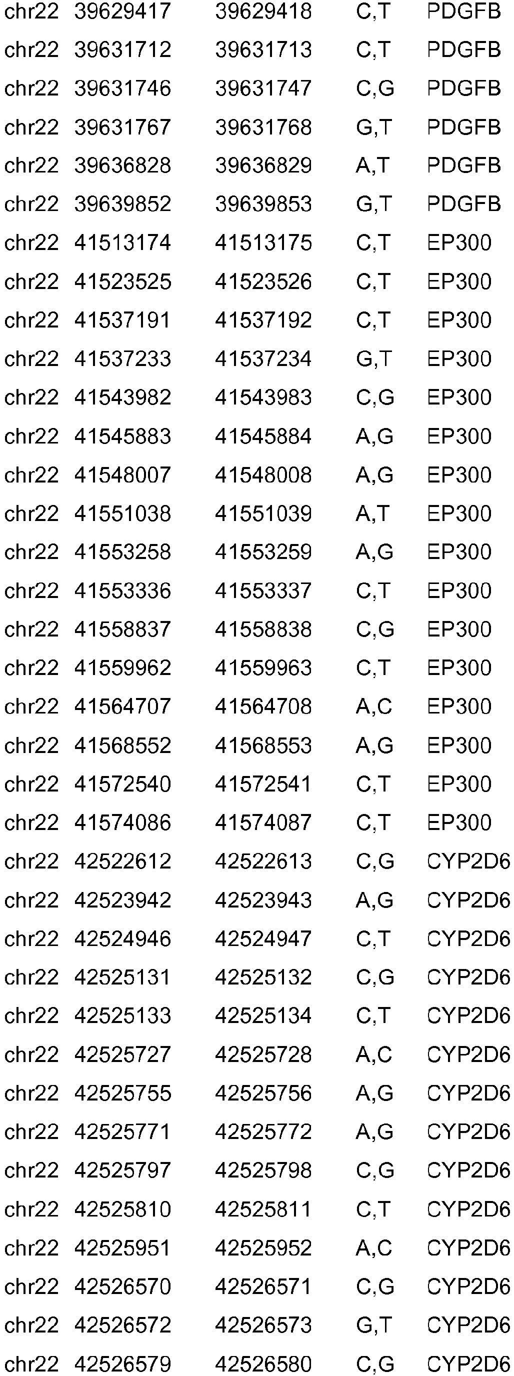

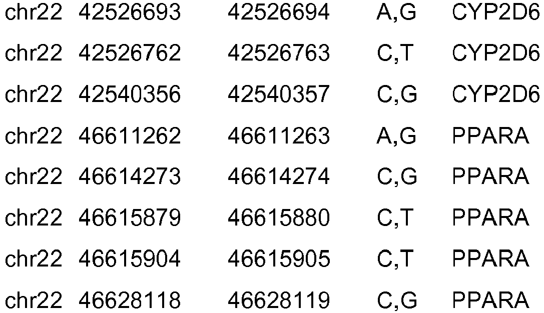

### Appendix 2

| | |
|---|---|
| chr1 | 36768200 rs1573020 |
| chr1 | 159174683 rs2814778 |
| chr1 | 204790977 rs2065160 |
| chr2 | 7149155 rs896788 |
| chr2 | 109513601 rs3827760 |
| chr2 | 136616754 rs182549 |
| chr3 | 168645035 rs1498444 |
| chr4 | 38803255 rs4540055 |
| chr4 | 159181963 rs2026721 |
| chr5 | 33951693 rs16891982 |
| chr7 | 4457003 rs917118 |
| chr10 | 17064992 rs7897550 |
| chr10 | 34755348 rs1978806 |
| chr11 | 32424389 rs5030240 |
| chr12 | 29369871 rs10843344 |
| chr12 | 56603834 rs773658 |
| chr13 | 20901724 rs1335873 |
| chr13 | 22374700 rs1886510 |
| chr13 | 34864240 rs2065982 |
| chr14 | 36170607 rs10141763 |
| chr14 | 101142890 rs730570 |
| chr15 | 28365618 rs12913832 |
| chr15 | 48426484 rs1426654 |
| chr16 | 31079371 rs881929 |
| chr16 | 90105333 rs3785181 |
| chr17 | 75551667 rs2304925 |
| chr18 | 75432386 rs1024116 |
| chr19 | 42410331 rs2303798 |
| chr20 | 38849642 rs1321333 |
| chr21 | 16685598 rs722098 |
| chr21 | 17710424 rs239031 |
| chr21 | 25672460 rs2572307 |
| chr22 | 26350103 rs5997008 |
| chr22 | 47836412 rs2040411 |

## Claims

1. A method for genomic and/or genetic analysis of a human nucleic acid sample comprising the following steps:
a) providing a group of human reference genomes;
b) testing of the human nucleic acid sample for sex and/or ancestry;
c) selecting one or more population-specific human reference genomes, PHREGs, from the group of human reference genomes on the basis on the results of the sex and/or ancestry test in step b); and
d) aligning the human nucleic acid sample sequence to the selected PHREGs.

2. The method of claim 1, wherein the alignment is performed on a majority allele level, or on a non-rare alleles level.

3. The method of claim 1 or 2, comprising the additional step:
e) performing variant calling of the aligned human nucleic acid sample with respect to the selected PHREGs.

4. The method of claim 3, wherein the variant calling is performed on a majority allele level, or on a non-rare alleles level.

5. The method of any one of the preceding claims, wherein the human reference genomes provided in step a) are published human reference genomes or are derived from published human reference genomes.

6. The method of any one of the preceding claims, wherein step a) comprises adjusting the human reference genomes to an encoding level, the encoding level comprising either unique nucleotide codes or ambiguous nucleotide codes.

7. The method of any one of the preceding claims, wherein the human reference genomes provided in step a) are PHREGs.

8. The method of any one of the preceding claims, wherein the sex test comprises one or more of the following:
testing at least one position in a sex-specific gene on chromosome X and/or
on chromosome Y; leveraging alignment differences of human genome samples on chromosome X and/or chromosome Y; cytogenetic tests; FISH analysis; CGH analysis.

9. The method of any one of the preceding claims, wherein the ancestry test is based on a machine learning algorithm used on a human nucleic acid sample, or on another classification scheme that leverages ancestry-specific variants.

10. The method of any one of the preceding claims, wherein the ancestry test comprises using the genotype of at least one genomic position and/or testing of SNP arrays or SNP chips and/or testing of markers from Sanger sequencing or mass spectroscopy.

11. The method of any one of the preceding claims, wherein the ancestry test comprises testing at least one gene selected from the group of genes consisting of ABL2, ATP1A3, CIC, CYP2C8, CYP2C9, EPHA3, EPHA7, ERBB3, ERG, ETV1, F2, FAS, HFE, IL11RA, IL2RA, ITGB6, KIF11, KIT, KLK3, LRP6, MDM4, NAT2, NTRK2, PDGFB, PIK3R1, PLA2G3, PLAU, PRKCB, RICTOR, SLC7A11, STAT3, T, TSC1, VCAM1, VDR, VEGFB, ACVRL1, AXL, CA9, CALCR, CASP9, ENG, EPHB1, ERBB4, ESR1, FGFR2, HPSE, HSP90AA1, ITK, MRE11A, PLK1, PTPRC, SERPINE1, SMC4, TERT, TLR3, WISP3, WT1, XRCC1, ANGPT2, ARID2, BARD1, CBR3, CDH2, CYP1B1, DDR2, DNMT3A, EPCAM, ERCC2, FANCG, FANCL, GSTP1, IRS2, ITGB1, JAK3, LHCGR, MSH6, NCF2, RNF43, SLC5A5, TMPRSS2, TNFRSF8, AKT1, CD248, CD4, ESR2, EZH2, IGF1R, ITGAV, ITGB2, KLHL6, MAP3K1, MET, MLL, MTHFR, NFKB1, NUP93, PARP8, RB1, RPE65, TSHR, ABL1, BLM, CYP19A1, DPP4, EPHA6, ERBB2, EWSR1, FOXP4, ITGAM, KDM5A, LPA, LTK, MLH1, PBRM1, PHLPP2, SF3B1, TNFRSF10A, ABCG2, ACPP, ADAM15, DPYD, EPHA5, EPHB6, FOLH1, KDR, MSH3, MST1R, NTRK1, ROCK2, SLC6A2, TET2, TGM2, TH, ABCB1, CD22, CD40, CD44, CDH20, CYP11B2, ERCC5, GPR124, IL7R, ITGB3, ITGB5, NCL, NOD2, NR4A1, PGR, PLCG1, PPP2R1A, PRAME, PTCH2, RET, SETD2, XPC, ASXL1, EPHB4, PLA2G6, SYK, TET1, EP300, FLT1, ITGA1, LOXL2, PDGFRB, PIK3CD, SSTR5, TEC, APC, ATR, CLU, CREBBP, CYP2D6, EML4, MMP2, PARP2, PDGFRA, TRPM8, CSF1R, DOT1L, FGFR3, FGFR4, GLP2R, IKBKE, JAK1, NOTCH2, SPEN, SPG7, BRCA1, CYP11B1, GNAS, ITGA5, LTF, NRP2, PTK2B, TNKS, ABCC1, CEACAM5, CYP4B1, EGFR, FLT3, INSR, PTCH1, SMARCA4, ZNF217, BCR, EEF2, SELP, SLCO1B1, ABCC2, FLT4, MTR, IL4R, MTOR, RPTOR, TEK, ATM, CARD11, FANCD2, MEFV, NF1, TP73, BRCA2, CD109, PTPRD, ABCC6, IGF2R, P2RX7, ROS1, ACE, PARP1, PRKDC, CENPE, TSC2, ALK, NOTCH1, TNC, NOTCH3, POLE, MLL2, MYH11, POLD1, GRIN3B, F5, FANCA, LRP1B, LRP2, VWF.

12. The method of any one of the preceding claims, wherein the human nucleic acid sample comprises a set of reads issued from a next-generation sequencing procedure, NGS, and wherein the alignment comprises a step of mapping the reads to the selected PHREGs.

13. A computer system for genomic and/or genetic analysis of a human nucleic acid sample, the computer system comprising:
a) a first module comprising computer instructions for providing a group of human reference genomes;
b) a second module for testing of a human nucleic acid sample for sex and/or ancestry;
c) a third module comprising computer instructions for selecting one or more population-specific human reference genomes, PHREGs, from the group of human reference genomes on the basis of the results of the sex and/or ancestry test; and
d) a fourth module comprising computer instructions for aligning the human nucleic acid sample to the determined PHREGs.

14. Computer program comprising instructions which, when the program is executed by a computer, cause the computer to carry out the steps a), b), c) and d) of any one of the methods of claims 1 to 12.

15. Computer-readable storage medium comprising instructions which, when executed by a computer, cause the computer to carry out the steps a), b), c) and d) of any one of the methods of claims 1 to 12.

## Patentansprüche

1. Verfahren zur genomischen und/ oder genetischen Analyse einer humanen Nukleinsäureprobe, umfassend die folgenden Schritte:
a) Bereitstellen einer Gruppe humaner Referenzgenome;
b) Testen der humanen Nukleinsäureprobe auf Geschlecht und/oder Abstammung;
c) Auswählen eines oder mehrerer populationsspezifischer humaner Referenzgenome, PHREGs, aus der Gruppe humaner Referenzgenome auf Grundlage der Ergebnisse des Geschlechts- und/oder Abstammungstests in Schritt b); und
d) Ausrichten der Sequenz der humanen Nukleinsäureprobe an die ausgewählten PHREGs.

2. Verfahren gemäß Anspruch 1, wobei das Ausrichten auf Ebene des Majoritätsallels oder auf Ebene der nicht seltenen Allele durchgeführt wird.

3. Verfahren gemäß Anspruch 1 oder 2, umfassend den zusätzlichen Schritt:
e) Durchführen eines Variant-Callings der ausgerichteten humanen Nukleinsäureprobe in Bezug auf die ausgewählten PHREGs.

4. Verfahren gemäß Anspruch 3, wobei das Variant-Calling auf Ebene des Majoritätsallels oder auf Ebene der nicht seltenen Allele durchgeführt wird.

5. Verfahren gemäß einem der vorhergehenden Ansprüche, wobei die in Schritt a) bereitgestellten humanen Referenzgenome veröffentlichte humane Referenzgenome sind oder von veröffentlichten humanen Referenzgenomen abgeleitet sind.

6. Verfahren gemäß einem der vorhergehenden Ansprüche, wobei Schritt a) das Anpassen der humanen Referenzgenome an eine Kodierungsebene umfasst, wobei die Kodierungsebene entweder eindeutige Nukleotidcodes oder mehrdeutige Nukleotidcodes umfasst.

7. Verfahren gemäß einem der vorangehenden Ansprüche, wobei die in Schritt a) bereitgestellten humanen Referenzgenome PHREGs sind.

8. Verfahren gemäß einem der vorhergehenden Ansprüche, wobei der Geschlechtstest eines oder mehrere der Folgenden umfasst:
Testen mindestens einer Position in einem geschlechtsspezifischen Gen auf dem X-Chromosom und/oder auf dem Y-Chromosom; Ausnutzen von Ausrichtungsunterschieden humaner Genomproben auf dem X-Chromosom und/oder dem Y-Chromosom; zytogenetische Tests; FISH-Analyse; CGH-Analyse.

9. Verfahren gemäß einem der vorhergehenden Ansprüche, wobei der Abstammungstest auf einem Maschinen-Lernalgorithmus, der auf eine humane Nukleinsäureprobe angewendet wird, oder auf einem anderen Klassifizierungsschema, das abstammungsspezifische Varianten ausnutzt, basiert.

10. Verfahren gemäß einem der vorhergehenden Ansprüche, wobei der Abstammungstest das Verwenden des Genotyps von mindestens einer genomischen Position und/ oder das Testen von SNP-Arrays oder SNP-Chips und/oder das Testen von Markern aus der Sanger-Sequenzierung oder Massenspektroskopie umfasst.

11. Verfahren gemäß einem der vorhergehenden Ansprüche, wobei der Abstammungstest das Testen mindestens eines Gens umfasst, ausgewählt aus der Gruppe von Genen bestehend aus ABL2, ATP1A3, CIC, CYP2C8, CYP2C9, EPHA3, EPHA7, ERBB3, ERG, ETV1, F2, FAS, HFE, IL11RA, IL2RA, ITGB6, KIF11, KIT, KLK3, LRP6, MDM4, NAT2, NTRK2, PDGFB, PIK3R1, PLA2G3, PLAU, PRKCB, RICTOR, SLC7A11, STAT3, T, TSC1, VCAM1, VDR, VEGFB, ACVRL1, AXL, CA9, CALCR, CASP9, ENG, EPHB1, ERBB4, ESR1, FGFR2, HPSE, HSP90AA1, ITK, MRE11A, PLK1, PTPRC, SERPINE1, SMC4, TERT, TLR3, WISP3, WT1, XRCC1, ANGPT2, ARID2, BARD1, CBR3, CDH2, CYP1B1, DDR2, DNMT3A, EPCAM, ERCC2, FANCG, FANCL, GSTP1, IRS2, ITGB1, JAK3, LHCGR, MSH6, NCF2, RNF43, SLC5A5, TMPRSS2, TNFRSF8, AKT1, CD248, CD4, ESR2, EZH2, IGF1R, ITGAV, ITGB2, KLHL6, MAP3K1, MET, MLL, MTHFR, NFKB1, NUP93, PARP8, RB1, RPE65, TSHR, ABL1, BLM, CYP19A1, DPP4, EPHA6, ERBB2, EWSR1, FOXP4, ITGAM, KDM5A, LPA, LTK, MLH1, PBRM1, PHLPP2, SF3B1, TNFRSF10A, ABCG2, ACPP, ADAM15, DPYD, EPHA5, EPHB6, FOLH1, KDR, MSH3, MST1R, NTRK1, ROCK2, SLC6A2, TET2, TGM2, TH, ABCB1, CD22, CD40, CD44, CDH20, CYP11B2, ERCC5, GPR124, IL7R, ITGB3, ITGB5, NCL, NOD2, NR4A1, PGR, PLCG1, PPP2R1A, PRAME, PTCH2, RET, SETD2, XPC, ASXL1, EPHB4, PLA2G6, SYK, TET1, EP300, FLT1, ITGA1, LOXL2, PDGFRB, PIK3CD, SSTR5, TEC, APC, ATR, CLU, CREBBP, CYP2D6, EML4, MMP2, PARP2, PDGFRA, TRPM8, CSF1R, DOT1L, FGFR3, FGFR4, GLP2R, IKBKE, JAK1, NOTCH2, SPEN, SPG7, BRCA1, CYP11B1, GNAS, ITGA5, LTF, NRP2, PTK2B, TNKS, ABCC1, CEACAM5, CYP4B1, EGFR, FLT3, INSR, PTCH1, SMARCA4, ZNF217, BCR, EEF2, SELP, SLCO1B1, ABCC2, FLT4, MTR, IL4R, MTOR, RPTOR, TEK, ATM, CARD11, FANCD2, MEFV, NF1, TP73, BRCA2, CD109, PTPRD, ABCC6, IGF2R, P2RX7, ROS1, ACE, PARP1, PRKDC, CENPE, TSC2, ALK, NOTCH1, TNC, NOTCH3, POLE, MLL2, MYH11, POLD1, GRIN3B, F5, FANCA, LRP1B, LRP2, VWF.

12. Verfahren gemäß einem der vorhergehenden Ansprüche, wobei die humane Nukleinsäureprobe einen Satz von Leseabschnitten aus einem Next Generation Sequencing (NGS) Sequenzierungsvorgang umfasst, und wobei das Ausrichten einen Schritt der Zuordnung der Leseabschnitte zu den ausgewählten PHREGs umfasst.

13. Computersystem für die genomische und/oder genetische Analyse einer humanen Nukleinsäureprobe, wobei das Computersystem umfasst:
a) ein erstes Modul umfassend Computeranweisungen zum Bereitstellen einer Gruppe humaner Referenzgenome;
b) ein zweites Modul zum Testen einer humanen Nukleinsäureprobe auf Geschlecht und/oder Abstammung;
c) ein drittes Modul umfassend Computeranweisungen zum Auswählen eines oder mehrerer populationsspezifischer humaner Referenzgenome, PHREGs, aus der Gruppe humaner Referenzgenome auf Grundlage der Ergebnisse des Geschlechts- und/oder Abstammungstests; und
d) ein viertes Modul umfassend Computeranweisungen für das Ausrichten der humanen Nukleinsäureprobe an die ermittelten PHREGs.

14. Computerprogramm umfassend Anweisungen, die, wenn das Programm von einem Computer ausgeführt wird, den Computer veranlassen, die Schritte a), b), c) und d) eines der Verfahren gemäß den Ansprüchen 1 bis 12 auszuführen.

15. Computerlesbares Speichermedium umfassend Anweisungen, die, wenn von einem Computer ausgeführt, den Computer veranlassen, die Schritte a), b), c) und d) eines der Verfahren gemäß den Ansprüchen 1 bis 12 auszuführen.

## Revendications

1. Procédé d'analyse génomique et/ou génétique d'un échantillon d'acide nucléique humain comprenant les étapes suivantes :
a) fourniture d'un groupe de génomes de référence humains ;
b) test de l'échantillon d'acide nucléique humain pour le sexe et/ou l'ascendance ;
c) sélection d'un ou plusieurs génomes de référence humains spécifiques à une population, PHREG, dans le groupe de génomes de référence humains sur la base des résultats du test de sexe et/ou d'ascendance dans l'étape b) ; et
d) alignement de la séquence d'échantillon d'acide nucléique humain avec les PHREG sélectionnés.

2. Procédé selon la revendication 1, dans lequel l'alignement est effectué sur un niveau d'allèle majoritaire, ou sur un niveau d'allèles non rares.

3. Procédé selon la revendication 1 ou 2, comprenant l'étape supplémentaire :
e) conduite d'un appel de variant de l'échantillon d'acide nucléique humain aligné par rapport aux PHREG sélectionnés.

4. Procédé selon la revendication 3, dans lequel l'appel de variant est effectué sur un niveau d'allèle majoritaire, ou sur un niveau d'allèles non rares.

5. Procédé selon l'une quelconque des revendications précédentes, dans lequel les génomes de référence humains fournis dans l'étape a) sont des génomes de référence humains publiés ou sont dérivés de génomes de référence humains publiés.

6. Procédé selon l'une quelconque des revendications précédentes, dans lequel l'étape a) comprend l'ajustement des génomes de référence humains à un niveau de codage, le niveau de codage comprenant des codes de nucléotide unique ou des codes de nucléotide ambigus.

7. Procédé selon l'une quelconque des revendications précédentes, dans lequel les génomes de référence humains fournis dans l'étape a) sont des PHREG.

8. Procédé selon l'une quelconque des revendications précédentes, dans lequel le test de sexe comprend l'un ou plusieurs des suivants :
test d'au moins une position dans un gène spécifique à un sexe sur le chromosome X et/ou sur le chromosome Y ; l'utilisation de différences d'alignement d'échantillons de génome humain sur le chromosome X et/ou le chromosome Y ; des tests cytogénétiques ; l'analyse FISH ; l'analyse CGH.

9. Procédé selon l'une quelconque des revendications précédentes, dans lequel le test d'ascendance est basé sur un algorithme d'apprentissage automatique utilisé sur un échantillon d'acide nucléique humain, ou sur un autre schéma de classification qui est basé sur des variants spécifiques à l'ascendance.

10. Procédé selon l'une quelconque des revendications précédentes, dans lequel le test d'ascendance comprend l'utilisation du génotype d'au moins une position génomique et/ou le test de réseaux à SNP ou de puces à SNP et/ou le test de marqueurs de séquençage Sanger ou de spectrométrie de masse.

11. Procédé selon l'une quelconque des revendications précédentes, dans lequel le test d'ascendance comprend le test d'au moins un gène choisi dans le groupe de gènes constitué de ABL2, ATP1A3, CIC, CYP2C8, CYP2C9, EPHA3, EPHA7, ERBB3, ERG, ETV1, F2, FAS, HFE, IL11RA, IL2RA, ITGB6, KIF11, KIT, KLK3, LRP6, MDM4, NAT2, NTRK2, PDGFB, PIK3R1, PLA2G3, PLAU, PRKCB, RICTOR, SLC7A11, STAT3, T, TSC1, VCAM1, VDR, VEGFB, ACVRL1, AXL, CA9, CALCR, CASP9, ENG, EPHB1, ERBB4, ESR1, FGFR2, HPSE, HSP90AA1, ITK, MRE11A, PLK1, PTPRC, SERPINE1, SMC4, TERT, TLR3, WISP3, WT1, XRCC1, ANGPT2, ARID2, BARD1, CBR3, CDH2, CYP1B1, DDR2, DNMT3A, EPCAM, ERCC2, FANCG, FANCL, GSTP1, IRS2, ITGB1, JAK3, LHCGR, MSH6, NCF2, RNF43, SLC5A5, TMPRSS2, TNFRSF8, AKT1, CD248, CD4, ESR2, EZH2, IGF1R, ITGAV, ITGB2, KLHL6, MAP3K1, MET, MLL, MTHFR, NFKB1, NUP93, PARP8, RB1, RPE65, TSHR, ABL1, BLM, CYP19A1, DPP4, EPHA6, ERBB2, EWSR1, F0XP4, ITGAM, KDM5A, LPA, LTK, MLH1, PBRM1, PHLPP2, SF3B1, TNFRSF10A, ABCG2, ACPP, ADAM15, DPYD, EPHA5, EPHB6, F0LH1, KDR, MSH3, MST1R, NTRK1, R0CK2, SLC6A2, TET2, TGM2, TH, ABCB1, CD22, CD40, CD44, CDH20, CYP11B2, ERCC5, GPR124, IL7R, ITGB3, ITGB5, NCL, N0D2, NR4A1, PGR, PLCG1, PPP2R1A, PRAME, PTCH2, RET, SETD2, XPC, ASXL1, EPHB4, PLA2G6, SYK, TET1, EP300, FLT1, ITGA1, L0XL2, PDGFRB, PIK3CD, SSTR5, TEC, APC, ATR, CLU, CREBBP, CYP2D6, EML4, MMP2, PARP2, PDGFRA, TRPM8, CSF1R, D0T1L, FGFR3, FGFR4, GLP2R, IKBKE, JAK1, N0TCH2, SPEN, SPG7, BRCA1, CYP11B1, GNAS, ITGA5, LTF, NRP2, PTK2B, TNKS, ABCC1, CEACAM5, CYP4B1, EGFR, FLT3, INSR, PTCH1, SMARCA4, ZNF217, BCR, EEF2, SELP, SLC01B1, ABCC2, FLT4, MTR, IL4R, MTOR, RPTOR, TEK, ATM, CARD11, FANCD2, MEFV, NF1, TP73, BRCA2, CD109, PTPRD, ABCC6, IGF2R, P2RX7, ROSI, ACE, PARP1, PRKDC, CENPE, TSC2, ALK, N0TCH1, TNC, NOTCH3, POLE, MLL2, MYH11, POLD1, GRIN3B, F5, FANCA, LRP1B, LRP2, VWF.

12. Procédé selon l'une quelconque des revendications précédentes, dans lequel l'échantillon d'acide nucléique humain comprend un ensemble de lectures dérivé d'une procédure de séquençage de nouvelle génération, NGS, et dans lequel l'alignement comprend une étape de mise en correspondance des lectures avec les PHREG sélectionnés.

13. Système informatique pour analyse génomique et/ou génétique d'un échantillon d'acide nucléique humain, le système informatique comprenant :
a) un premier module comprenant des instructions informatiques pour fournir un groupe de génomes de référence humains ;
b) un deuxième module pour le test d'un échantillon d'acide nucléique humain pour le sexe et/ou l'ascendance ;
c) un troisième module comprenant des instructions informatiques pour sélectionner un ou plusieurs génomes de référence humains spécifiques à une population, PHREG, dans le groupe de génomes de référence humains sur la base des résultats du test de sexe et/ou d'ascendance ; et
d) un quatrième module comprenant des instructions informatiques pour aligner l'échantillon d'acide nucléique humain avec les PHREG déterminés.

14. Programme informatique comprenant des instructions qui, lorsque le programme est exécuté par un ordinateur, amènent l'ordinateur à effectuer les étapes a), b), c) et d) de l'un quelconque des procédés des revendications 1 à 12.

15. Support de stockage lisible par ordinateur comprenant des instructions qui, lorsqu'elles sont exécutées par un ordinateur, amènent l'ordinateur à conduire les étapes a), b), c) et d) de l'un quelconque des procédés des revendications 1 à 12.
